# FASCICULE DE BREVET EUROPEEN

(11) **EP 1 170 281 B1**
(45) Date de publication et mention de la délivrance du brevet: **21.12.2005**
(21) Numéro de dépôt: 01401677.8
(22) Date de dépôt: 26.06.2001
(51) Int. Cl.: C07C 219/24, A61K 31/21, C07C 271/34, C07C 217/44, C07C 219/18, C07C 271/12, C07D 213/79, C07D 213/65, C07C 323/20, A61K 31/325, A61K 31/435, A61K 31/13, C07C 217/52, C07D 213/70, C07D 295/08, A61P 25/00

(54) **Nouveaux composés cyclopropaniques 1,1 et 1,2-disubstitués, leur procédé de préparation et les compositions pharmaceutiques qui les contiennent**
1,1- and 1,2-Disubstituierte Cyclopropane, Verfahren zu deren Herstellung und deren pharmazeutischen Zusammensetzungen
1,1- and 1,2-disubstituted cyclopropanes, process for their preparation and pharmaceutical compositions thereof

(30) Priorité: 27.06.2000 FR 0008203
(43) Date de publication de la demande: 09.01.2002
(73) Titulaire: Les Laboratoires Servier, 92200 Neuilly sur Seine (FR)
(72) Inventeur: Goldstein, Solo, 92150 Suresnes (FR); Guillonneau, Claude, 92140 Clamart (FR); Charton, Yves, 92330 Sceaux (FR); Lockhart, Brian, 78290 Croissy sur Seine (FR); Lestage, Pierre, 78170 La Celle Saint Cloud (FR)

(56) Documents cités:
- EP-A- 0 066 993
- WO-A-96/25160
- FR-M- 723
- US-A- 6 034 275
- C. KAISER ET AL.: "2-Substituted cyclopropylamines. I. Derivatives and analogs of 2-phenylcyclopropylamine" JOURNAL OF MEDICINAL AND PHARMACEUTICAL CHEMISTRY., vol. 5, 1962, pages 1243-1265, XP002163312 AMERICAN CHEMICAL SOCIETY. EASTON., US ISSN: 0022-2623

## Description

La présente invention concerne de nouveaux composés cyclopropaniques 1,1 et 1,2-disubstitués, leur procédé de préparation et les compositions pharmaceutiques qui les contiennent.

Les composés de la présente invention sont particulièrement intéressants d'un point de vue pharmacologique pour leur interaction spécifique avec les récepteurs nicotiniques centraux de type α4β2, trouvant leur application dans le traitement des neuropathologies associées au vieillissement cérébral, des troubles de l'humeur, de la douleur et du sevrage tabagique.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence. Il est largement démontré que parmi les différents neurotransmetteurs, l'acétylcholine tient une place prépondérante dans les fonctions de mémoire et que les voies neuronales cholinergiques sont dramatiquement détruites lors de certaines maladies neurodégénératives ou en déficit d'activation lors du vieillissement cérébral. C'est pourquoi, de nombreuses approches thérapeutiques ont visé à empêcher la destruction du neuromédiateur *via* l'inhibition de l'acétylcholine-estérase ou ont cherché à se substituer au neuromédiateur déficitaire. Dans ce dernier cas, les agonistes cholinergiques proposés ont été de type muscarinique, spécifiques pour les récepteurs post-synaptiques M1.

Récemment, il a été montré que l'atteinte cholinergique liée à la maladie d'Alzheimer touchait davantage les neurones portant les récepteurs nicotiniques que ceux portant les récepteurs muscariniques (Schroder et Coll., « Alzheimer disease : therapeutic strategies », Birkhauser Boston, 1994, 181-185). De plus, de nombreuses études ont démontré que la nicotine possède des propriétés facilitatrices de la mémoire (Prog. Neuropsychopharmacol., 1992, 16, 181-191) et que ces propriétés s'exercent tout autant sur les fonctions mnésiques (Psychopharmacol., 1996, 123, 88-97) que sur les facultés d'attention et de vigilance (Psychopharmacol., 1995, 118, 195-205). Par ailleurs, la nicotine exerce des effets neuroprotecteurs vis-à-vis d'agents excitotoxiques tel que le glutamate (Brain Res., 1994, 644, 181-187).

L'ensemble de ces données est très probablement à relier avec les études épidémiologiques qui ont montré une moindre incidence de maladie d'Alzheimer ou de Parkinson chez les sujets fumeurs. De plus, plusieurs études ont montré l'intérêt de la nicotine dans le traitement des troubles de l'humeur tels que les états dépressifs, anxieux ou schizophréniques. Enfin, il a été montré que la nicotine possède des propriétés antalgiques. L'ensemble des propriétés thérapeutiques de la nicotine, ainsi que celles décrites pour d'autres agents nicotiniques, est sous tendu par une activité vis-à-vis de récepteurs centraux qui diffèrent structurellement et pharmacologiquement des récepteurs périphériques (muscle et ganglion). Les récepteurs centraux de type α4β2 sont les plus représentés dans le système nerveux central et ont été impliqués dans la plupart des effets thérapeutiques de la nicotine (Life Sci., 1995, 56, 545-570).

Plusieurs documents tels que Synlett., 1999, 7, 1053-1054 ; J. Med. Chem, 1985, 28(12), 1953-1957 et 1980, 23(3), 339-341 ; 1970, 13(5), 820-826 ; 1972, 15(10), 1003-1006 ; J. Am. Chem. Soc., 1987, 109(13), 4036-4046, ou quelques brevets ou demandes de brevets comme DE 36 08 727, EP 124 208 ou WO 94/10158 décrivent et revendiquent des composés comportant un motif cyclopropanique 1,1 ou 1,2-disubstitué. Aucunes de ces références ne décrivent ou ne suggèrent pour ces composés une activité pharmacologique spécifique vis-à-vis des récepteurs nicotiniques et plus particulièrement vis-à-vis des récepteurs nicotiniques centraux de type α4β2, propriété originale des composés décrits par la Demanderesse. Le brevet WO 96/25160 décrit des composés dérivés d'azabicycle ayant une activité pharmacologique spécifique vis-à-vis des récepteurs nicotiniques cholinergiques. De ce fait, aucun élément dans l'art antérieur ne présageait les caractéristiques spécifiques et surprenantes des produits revendiqués dans la présente demande.

Les composés de la présente invention sont donc nouveaux et constituent de puissants ligands nicotiniques sélectifs du sous-type réceptoriel α4β2 central. De ce fait, ils sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

Plus particulièrement, la présente invention concerne les composés de formule (I): 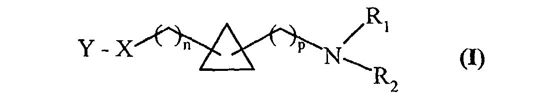 dans laquelle :
p représente un entier compris entre 0 et 6 inclus,
n représente un entier compris entre 0 et 6 inclus,
R₁ et R₂, identiques ou différents, indépendamment l'un de l'autre, représentent un groupement choisi parmi atome d'hydrogène, groupement alkyle (C₁-C₆) linéaire ou ramifié, aryle, et arylalkyle (C₁-C₆) linéaire ou ramifié, ou R₁+R₂ forment ensemble, avec l'atome d'azote qui les porte, un système monocyclique ou bicyclique, saturé, de 3 à 10 chaînons et contenant éventuellement un second hétéroatome choisi parmi oxygène, azote et soufre,
X représente un groupement choisi parmi atome d'oxygène, atome de soufre, groupement -CH=CH-, méthylène, groupement de formule -HC=N-O- et groupement de formule -O-CH₂-CH=CH- dans lesquels l'atome d'oxygène est relié à la partie Y des composés de formule (I),
Y représente un groupement choisi parmi aryle, hétéroaryle, arylalkyle (C₁-C₆) linéaire ou ramifié, hétéroarylalkyle (C₁-C₆) linéaire ou ramifié, -C(O)-A, et -C(S)-A,
A représente un groupement choisi parmi alkyle (C₁-C₆) linéaire ou ramifié, aryle, hétéroaryle, arylalkyle (C₁-C₆) linéaire ou ramifié, hétéroarylalkyle (C₁-C₆) linéaire ou ramifié, et NR₃R₄ dans lequel R₃ et R₄, identiques ou différents, représentent chacun un groupement choisi parmi atome d'hydrogène, groupement alkyle (C₁-C₆) linéaire ou ramifié, aryle, et arylalkyle (C₁-C₆) linéaire ou ramifié, ou R₃+R₄ forment ensemble, avec l'atome d'azote qui les porte, un système monocyclique ou bicyclique en (C₃-C₁₀),
leurs isomères ainsi que leurs sels d'addition à un acide ou à une base pharmaceutiquement acceptable,
étant entendu que :
* *dans le cas de composés deformule (I) 1,1-disubstitué,*
   - p est différent de zéro quand X représente un groupement méthylène, n prend la valeur zéro, Y représente un groupement aryle ou hétéroaryle, R₁ et R₂, identiques ou différents, représentent un atome d'hydrogène, un groupement alkyle (C₁-C₄) linéaire ou ramifié, benzyle, phényléthyle, ou forment ensemble avec l'atome d'azote qui les porte un groupement morpholino, thiomorpholino, ou un système cyclique carboné saturé en 5 à 7 chaînons,
   - p est différent de zéro quand X représente un groupement méthylène, n prend la valeur zéro, Y représente un groupement acétyle, R₁ et R₂, identiques ou différents, représentent un atome d'hydrogène, un groupement alkyle (C₁-C₄) linéaire ou ramifié, phényle, benzyle, ou forment ensemble avec l'atome d'azote qui les porte un groupement pipéridinyle ou morpholino,
   - R₁ et R₂ ne représentent pas simultanément un groupement méthyle :
      * soit quand p et n prennent chacun la valeur 1, X représente un atome d'oxygène, et Y représente un groupement choisi parmi p-nitrobenzoyle, p-aminobenzoyle, p-chlorophénylaminocarbonyle, et acétyle,
      * soit quand p prend la valeur zéro, n prend la valeur 1, X représente un atome d'oxygène ou de soufre, et Y représente un groupement 2-quinolyle substitué en position trois par un groupement alkyle (C₃-C₄) linéaire ou ramifié, ou phényle,
   - Y ne représente pas un groupement 1,2-benzisoxazol-3-yl quand n prend la valeur 1, p prend la valeur zéro et X représente un atome d'oxygène,
* *dans le cas de composés de formule (I) 1,2-disubstitué,*
   - R₁ et R₂ ne représentent pas simultanément un atome d'hydrogène quand p et n prennent chacun la valeur zéro et X-Y représentent ensemble un groupement phénoxy (éventuellement substitué par un ou deux groupements, identiques ou différents, choisis parmi méthoxy, diméthylamino, atome d'halogène, méthyle, trifluorométhyle, nitro, et amino), phénylsulfanyle, benzyloxy, benzyle, ou 2-phényléthyle,
   - R₁ et R₂ ne représentent pas simultanément un groupement méthyle, quand p et n prennent chacun la valeur zéro, et X-Y représentent ensemble un groupement phénoxy (éventuellement substitué par un groupement choisi parmi atome de chlore et trifluorométhyle), un groupement phénylsulfanyle, ou un groupement benzyle,
étant entendu également que les composés de formule (I) sont différents des composés suivants :
- (1-benzylcyclopropyl)méthanamine,
- (1-benzylcyclopropyl)-N,N-diméthylméthanamine,
- 2-(phénoxycyclopropyl)méthanamine,
- 2-(phénoxyméthyl)-cyclopropanamine,
- (N,N-diméthyl)-2-(acétoxyméthyl)-cyclopropaneméthanamine,
- N-{2-[2-(benzyloxy)éthyl]cyclopropyl}-N,N-diméthylamine.

Par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi atomes d'halogène, groupements alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié.

Par groupement hétéroaryle, on comprend un système monocyclique aromatique ou bicyclique de 5 à 12 chaînons, contenant de un à trois hétéroatomes, identiques ou différents, choisis parmi oxygène, azote et soufre, et dont l'un des cycles, dans le cas d'un système bicyclique, possède un caractère aromatique, l'autre cycle pouvant être aromatique ou partiellement hydrogéné, chacun de ces groupements pouvant être éventuellement substitué par un ou plusieurs groupements identiques ou différents, choisis parmi les substituants précédemment définis dans le cas d'un groupement aryle.

D'une façon générale, les composés 1,1-disubstitué et 1,2-disubstitué concerne respectivement des composés possédant un motif

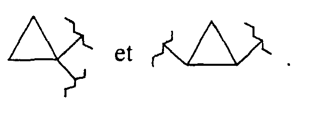

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Des composés préférés de l'invention sont les composés pour lesquels n est un entier compris entre 0 et 2 inclus.

D'une façon avantageuse, les composés préférés de l'invention sont les composés pour lesquels p est un entier prenant la valeur 0 ou 1.

Les substituants R₁ et R₂ préférés selon l'invention sont l'atome d'hydrogène et le groupement alkyle (C₁-C₆) linéaire ou ramifié.

Le substituant X préféré selon l'invention est l'atome d'oxygène.

Les substituants Y préférés selon l'invention sont les groupements choisis parmi -C(O)NR₃R₄ dans lequel R₃ et R₄ sont tels que définis dans la formule (I), acétyle, -C(O)-hétéroaryle, arylalkyle (C₁-C₆) linéaire ou ramifié, et hétéroaryle. D'une façon avantageuse, le groupement hétéroaryle préféré dans les définitions de Y est le groupement pyridinyle.

Selon une variante avantageuse de l'invention, les composés préférés sont les composés de formule (I) 1,1-disubstitués appartenant à la formule **(IA) :** 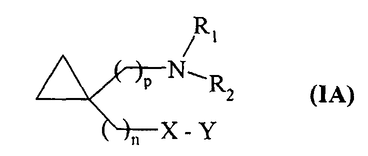 dans laquelle n, p, X, Y, R₁ et R₂ sont tels que définis dans la formule (I).

Selon une autre variante avantageuse de l'invention, les composés préférés sont les composés de formule (I) 1,2-disubstitués appartenant à la formule **(IB) :** 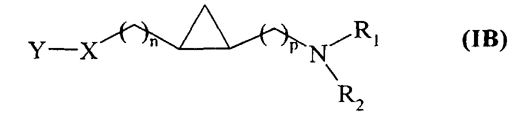 dans laquelle n, p, X, Y, R₁ et R₂ sont tels que définis dans la formule (I).

D'une façon particulièrement avantageuse, les composés préférés de l'invention sont les composés de formule (IB) dans laquelle p représente 0 ou 1, n représente 0 ou 1, R₁ et R₂, identiques ou différents, représentent un atome d'hydrogène ou un groupement alkyle (C₁-C₆) linéaire ou ramifié, X représente un atome d'oxygène, et Y représente un groupement choisi parmi phénylalkyl (C₁-C₆) linéaire ou ramifié, pyridinyle, et -C(O)-A dans lequel A représente un groupement alkyle (C₁-C₆) linéaire ou ramifié, monoalkyl(C₁-C₆)amino, ou dialkyl(C₁-C₆)amino, la partie alkyle étant linéaire ou ramifié.

D'une autre façon très intéressante, les composés préférés de l'invention sont les composés de formule (IA), dans laquelle p représente 0 ou 1, n est un entier compris entre 0 et 3 inclus, R₁ et R₂, identiques ou différents, représentent un atome d'hydrogène, un groupement alkyle (C₁-C₆) linéaire ou ramifié, ou forment ensemble avec l'atome d'azote qui les porte un radical pyrrolidinyle, X représente un atome d'oxygène, de soufre ou un groupement -CH=CH-, et Y représente un groupement choisi parmi phényle (éventuellement substitué par un groupement hydroxy, alkyle (C₁-C₆) linéaire ou ramifié, ou un atome d'halogène), pyridinyle, pyridinylalkyle (C₁-C₆) linéaire ou ramifié (le radical pyridinyle dans chacun des groupements étant éventuellement substitué par un groupement choisi parmi atome d'halogène, et groupement alkyle (C₁-C₆) linéaire ou ramifié), et -C(O)-A dans lequel A représente un groupement choisi parmi alkyle (C₁-C₆) linéaire ou ramifié, monoalkyl(C₁-C₆)amino linéaire ou ramifié, dialkyl(C₁-C₆)amino linéaire ou ramifié, et pyridinyle.

D'une façon particulièrement avantageuse, les composés préférés de l'invention sont le :
■ 2-[1-(diméthylamino)cyclopropyl]éthyl méthylcarbamate,
■ 2-[1-(diméthylamino)cyclopropyl]éthyl diméthylcarbamate,
■ [1-(diméthylamino)cyclopropyl]méthyl diméthylcarbamate,
■ [1-(diméthylamino)cyclopropyl]méthyl acétate,
■ 2-[1-(diméthylamino)cyclopropyl]éthyl acétate,
■ 1-[(diméthylamino)méthyl]cyclopropyl acétate,
■ [1-(diméthylamino)cyclopropyl]méthyl nicotinate,
■ *N*,*N*-diméthyl-1-[(3-pyridinyloxy)méthyl]cyclopropanamine,
■ *N*-méthyl-1-[(3-pyridinyloxy)méthyl]cyclopropanamine,
■ *N*,*N*-diméthyl-1-[(3-pyridinylméthoxy)méthyl]cyclopropanamine,
■ *N*,*N*-diméthyl-1-[2-(3-pyridinyloxy)éthyl]cyclopropanamine,
■ 4-({2-[1-diméthylamino)cyclopropyl]éthyl}sulfanyl)phénol,
■ (±)*cis*-2-(diméthylamino)cyclopropyl méthylcarbamate,
■ (±)*trans*-2-(diméthylamino)cyclopropyl méthylcarbamate,
■ (±)*cis*-2-(diméthylamino)cyclopropyl acétate,
■ (±)*trans*-2-(diméthylamino)cyclopropyl acétate,
■ (±)*cis*-2-(diméthylamino)cyclopropyl]méthyl acétate,
■ (±)*trans*-2-(diméthylamino)cyclopropyl]méthyl acétate,
■ (±)*cis*-2-[(benzyloxy)méthyl]-*N*,*N*-diméthylcyclopropanamine,
■ (±)*trans*-2-[(benzyloxy)méthyl]-*N*,*N*-diméthylcyclopropanamine,
■ (±)*trans*-2-[(diméthylamino)méthyl]cyclopropyl acétate,
■ dichlorhydrate de 1-[(3-pyridinyloxy)méthyl]cyclopropanamine,
■ chlorhydrate de *N*-méthyl-1-{[(6-méthyl-3-pyridinyl)oxy]méthyl}cyclopropanamine,
■ chlorhydrate de *N*-méthyl-1-{[(6-chloro-3-pyridinyl)oxy]méthyl}cyclopropanamine,
■ chlorhydrate de *N*-{1-[(3-fluorophénoxy)méthyl]cyclopropyl}-*N*-méthylamine,
■ fumarate de 3-[1-(diméthylamino)cyclopropyl]propyl diméthylcarbamate,
■ fumarate de 3-[1-(diméthylamino)cyclopropyl]propyl méthylcarbamate,
■ dichlorhydrate de *N*-méthyl-1-[(2-pyridinylsulfanyl)méthyl]cyclopropanamine,
■ dichlorhydrate de *N*-méthyl-1-[3-(3-pyridinyloxy)propyl]cyclopropanamine,
■ dichlorhydrate de *N*-méthyl-1-[2-(3-pyridinyl)éthyl]cyclopropanamine,
■ fumarate de *N*-méthyl-1-[(Z)-2-(3-pyridinyl)éthényl]cyclopropanamine,
■ fumarate de [1-(1-pyrrolidinyl)cyclopropyl]méthyl diméthylcarbamate,
■ chlorhydrate de *N*,*N*-diméthyl-1-[2-(3-pyridinyl)éthyl]cyclopropanamine,
■ fumarate de 3-{[1-(1-pyrrolidinyl)cyclopropyl]méthoxy}pyridine,
■ fumarate de *N*-méthyl-1-[2-(3-pyridinyloxy)éthyl]cyclopropanamine,
■ chlorhydrate de 2-[1-(méthylamino)cyclopropyl]éthyl diméthylcarbamate, et le
■ fumarate de 2-[1-(1-pyrrolidinyl)cyclopropyl]éthyl diméthylcarbamate.

Les isomères ainsi que les sels d'addition à un acide ou à une base pharmaceutiquement acceptable des composés préférés font partie intégrante de l'invention.

La présente invention concerne également le procédé de préparation des composés de formule (I), caractérisé en ce qu'on utilise comme produit de départ un composé de formule **(II)** : 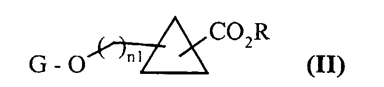 dans lequel G représente un groupement protecteur des fonctions hydroxy classiquement utilisé en synthèse organique, R représente un atome d'hydrogène ou un groupement alkyle (C₁-C₆) linéaire ou ramifié, et n₁ représente 0 ou 1,
composés de formule (II), qui sont :
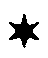 ***soit*** mis à réagir avec de l'ammoniaque liquide en présence d'un cyanure alcalin en solvant alcoolique, pour conduire aux composés de formule **(III) :** 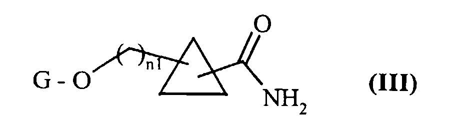 dans laquelle G représente un groupement protecteur des fonctions hydroxy, et n₁ représente 0 ou 1,
   composés de formule (III) qui sont traités par un dihalogène en présence d'une base telle que la soude, pour conduire aux composés de formule **(IV) :** 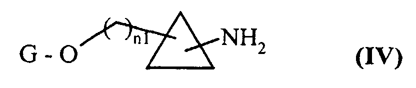 dans laquelle G et n₁ sont tels que définis précédemment,
   composés de formule (IV) dont on protège sélectivement la fonction amine primaire par un groupement protecteur G₂ classiquement utilisé en chimie organique tel que le groupement BOC (t-butyloxycarbonyle), pour conduire aux composés de formule **(V) :** 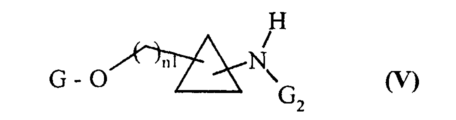 dans lequel n₁, G et G₂ sont tels que définis précédemment,
   composés de formule (V) qui sont ensuite successivement :
   - traités par un composé de formule **(VIA),** en milieu basique :

      R₁ - L₁ **(VIA)**

      dans laquelle R₁ est tel que défini dans la formule (I) et L₁ représente un groupe partant usuel de la synthèse organique,
   - puis dont la fonction amine est déprotégée puis soit ne subissent pas d'autre traitement et conduisent alors aux composés de formule **(VIIA) :** 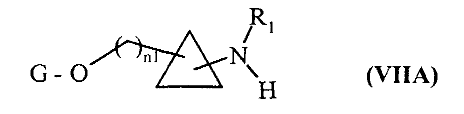 dans laquelle R₁, n₁ et G sont tels que définis précédemment,
      soit sont mis à réagir avec un composé de formule **(VIB)** :

      R₂ₐ - L₁ **(VIB)**

      dans laquelle R₂ₐ a les mêmes définitions que R₂, tel que défini dans la formule (I), à l'exception de la valeur atome d'hydrogène, et L₁ est tel que défini précédemment,
      pour conduire aux composés de formule **(VIIB) :** 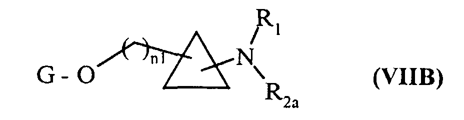 dans laquelle R₁, R₂ₐ, n₁ et G sont tels que définis précédemment,
      l'ensemble des composés de formule (VIIA) et (VIIB) formant les composés de formule **(VII) :** 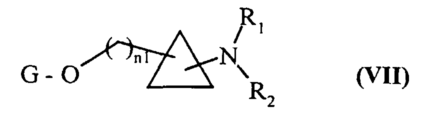 dans laquelle n₁, G, R₁ et R₂ sont tels que définis précédemment,
      composés de formule (VII) dont la fonction hydroxy est déprotégée puis qui sont :
      ◆ **soit** traités par un composé de formule **(VIII) :**

         Y - L₁ **(VIII)**

         dans laquelle Y est tel que défini dans la formule (I) et L₁ est tel que défini précédemment,
         pour conduire aux composés de formule **(I/a),** cas particulier des composés de formule (I) : 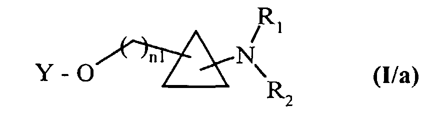 dans laquelle n₁, Y, R₁ et R₂ sont tels que définis dans la formule (I),
      ◆ **soit** mis à réagir avec du SOCl₂ pour conduire aux composés de formule **(IX) :** 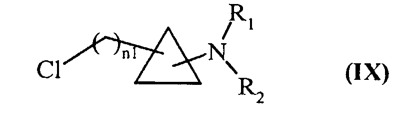 dans laquelle n₁, R₁ et R₂ sont tels que définis précédemment,
         composés de formule (IX) qui sont mis à réagir en milieu basique avec un composé de formule **(X) :**

         Y₁ - SH **(X)**

         dans laquelle Y₁ représente un groupement aryle, arylalkyle (C₁-C₆) linéaire ou ramifié, hétéroaryle ou hétéroarylalkyle (C₁-C₆) linéaire ou ramifié,
         pour conduire aux composés de formule **(I/b),** cas particulier des composés de formule (I) : 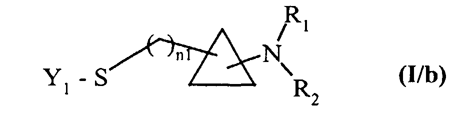 dans laquelle Y₁, n₁, R₁ et R₂ ont les mêmes significations que précédemment,
      ◆ **soit** soumis, dans le cas où n₁ prend la valeur 1, à l'action d'un agent oxydant classique de la synthèse organique pour conduire aux composés de formule **(XI) :** 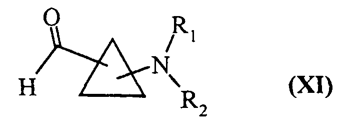 dans laquelle R₁ et R₂ sont tels que définis précédemment,
         composés de formule (XI) qui sont :
         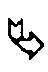 *soit* traités par une hydroxylamine de formule **(XII) :**

            H₂N - OY₂ **(XII)**

            dans laquelle Y₂ représente un atome d'hydrogène ou un groupement Y₁ tel que défini précédemment,
            pour conduire aux composés de formule **(I/c),** cas particulier des composés de formule (I) : 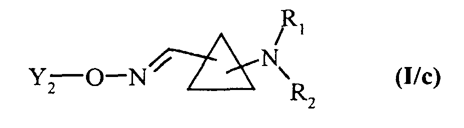 dans laquelle Y₂, R₁ et R₂ sont tels que définis précédemment,
            composés de formule (I/c) dans le cas particulier où Y₂ représente un atome d'hydrogène, qui sont soumis à l'action d'un composé de formule **(XIV) :**

            Y₃ - L₁ **(XIV)**

            dans laquelle L₁ est tel que défini précédemment et Y₃ représente un groupement de formule -C(O)-A ou -C(S)-A tels que définis dans la formule (I), pour conduire aux composés de formule **(I/d),** cas particulier des composés de formule (I) : 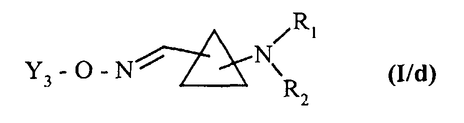 dans laquelle Y₃, R₁ et R₂ sont tels que définis précédemment,
         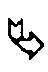 *soit* traités dans des conditions de réaction de Wittig puis soumis à l'action d'un agent réducteur classique de la synthèse organique, pour conduire aux composés de formule **(I/e),** cas particulier des composés de formule (I), 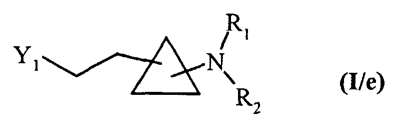 dans laquelle Y₁, R₁ et R₂ sont tels que définis précédemment,
         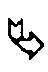 *soit* soumis à l'action d'un composé de formule Ph₃P=CH-CO₂Et puis réduit par action d'un agent réducteur de la synthèse organique pour conduire aux composés de formule **(XV) :** 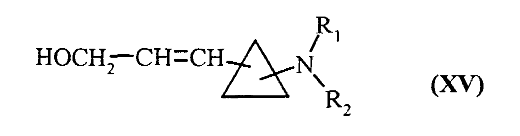
         dans laquelle R₁ et R₂ sont tels que définis précédemment,
         composés de formule (XV) qui sont traités par un composé de formule (VIII) tel que défini précédemment, pour conduire aux composés de formule **(I/f),** cas particulier des composés de formule (I) : 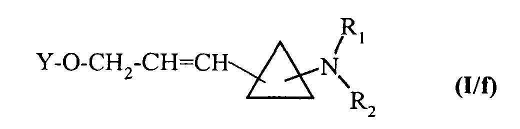 dans laquelle Y, R₁ et R₂ sont tels que définis dans la formule (I),

      ◆ **soit** transformés, dans le cas où n₁ prend la valeur 1, en leur dérivé halogéné correspondant selon des conditions usuelles de la chimie organique, puis mis à réagir avec un cyanure alcalin en présence de diméthylsulfoxyde, pour conduire aux composés de formule **(XVI) :** 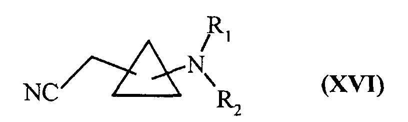 dans laquelle R₁, R₂ sont tels que définis précédemment,
         composés de formule (XVI) qui sont transformés en ester selon des conditions classiques puis soumis à un agent réducteur pour conduire aux composés de formule **(XVIIA) :** 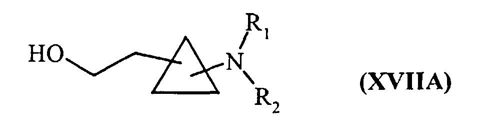 dans laquelle R₁, R₂ sont tels que définis précédemment,
         composés de formule (XVIIA) qui peuvent être soumis de nouveau, et de façon répétitive, à la même série de réactions ayant conduit aux composés de formule (XVI) et (XVIIA), pour conduire aux composés de formule **(XVIIB) :** 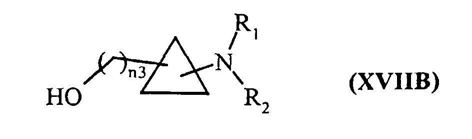 dans laquelle R₁, R₂ sont tels que définis précédemment et n₃ est un entier compris entre 3 et 6 inclus,
         l'ensemble des composés de formule (XVIIA) et (XVIIB) forment les composés de formule **(XVIII) :** 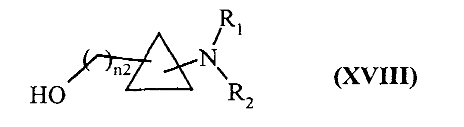 dans laquelle R₁ et R₂ sont tels que définis précédemment et n₂ est un entier compris entre 2 et 6 inclus,
         composés de formule (XVIII) qui sont :
         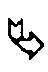 *soit* mis à réagir avec un composé de formule Y-L₁ tel que décrit précédemment, pour conduire aux composés de formule **(I/g),** cas particulier des composés de formule (I): 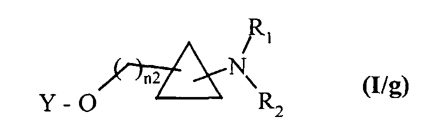 dans laquelle Y, n₂, R₁ et R₂ sont tels que décrits précédemment,
         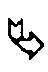 *soit* mis à réagir avec du SOCl₂ puis traités par un composé de formule (X) tel que décrit précédemment pour conduire aux composés de formule **(I/h),** cas particulier des composés de formule (I), 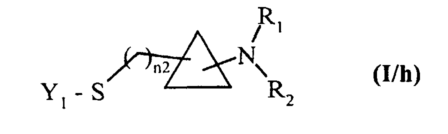 dans laquelle Y₁, n₂, R₁ et R₂ sont tels que décrits précédemment,
         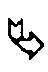 *soit* soumis à l'action d'un agent oxydant, l'aldéhyde intermédiaire obtenu étant alors mis à réagir avec une hydroxylamine de formule (XII) telle que décrite précédemment, et dans laquelle Y₂ représente spécifiquement un atome d'hydrogène puis si on le souhaite les composés obtenus sont soumis à l'action d'un composé de formule (XIV) telle que décrite précédemment, pour conduire aux composés de formule **(I/i),** cas particulier des composés de formule (I): 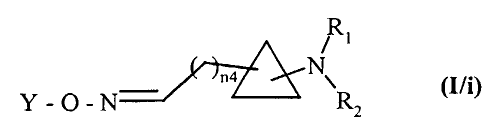 dans laquelle R₁, R₂ et Y sont tels que définis dans la formule (I) et n₄ représente un entier de valeur (n₂-1) avec n₂ tel que défini précédemment,
         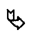 *soit* traités, après action d'un agent oxydant, dans des conditions de réaction de Wittig, puis traités dans des conditions de réduction classique de la synthèse organique, pour conduire aux composés de formule **(I/j),** cas particulier des composés de formule (I) : 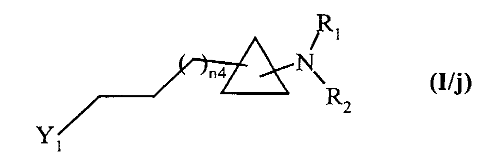 dans laquelle Y₁, n₄, R₁ et R₂ sont tels que décrits précédemment,
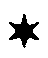 ***soit*** mis à réagir en présence de Me₃Al dans un solvant apolaire, avec un composé de formule **(XIX)** :

   HNR₁R₂ **(XIX)**

   dans laquelle R₁ et R₂ sont tels que définis dans la formule (I), pour conduire aux composés de formule **(XX) :** 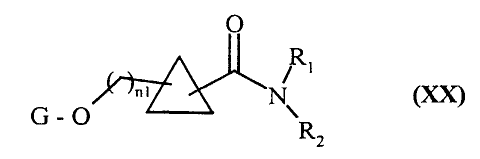 dans laquelle n₁, G, R₁ et R₂ sont tels que définis précédemment,
   composés de formule (XX) qui sont soumis à l'action d'un agent réducteur classiquement utilisé en synthèse organique, pour conduire aux composés de formule **(XXI) :** 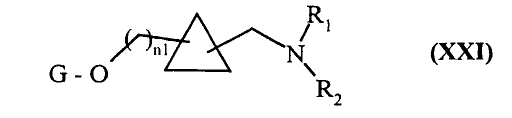 dans laquelle G, n₁, R₁ et R₂ sont tels que définis précédemment,
   composés de formule (XXI) qui peuvent être soumis à l'ensemble des réactions auxquelles sont soumis les composés de formule (VII), pour conduire aux composés de formule **(I/k),** cas particulier des composés de formule (I): 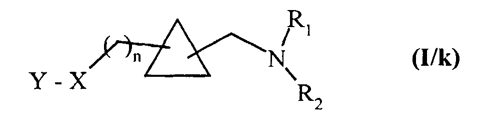 dans laquelle X, Y, n, R₁ et R₂ sont tels que définis dans la formule (I),
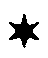 ***soit*** mis à réagir avec du chlorure de thionyle, dans le cas où R représente un atome d'hydrogène, puis mis en présence de diazométhane en milieu aqueux, pour conduire aux composés de formule **(XXII) :** 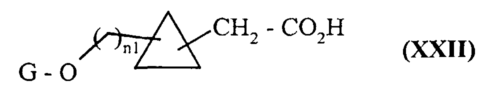 dans laquelle n₁ et G sont tels que définis précédemment,
   composés de formule (XXII) qui peuvent à nouveau subir plusieurs fois la même série de réaction, pour conduire aux composés de formule **(XXIII)** : 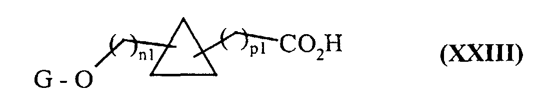 dans laquelle n₁ et G sont tels que définis précédemment, et p₁ représente un entier compris entre 2 et 6 inclus,
   composés de formule (XXIII) qui sont mis à réagir avec du diphénylphosphoryle azide, hydrolysés puis traités avec un composé de formule (VIA) tel que décrit précédemment, pour conduire aux composés de formule **(XXIV) :** 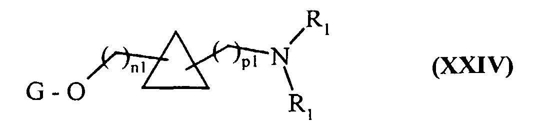 dans laquelle R₁ est tel que défini dans la formule (I) et G, n₁ et p₁ sont tels que définis précédemment,
   composés de formule (XXIV) qui peuvent être soumis à l'ensemble des réactions auxquelles sont soumis les composés de formule (VII), pour conduire aux composés de formule (**I/I),** cas particulier des composés de formule (I) : 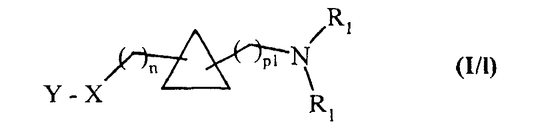 dans laquelle X, Y, R₁ et n sont tels que définis dans la formule (I) et p₁ est tel que défini précédemment,
   l'ensemble des composés de formule (I/a) à (I/l) formant l'ensemble des composés de l'invention qui sont purifiés, le cas échéant, selon une technique classique de purification, qui peuvent être séparés en leurs différents isomères, selon une technique classique de séparation et qui sont transformés, le cas échéant, en leurs sels d'addition à un acide ou à une base pharmaceutiquement acceptable.

On notera que dans le cas des composés de formule (XI), l'aldéhyde n'est pas stable lorsque R₁ et R₂ représentent chacun un groupement méthyle.

Selon une variante du procédé de préparation, certains composés de l'invention de formule (IA) peuvent être obtenus à partir des composés de formule **(a**_{**1**}**)** : 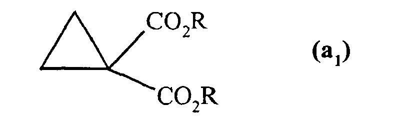 dans laquelle R représente un groupement alkyle (C₁-C₆) linéaire ou ramifié,
composés de formule (a₁) dont l'une des fonctions ester est saponifiée, puis qui sont soumis à l'action de l'azide diphénylphosphoryle et du méthanol ou du *tert*-butanol, en milieu polaire et aprotique, pour conduire aux composés de formule **(b**_{**1**}**)** : 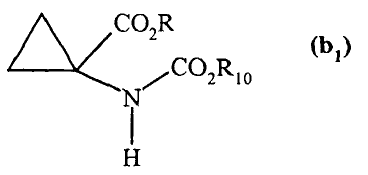 dans laquelle R est tel que défini précédemment, et R₁₀ représente un groupement méthyle ou *tert*-butyle,
composés de formule (b₁) dont la fonction carbamate est substituée par action d'un composé de formule **(c**_{**1**}**)** :

R₁ - Hal **(c**_{**1**}**)**

dans laquelle R₁ est tel que défini dans la formule (I) et Hal représente un atome d'halogène, pour conduire aux composés de formule (**d**_{**1**}) : 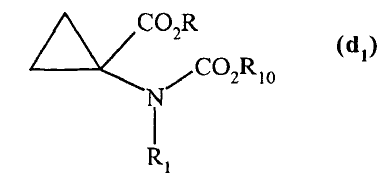 dans laquelle R, R₁ et R₁₀ sont tels que définis précédemment,
composés de formule (d₁) qui sont réduits pour conduire aux composés de formule **(e**_{**1**}**)** dans le cas où R₁₀ représente un groupement méthyle, et aux composés de formule **(e**_{**2**}**)** dans le cas où R₁₀ représente un groupement *tert*-butyle : 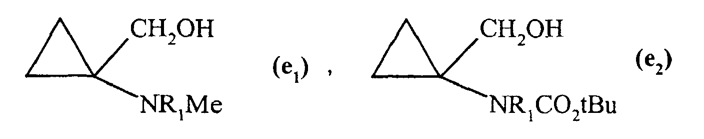 dans lesquelles R₁ est tel que défini précédemment,
composés de formule (e₂) dont on déprotège la fonction carbamate selon des méthodes classiques de la synthèse organique, pour conduire aux composés de formule **(e**_{**3**}**)** : 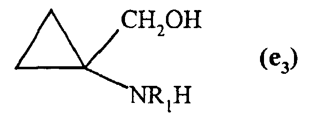 dans laquelle R₁ est tel que défini précédemment,
composés de formules (e₁) et (e₃) qui peuvent être soumis à l'une quelconque des réactions auxquelles sont soumis les composés de formule (VII) dans le procédé général de formation des composés de formule (I), pour conduire aux composés de formule **(I/m),** cas particulier des composés de formule (I): 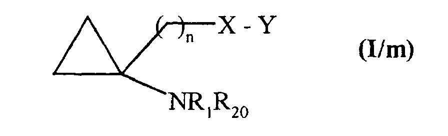 dans laquelle R₁, n, X et Y sont tels que définis dans la formule (I), et R₂₀ représente un groupement méthyle ou un atome d'hydrogène selon que le produit utilisé est de formule (e₁) ou (e₃).

Les composés de formule (II), (VIA), (VIB), (VIII), (X), (XII), (XIV), (XVIII), (a₁) et (c₁) sont soit des produits commerciaux, soit obtenus selon des méthodes classiques de la synthèse organique, bien connues de l'homme de l'art.

Les composés de formule (II), 1,2-disubstitués, peuvent notamment être obtenus à partir de dérivés hydroxyallylique ou hydroxyvinylique, dont la fonction hydroxy est protégée par un groupement protecteur classique de la synthèse organique. Ces composés de formule **(II/a) :** 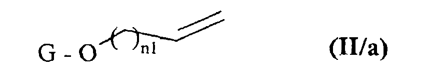 dans laquelle G est un groupement protecteur et n₁ représente 0 ou 1, sont mis à réagir, en présence de tétraacétate de rhodium, avec un composé de formule N₂CH₂CO₂R dans laquelle R représente un groupement alkyle (C₁-C₆) linéaire ou ramifié, pour conduire aux composés de formule **(II)** tels qu'attendus :

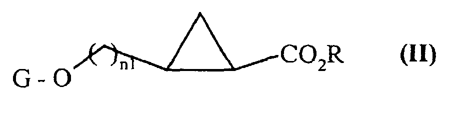

Concernant les méthodes classiques de protection et déprotection des fonctions hydroxy ou amino (groupements G, G₂ définis dans le procédé général d'obtention des composés de formule (I)), l'homme de l'art se reportera aisément au livre de T. W. Greene, "Protective Group in Organic Synthesis", Willey-Interscience, New-York, 1981.

D'une façon générale, on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon des techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur.

Les composés de l'invention, qui sont présents sous la forme d'un mélange de diastéréoisomères, sont isolés sous forme pure par l'utilisation des techniques classiques de séparation telles que les chromatographies.

Dans certains cas particuliers, le procédé de préparation des composés de l'invention peut conduire à la formation prédominante d'un énantiomère ou d'un diastéréoisomère par rapport à l'autre.

Les composés de la présente invention, de part leurs propriétés pharmacologiques de ligands nicotiniques, et sélectivement du sous-type réceptoriel α4β2, sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 1 mg à 500 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utile pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion donné ainsi que la microanalyse élémentaire correspondent à celui du produit salifié.

### PREPARATION A : Chlorhydrate de (±) cis 2-(diméthylamino)cyclopropanol

### Stade 1 : 2-(Vinyloxy)tétrahydro-2H-pyrane

Un mélange de 1,52 mol de 2-(2-chloroéthoxy)-tétrahydro-2*H*-pyrane, 93 g de soude réduite en poudre et 25 g de tétrabutyle ammonium monosulfate est agité 1 heure, puis distillé à 50°C sous 20 torr. Le distillat est ensuite séché sur sulfate de sodium permettant d'isoler le produit attendu.

### Stade 2 : 2-(Tétrahydro-2H-pyran-2-yloxy)cyclopropanecarboxylate d'éthyle

A une solution de 0,75 mol du composé obtenu au stade 1 dans 200 ml d'éther sont ajoutés 1,5 g d'acétate de rhodium puis en 6 heures une solution de 93 g de diazoacétate d'éthyle dans 50 ml d'éther. Après 16 heures d'agitation à température ambiante, le milieu réactionnel est filtré puis distillé à 50-90°C sous 0,5 torr. Le résidu obtenu est redistillé à 80-84°C sous 0,2 torr permettant d'isoler le produit attendu.

### Stade 3 : 2-(Tétrahydro-2H-pyran-2-yloxy)cyclopropanecarboxamide

Dans un autoclave, une solution de 0,25 mol du composé obtenu au stade 2, 2 g de cyanure de sodium, 300 ml d'une solution 2N d'ammoniaque dans le méthanol et 80 ml d'ammoniaque liquide est chauffée 5 jours à 65°C, puis concentrée à sec. Le résidu est repris par un mélange dichlorométhane/solution saturée en carbonate de potassium, filtré sur célite, puis traité de façon classique. Après évaporation sous pression réduite, le résidu est trituré dans l'éther de pétrole, filtré, rincé et séché permettant d'isoler le produit attendu.

### Stade 4 : 2-(Tétrahydro-2H-pyran-2-yloxy)cyclopropylamine

A une solution refroidie de 2,2 mol de soude dans 800 ml d'eau sont ajoutés 33 g de chlore, puis 52 g du composé obtenu au stade 3. Après retour à température ambiante, le milieu réactionnel est chauffé 16 heures à 65°C, puis refroidi à 20°C et saturé avec du carbonate de potassium. Après extraction à l'éther, les phases organiques réunies sont séchées puis concentrées sous pression réduite permettant d'isoler le produit attendu.

### Stade 5 : tert-Butyl 2-(tétrahydro-2H-pyran-2-yloxy)cyclopropylcarbamate

A une solution de 0,25 mol du composé obtenu au stade 4 dans 300 ml de dichlorométhane est ajoutée 0,65 mol de triéthylamine. Le milieu est refroidi à 0°C et 0,3 mol de di-*tert*-butyl dicarbonate dans 250 ml de dichlorométhane est additionnée en une heure. Après retour à température ambiante, le milieu réactionnel est agité 20 heures, puis 200 ml d'une solution saturée de carbonate de sodium sont additionnés. Après décantation, les phases organiques sont traitées de façon usuelle, puis concentrées sous pression réduite. Une chromatographie sur gel de silice du résidu (dichlorométhane/acétate d'éthyle : 95/5) permet d'isoler le produit attendu sous forme de mélange diastéréoisomérique.

### Stade 6 : cis tert-Butyl-N-méthyl[2-(tétrahydro-2H-pyran-2-yloxy)cyclopropyl] carbamate et trans tert-butyl-N-méthyl[2-tétrabydro-2H-pyran-2-yloxy)cyclopropyl]carbamate

A une solution refroidie à 0°C de 4,4 g d'hydrure de sodium dans 250 ml de diméthylformamide sont ajoutés 26 g du composé obtenu au stade 5 dans 20 ml de diméthylformamide. Après retour à température ambiante, le milieu est agité 2 heures puis 15,6 g d'iodure de méthyle sont additionnés en 15 minutes. Après 16 heures d'agitation, le milieu réactionnel est concentré, repris par un mélange éther/solution saturée de carbonate de sodium. Les phases organiques sont ensuite traitées de façon usuelle puis concentrées. Une chromatographie sur gel de silice (cyclohexane/tétrahydrofurane) permet d'isoler l'isomère *trans* puis l'isomère *cis* du produit attendu.

### Stade 7 : cis N,N-Diméthyl-2-(tétrahydro-2H-pyran-2-yloxy)cyclopropanamine

A une solution refroidie à 0°C de 7,5 g de l'isomère *cis* obtenu au stade précédent dans 75 ml de tétrahydrofurane sont additionnés 46 ml de Red-Al® à 65 % dans le toluène. Le milieu réactionnel est agité 2 heures à 0°C, 16 heures à température ambiante, puis refroidi à 0°C et hydrolysé par 100 ml d'eau distillée. Après extraction à l'éther, les phases organiques réunies sont traitées de façon usuelle puis concentrées. Une chromatographie sur gel de silice du résidu (dichlorométhane/tétrahydrofurane : 90/10) permet d'isoler le produit attendu.

### Stade 8 : Chlorhydrate de (±) cis 2-(diméthylamino)cyclopropanol

A 1,8 g du composé obtenu au stade 7 dans 30 ml d'éther sont additionnés, sous atmosphère inerte, 6 ml d'une solution 4N d'acide chlorhydrique dans le dioxane. Après 16 heures à température ambiante, le milieu est filtré, rincé à l'éther, puis séché sous pression réduite permettant d'isoler le produit attendu.
**Point de fusion** **: 160-162°C**

### PREPARATION B : Chlorhydrate de (±) trans 2-(diméthylamino)cyclopropanol

### Stade 1 : trans N,N-Diméthyl-2-(tétrahydro-2H-pyran-2-yloxy) cyclopropanamine

Le produit est obtenu selon le procédé du stade 7 de la préparation A en utilisant comme substrat l'isomère *trans* obtenu au stade 6 de la préparation A.

### Stade 2 : Chlorhydrate de (±) trans 2-(diméthylamino)cyclopropanol

Le produit est obtenu selon le procédé du stade 8 de la préparation A en utilisant comme substrat le composé obtenu au stade 1. Le produit recristallise dans l'acétonitrile.
**Point de fusion** **: 118-120°C**

### PREPARATION C : (±) trans Méthyl 2-[(benzyloxy)méthyl]cyclopropyl (méthyl)carbamate

### Stade 1 : 2-[(Benzyloxy)méthyl]cyclopropanecarboxylate d'éthyle

A une solution de 0,3 mol d'allylbenzyléther dans 150 ml d'éther sont ajoutés à l'aide d'un pousse-seringue 31,2 g d'éthyle diazoacétate. Après 16 heures d'agitation, 31,2 g d'éthyle diazoacétate sont à nouveau ajoutés. Après 24 heures, le milieu est filtré. La phase organique est lavée par une solution saturée de NaHCO₃ puis traitée de façon usuelle. Une chromatographie sur gel de silice (dichlorométhane) permet d'isoler le produit attendu.

### Stade 2 : Acide 2-[(benzyloxy)méthyl]cyclopropanecarboxylique

Une solution de 0,23 mol du composé obtenu au stade 1 dans 500 ml d'éthanol, 230 ml de soude 1N et 5 ml de diméthylsulfoxyde est portée 2 heures à reflux puis concentrée. Le résidu est repris par un mélange eau/éther. Après traitement classique, les phases organiques réunies sont concentrées. Une chromatographie sur gel de silice du résidu (dichlorométhane/tétrahydrofurane : 97/3) permet d'isoler un mélange *cis*/*trans* des produits obtenus dont certaines fractions sont enrichies en isomère *cis* et d'autres en isomère *trans*.

### Stade 3 : (±) trans Méthyl 2-[(benzyloxy)méthyl]cyclopropylcarbamate

A 11 g de l'isomère *trans* du produit obtenu au stade 2 dans 100 ml de toluène, et 5,4 g de triéthylamine sont additionnés 14,7 g de diphénylphosphoryle azide. Le milieu est chauffé 2 h 30 à 80°C, puis 2,6 g de méthanol sont ajoutés. Après 16 heures à 80°C, le milieu réactionnel est refroidi, lavé par une solution saturée en NaHCO₃, séché puis concentré. Une chromatographie sur gel de silice du résidu (dichlorométhane) permet d'isoler le produit attendu dans un rapport diastéréoisomèrique *trans*/*cis* : 94/6.

### Stade 4 : (±) trans Méthyl 2-[(benzyloxy)méthyl]cyclopropyl(méthyl)carbamate

A une solution refroidie à 0°C de 10,2 g du composé obtenu au stade 3 dans 150 ml de diméthylformamide sont additionnés, par fractions, 2,1 g d'hydrure de sodium. Après 30 minutes à 0°C, puis 24 heures à température ambiante, 7,24 g d'iodure de méthyle sont additionnés et l'agitation est maintenue 72 heures. Après évaporation du solvant, le résidu est repris à l'éther, lavé par une solution saturée de NaHCO₃ puis par une solution à 10 % de chlorure de lithium. Après traitement usuel, une chromatographie sur gel de silice du résidu (dichlorométhane) permet d'isoler le produit attendu.

### PREPARATION D : (±) cis Méthyl 2-[(benzyloxy)méthyl]cyclopropyl (méthyl)carbamate

### Stade 1 : (±) cis Méthyl 2-[(benzyloxy)méthyl]cyclopropylcarbamate

Le produit est obtenu selon le procédé du stade 3 de la préparation C en utilisant comme substrat l'isomère *cis* obtenu au stade 2 de la préparation C. Le produit est isolé dans un rapport diastéréoisomérique cis/trans : 77/23.

### Stade 2 : (±) cis Méthyl 2-[(benzyloxy)méthyl]cyclopropyl(méthyl)carbamate

Le produit est obtenu selon le procédé du stade 4 de la préparation C en utilisant comme substrat le produit obtenu au stade 1.

### PREPARATION E : Chlorhydrate de trans 2-[(diméthylamino)méthyl]cyclopropanol

### Stade 1 : N,N-Diméthyl-2-(tétrahydro-2H-pyran-2-yloxy)cyclopropane carboxamide

A une solution de 70 ml de triméthylaluminium 2M dans le toluène, et 250 ml de toluène est additionnée à -15°C, 6,1 g de diméthylamine. Après 20 minutes, le milieu est amené à température ambiante puis après 1 h 30, 26,3 g du composé obtenu au stade 2 de la préparation A dans 75 ml de toluène sont ajoutés. Le milieu réactionnel est ensuite chauffé 16 heures à 85°C, puis refroidi par un bain de glace, additionné de 270 ml d'une solution 0,5 N d'acide chlorhydrique puis filtré et décanté. Les phases organiques réunies sont traitées de façon usuelle puis concentrées sous pression réduite permettant d'isoler le produit attendu dans un rapport diastéréoisomérique *trans*/*cis* de 80/20.

### Stade 2 : trans N,N-Diméthyl-N-{[2-(tétrahydro-2H-pyran-2-yloxy) cyclopropyl]méthyl} amine

A une suspension de 3,1 g d'AlLiH₄ dans 120 ml d'éther sont additionnés lentement 14 g du composé obtenu au stade 1 dans 50 ml d'éther. Après 16 heures au reflux, le milieu réactionnel est refroidi à 0°C, hydrolysé, filtré, puis concentré sous pression réduite. Une chromatographie sur gel de silice du résidu (dichlorométhane/méthanol : 90/10) permet d'isoler le produit attendu dans un rapport diastéréoisomérique *trans*/*cis* de 99/1.

### Stade 3 : Chlorhydrate de trans 2-[(diméthylamino)méthyl]cyclopropanol

Le produit est obtenu selon le procédé du stade 8 de la préparation A en utilisant comme substrat le composé obtenu au stade 2 et en ajoutant de l'éthanol au milieu réactionnel.
**Point de fusion** **: 93-96°C**

### PREPARATION F : [1-(Diméthylamino)cyclopropyl]méthanol

### Stade 1 : Acide 1-(méthoxycarbonyl)cyclopropanecarboxylique

Sur une solution refroidie à 5°C de 1,45 mol de 1,1-cyclopropanedicarboxylate de méthyle dans 2,5 l de méthanol sont additionnés 1,45 l de soude 1N. Après 4 jours d'agitation à température ambiante, le milieu est concentré au trois-quart, extrait à l'éther puis traité de façon usuelle permettant d'isoler le produit attendu.

### Stade 2 : 1-[(Méthoxycarbonyl)amino]cyclopropanecarboxylate de méthyle

Sur une solution portée à 80°C de 1,09 mol du composé obtenu au stade 1 dans 1,09 l de toluène et 153 ml de triéthylamine sont additionnés 300 g de diphénylphosphoryle azide. La réaction est fortement exothermique. A la fin de tout dégagement gazeux, la réaction est refroidie à 50°C, 66,3 ml de méthanol sont ajoutés, puis le milieu est de nouveau chauffé à 70°C pendant 2 heures. Après refroidissement et traitement classique, une chromatographie sur gel de silice (dichlorométhane/méthanol: 97/3) permet d'isoler le produit attendu.

### Stade 3 : 1-[(Méthoxycarbonyl)(méthyl)amino]cyclopropanecarboxylate de méthyle

A une solution refroidie à 5°C de 99,7 g du composé obtenu au stade 2 dans 1,7 l de diméthylformamide anhydre sont ajoutés par fractions 24,7 g d'hydrure de sodium. Après 15 minutes à 5°C puis 3 heures à température ambiante, 38,2 ml d'iodure de méthyle sont additionnés goutte à goutte. Après 20 heures de réaction, le milieu est évaporé. Le résidu est repris dans l'éther puis traité de façon classique. Une chromatographie sur gel de silice (dichlorométhane) permet d'isoler le produit attendu.

### Stade 4 : [1-(Diméthylamino)cyclopropyl]méthanol

Sur une solution de 44 g de LiAlH₄ dans 1,05 l de tétrahydrofurane sont additionnés en 30 minutes, 44 g du composé obtenu au stade 3 dissous dans 350 ml de tétrahydrofurane. Après 20 heures de reflux, le milieu est refroidi à 5°C et 44 ml d'eau, 44 ml de soude 4N puis 132 ml d'eau sont ajoutés. Une filtration suivie d'une concentration sous pression réduite permet d'isoler le produit attendu.
**Point de fusion** **: < 50°C**

### PREPARATION G : Chlorhydrate de 1-[(diméthylamino)méthyl]cyclopropanol

### Stade 1 : 1-Hydroxy-N,N-diméthylcyclopropanecarboxamide

A une solution de 6 g d'acide 1-hydroxycyclopropane carboxylique dans 120 ml de dichlorométhane et 10,5 ml de pyridine sont additionnés goutte à goutte 17,1 ml de chlorure de triméthylsilyle. Après 4 heures d'agitation à température ambiante, le milieu est refroidi à 0°C, et 10 gouttes de diméthylformamide puis 5,4 ml de chlorure d'oxalyle sont additionnés. La réaction est agitée 1 heure à 0°C, 1 heure à température ambiante, puis une solution de 2,4 g de diméthylamine dans 10 ml de pyridine est ajoutée. L'agitation est maintenue 20 heures, puis après refroidissement à 0°C, 14 g d'acide citrique dans 120 ml de méthanol sont ajoutés. Après retour à température ambiante pendant une heure, le milieu réactionnel est lavé par une solution d'acide chlorhydrique 1N, puis par une solution saturée en NaHCO₃ puis en NaCl. La phase organique est traitée de façon classique et une chromatographie sur gel de silice (dichlorométhane/tétrahydrofurane : 90/10) permet d'isoler le produit attendu.

### Stade 2 : Chlorhydrate de 1-[(diméthylamino)méthyl]cyclopropanol

A une solution de 0,9 g de AlLiH₄ dans 30 ml d'éther sont additionnés lentement 1,5 g du composé obtenu au stade 1 dans 20 ml d'éther. Après 5 heures de reflux, le milieu réactionnel est refroidi et hydrolysé avec de la glace. La phase aqueuse est décantée, saturée avec du carbonate de potassium et extraite au dichlorométhane. Les phases organiques sont séchées puis concentrées sous pression réduite. Le résidu est repris par 25 ml d'éther et 2 ml d'une solution 4N d'acide chlorhydrique dans le dioxane. Le précipité est filtré permettant d'isoler le produit attendu.

### PREPARATION H : Chlorhydrate de 2-[1-(diméthylamino)cyclopropyl]éthanol

### Stade 1 : Chlorhydrate de 1-(chlorométhyl)-N,N-diméthyleyclopropanamine

A une solution de 18,4 g du composé de la préparation F dans 240 ml d'éther sont ajoutés 80 ml d'éther chlorhydrique. Le précipité obtenu est filtré, rincé à l'éther et séché puis dilué dans 320 ml de toluène auxquels on ajoute goutte à goutte 32 ml de chlorure de thionyle. Après 3 heures à 60°C, le milieu est refroidi à 5°C, filtré, lavé au toluène et séché permettant d'isoler le produit attendu.
**Point de fusion** **: 198°C**

### Stade 2 : [1-(Diméthylamino)cyclopropyl]acetonitrile

A une solution de 43 g de cyanure de sodium, 3,5 g d'iodure de potassium dans 400 ml de diméthylsulfoxyde sont ajoutés 30,3 g du composé obtenu au stade 1. Après 20 heures d'agitation à température ambiante, 490 ml d'une solution aqueuse de carbonate de sodium à 10 % puis du chlorure de sodium sont ajoutés. Le milieu est extrait à l'éther. Après traitement classique des phases organiques, une chromatographie sur gel de silice permet d'isoler le produit attendu.

### Stade 3 : Méthyl [1-(diméthylamino)cyclopropyl]acétate

A une solution refroidie à 5°C de 14,5 g du composé obtenu au stade 2 dans 230 ml de méthanol anhydre sont ajoutés 40 ml de méthanol chlorhydrique anhydre 2N, puis de l'acide chlorhydrique gazeux jusqu'à saturation. Le milieu est agité 20 heures à température ambiante puis évaporé. Le résidu est repris par une solution de carbonate de sodium et extrait au dichlorométhane. Après traitement classique des phases organiques, une chromatographie sur gel de silice (dichlorométhane/tétrahydrofurane : 95/5) permet d'isoler le produit attendu.

### Stade 4 : Chlorhydrate de 2-[1-(diméthylamino)cyclopropyl]éthanol

A une solution de 5 g de AlLiH₄ dans 300 ml de tétrahydrofurane sont additionnés lentement 10,2 g du composé obtenu au stade 3 dissous dans 200 ml de tétrahydrofurane. Après 2 heures de reflux, le milieu réactionnel est refroidi à 5°C et 10,7 ml d'eau, 10,7 ml de soude 4N puis 32,1 ml d'eau sont additionnés. Après filtration et concentration sous pression réduite, une chromatographie sur gel de silice (dichlorométhane/méthanol: 95/5) permet d'isoler le produit attendu qui est transformé en son chlorhydrate par action d'une solution d'acide chlorhydrique dans le dioxane.

### PREPARATION I : tert-Butyl 1-(hydroxyméthyl)cyclopropyl(méthyl)carbamate

### Stade 1 : 1-[(tert-Butoxycarbonyl)amino]cyclopropanecarboxylate de méthyle

Une solution de 80 g d'acide 1-(méthoxycarbonyl)cyclopropanecarboxylique, 78 ml de triéthylamine dans 550 ml de toluène, additionnée de 152 g de diphénylphosphoryle azide est chauffée à 80°C. Après arrêt de dégagement gazeux, la température est ramenée à 50°C et 61 g de *tert*-butanol sont ajoutés. Après 7 heures de réaction à 80°C, le milieu est concentré. Le résidu est repris à l'éther, lavé par une solution saturée en Na₂CO₃ puis par une solution d'acide chlorhydrique 1N, puis par une solution de NaHCO₃. Après séchage, et évaporation de la phase organique, le résidu est repris par 300 ml de cyclohexane, puis concentré à sec. Le résidu obtenu est trituré dans le pentane, filtré puis séché permettant d'isoler le produit attendu.

### Stade 2 : 1-[(tert-Butoxycarbonyl)(méthyl)amino]cyclopropanecarboxylate de méthyle

Le produit est obtenu selon le procédé du stade 3 de la préparation F en utilisant comme substrat le composé obtenu au stade 1.

### Stade 3 : tert-Butyl 1-(hydroxyméthyl)cyclopropyl(méthyl)carbamate

A une solution de 23 g du composé obtenu au stade 2 dans 100 ml de tétrahydrofurane est additionnée une solution de 100 ml de borohydrure de lithium 2M dans le tétrahydrofurane. Après 20 heures d'agitation à température ambiante, puis 8 heures au reflux, le milieu réactionnel est refroidi à 0°C, hydrolysé, dilué à l'éther, décanté, séché et concentré. Une chromatographie sur gel de silice du résidu (dichlorométhane/tétrahydrofurane: 95/5) permet d'isoler le produit attendu.

### PREPARATION J : 1-(2-Chloroéthyl)-N,N-diméthylcyclopropanamine

Le produit est obtenu selon le procédé du stade 1 de la préparation H en utilisant comme substrat le composé de la préparation H.

### PREPARATION K : tert-butyl 1-(hydroxyméthyl)cyclopropylcarbamate

Le produit est obtenu selon le procédé du stade 3 de la préparation I en utilisant comme substrat le composé obtenu au stade 1 de la préparation I.
**Point de fusion :** **80-82°C**

### PREPARATION L : 3-[1-(Diméthylamino)cyclopropyl]-1-propanol

### Stade 1 : tert-butyl 1-formylcyclopropyl(méthyl)carbamate

Une solution de 8,3 g de diméthylsulfoxyde dans 25 ml de dichlorométhane est ajouté, à -60°C à une solution contenant 6,2 g de chlorure d'oxalyle dans 110 ml de dichlorométhane. Après 20 minutes, 8,9 g du composé de la préparation I dilués dans du dichlorométhane, et enfin après 30 minutes de réaction, 20 ml de triéthylamine sont ajoutés au milieu réactionnel. Après retour à température ambiante, 50 ml d'eau sont ajoutés et le milieu subit un traitement usuel. Une chromatographie sur gel de silice du résidu (dichlorométhane/tétrahydrofurane : 97/3) permet d'isoler le produit attendu.

### Stade 2 : 3-{1-[(tert-butoxycarbonyl)(méthyl)amino]cyclopropyl}-2-propénoate d'éthyle

1,15 g du produit obtenu au stade 1 et 2,01 g de (carboéthoxyméthylène) triphénylphosphorane dans 30 ml de dichlorométhane sont portés 20 heures au reflux, concentrés sous pression réduite, puis chromatographiés sur gel de silice (dichlorométhane/tétrahydrofurane : 97/3) permettant d'isoler le produit attendu.
**Point de fusion** **: 52°C**

### Stade 3 : 3-{1-[(tert-butoxycarbonyl)(méthyl)amino] cyclopropyl}propanoate d'éthyle

8,0 g du produit obtenu au stade 2 dissous dans 200 ml d'éthanol sont hydrogénés pendant 4 heures à 20°C et sous 5 bars, en présence de 1,0 g de Pd/C à 10 %. Après filtration et concentration à sec, une chromatographie sur gel de silice (dichlorométhane/tétrahydrofurane : 97/3) permet d'isoler le produit attendu (liquide).

### Stade 4 : 3-[1-(Diméthylamino)cyclopropyl]-1-propanol

8,5 g du produit obtenu au stade 3 dissous dans 100 ml de tétrahydrofurane sont additionnés sur 6,9 g d'hydrure de lithium aluminium en suspension dans 200 ml de tétrahydrofurane. Après 16 heures de reflux, le milieu est refroidi à 5°C et hydrolysé successivement par 6,9 ml d'eau, 6,9 ml de soude 4N puis 20,7 ml d'eau. Après traitement usuel, une chromatographie du résidu sur gel de silice permet d'isoler le produit attendu (liquide).

### PREPARATION M : tert-butyl 1-(bromométhyl)cyclopropyl(méthyl)carbamate

A une solution de 4 g du composé de la préparation I dans 100 ml d'éther sont additionnés à 20°C 7,9 g de triphénylphosphine puis 9,9 g de tetrabromométhane. Après 24 heures d'agitation, filtration et concentration à sec, une chromatographie sur gel de silice (dichlorométhane) permet d'isoler le produit attendu.
**Point de fusion** **: 62-64°C**

### PREPARATION N : tert-butyl (2E)-3-(3-pyridinyl)-2-propénoate

A une solution d'acide de 45 g (2*E*)-3-(3-pyridinyl)-2-propénoïque dans 300 ml d'éther est ajouté de l'éther chlorhydrique jusqu'à pH acide. Le précipité obtenu est filtré, lavé à l'éther et séché sous pression réduite. Le produit est alors repris dans 600 ml de chlorure de thionyle, porté au reflux 2 heures, puis concentré sous presion réduite, et repris successivement dans le toluène puis dans l'éther. Le résidu obtenu est alors dilué dans 1 litre de tétrahydrofurane, agité à 5°C et 67 g de *tert-*butylate de potassium dans 2,5 l de tétrahydrofurane sont ajoutés. Après 3 heures de réaction à 5°C et 1 heure à 20°C, le milieu réactionnel suit un traitement usuel permettant d'obtenir après évaporation sous pression réduite, le produit attendu.
**Point de fusion** **: huile**

### PREPARATION Q : Chlorure de triphényl (3-pyridinylméthyl)phosphonium

13,1 g de triphényphosphine sont ajoutés à une solution de 8,2 g de chlorhydrate de chlorure de 3-picolyle dans 120 ml de diméthylformamide. Après 5 minutes d'agitation, le milieu est porté 30 minutes au reflux dans un appareil à microondes, puis refroidi à 5°C. Les cristaux obtenus sont filtrés, rincés au diméthylformamide puis à l'éther, et séchés sous pression réduite permettant d'isoler le produit attendu.
**Point de fusion** **: 315-318°C**

### PREPARATION P : [1-(1-Pyrrolidinyl)cyclopropyl]méthanol

### Stade 1 : 1-(1-Pyrrolidinyl)cyclopropanecarbonitrile

Un mélange contenant une goutte de chlorure de triméthylsilyle et 67,8 g de [(1-éthoxycyclopropyl)oxy]-triméthylsilane dans 65 ml de méthanol est ajouté à 62,6 g de chlorhydrate de pyrrolidine et 28,7 g de cyanure de potassium dissous dans 260 ml de méthanol. Après 20 minutes d'agitation à 20°C, puis 20 heures à 50°C, le milieu est concentré à sec, repris dans le dichlorométhane, filtré et concentré à nouveau à sec. Le résidu est chromatographié sur gel de silice (CH₂Cl₂) permettant d'isoler le produit souhaité.

### Stade 2 : 1-(1-Pyrrolidinyl)cyclopropanecarboxamide

On fait buller à 20°C un courant d'acide chlorhydrique gazeux pendant 5 heures dans un mélange contenant 5,2 g du produit obtenu au stade 1 dissous dans 100 ml de méthanol. On concentre à sec, reprend dans 20 ml de solution aqueuse de carbonate de sodium et 20 ml de dichlorométhane. Après traitement usuel, une chromatographie sur gel de silice permet d'isoler le produit attendu.
**Point de fusion** **: 62°C**

### Stade 3 : 1-(1-pyrrolidinyl)cyclopropanecarboxylate d'éthyle

Une solution contenant 11,4 g de tétrafluoroborate de triéthyloxonium et 40 ml de dichlorométhane est ajoutée goutte à goutte à 0°C dans une suspension contenant 1,54 g du produit obtenu au stade 2, 10 g de phosphate disodique et 120 ml de dichlorométhane. Après 20 heures à 20°C, 40 ml de solution aqueuse de carbonate disodique à 10 % sont ajoutés. On décante et extrait la phase aqueuse avec du dichlorométhane. Les phases organiques jointes sont séchées sur sulfate de sodium puis concentrées à sec. On reprend le résidu dans 20 ml d'acide chlorhydrique aqueux 1N et on laisse la solution ainsi obtenue à 20°C pendant 3 heures. On alcalinise par du carbonate disodique et on extrait au dichlorométhane. On sèche les phases dichlorométhane jointes sur sulfate de sodium et on concentre à sec. Une chromatographie sur gel de silice (dichlorométhane/tétrahydrofurane : 95/5) permet d'isoler le produit souhaité.

### Stade 4 : [1-(1-Pyrrolidinyl)cyclopropyl]méthanol

On coule en 5 minutes 20,5 g du produit obtenu au stade 3 et 100 ml de tétrahydrofurane dans une suspension contenant 8,57 g d'hydrure de lithium aluminium et 400 ml de tétrahydrofurane. Le milieu est porté au reflux 2 heures, refroidi à 5°C et hydrolysé par ajouts successifs de 9 ml d'eau, 9 ml de soude 4N puis 27 ml d'eau. On filtre l'alumine et rince au tétrahydrofurane. Les filtrats joints sont concentrés à sec permettant d'obtenir le produit souhaité (liquide).

### PREPARATION O : tert-butyl 1-(2-hydroxyéthyl)cyclopropyl(méthyl)carbamate

### Stade 1 : tert-butyl 1-(bromométhyl)cyclopropyl(méthyl)carbamate

100,1 g de triphénylphosphine puis 125,5 g de tétrabromométhane sont ajoutés à une solution contenant 50,7 g du produit de la préparation I dans 1,25 l d'éther. On agite 20 heures à 20°C, filtre l'insoluble et concentre à sec. On chromatographie le résidu sur gel de silice (CH₂Cl₂) ce qui permet d'isoler le produit attendu.
**Point de fusion** : **53°C**

### Stade 2 : tert-butyl 1-(cyanométhyl)cyclopropyl(méthyl)carbamate

On agite à 70°C pendant 20 heures un mélange contenant 26,4 g du produit du stade 1, 26 g de cyanure de potassium et 2,6 g d'iodure de potassium dans 264 ml de diméthylsulfoxyde. On refroidit et ajoute 400 ml de carbonate de sodium à 10 % dans l'eau. Après extraction à l'éther et traitement usuel, une évaporation sous pression réduite du résidu permet d'isoler le produit attendu.

### Stade 3 : [1-(Méthylamino)cyclopropyl]acétate de méthyle

On ajoute 89 ml de méthanol chlorhydrique 4N dans une solution contenant 22,2 g du produit obtenu au stade 2 dissous dans 450 ml de méthanol. On fait buller 200 g d'acide chlorhydrique gazeux à température inférieure à 40°C. On abandonne 20 heures à température ambiante, puis concentre à sec et reprend le résidu dans 80 ml de carbonate de sodium à 10 %. Après traitement usuel, une chromatographie sur gel de silice (CH₂Cl₂/éthanol : 97/3) permet d'isoler le produit souhaité.

### Stade 4 : {1-[(tert-butoxycarbonyl)(méthyl)amino]cyclopropyl}acétate de méthyle

On ajoute goutte à goutte à 5°C une solution contenant 17,5 g de diterbutyldicarbonate et 120 ml de dichlorométhane dans une solution contenant 9,5 g du produit obtenu au stade 3 et 0,88 g de (3-diméthylamino)pyridine dans 120 ml de dichlorométhane. Après 2 heures à 5°C, puis 2 heures à 20°C, le milieu est lavé avec une solution aqueuse de chlorure d'ammonium puis avec une solution à 10 % d'hydrogénocarbonate de sodium. Après traitement usuel, une évaporation sous pression réduite permet d'obtenir le produit souhaité.

### Stade 5 : tert-butyl 1-(2-hydroxyéthyl)cyclopropyl(méthyl)carbamate

On coule à 20°C en 20 minutes, 20 ml de borohydrure de lithium 2M dans le tétrahydrofurane dans un mélange contenant 4,86 g du produit obtenu au stade 4 et 20 ml de tétrahydrofurane. Après 20 heures d'agitation à température ambiante puis 1 heure au reflux, le milieu est refroidi à 5°C puis hydrolysé par un mélange contenant 8 ml d'eau et 4 ml de solution aqueuse de carbonate de sodium à 10 %. On extrait plusieurs fois la phase aqueuse à l'éther. Après traitement usuel, une chromatographie du résidu sur gel de silice (CH₂Cl₂/tétrahydrofurane : 98/2) permet d'isoler le produit attendu (liquide).

### PREPARATION R : 2-[1-(1-Pyrrolidinyl)cyclopropyl]éthanol

### Stade 1 : tert-butyl 1-(bromométhyl)cyclopropylcarbamate

Le produit est obtenu selon le procédé de la préparation M en utilisant comme substrat le produit de la préparation K.
**Point de fusion** **: 96°C**

### Stade 2 : tert-butyl 1-(cyanométhyl)cyclopropylcarbamate

Le produit est obtenu selon le procédé de la préparation H stade 2, en utilisant comme substrat le produit obtenu précédemment.
**Point de fusion** **: 89°C**

### Stade 3 : [1-(Amino)cyclopropyl]acétate de méthyle

Le produit est obtenu selon le procédé de la préparation H stade 3, en utilisant comme substrat le produit obtenu précédemment.

### Stade 4 : [1-(2,5-Dioxo-1-pyrrolidinyl)cyclopropyl]acétate de méthyle

Une solution contenant 1,29 g du produit obtenu au stade précédent et 1 g d'anhydride succinique dans 30 ml de toluène, est portée au reflux pendant 1 heure, puis 2,8 ml de triéthylamine sont ajoutées. Après 20 heures de reflux, le milieu est concentré à sec. Le résidu est alors repris au toluène, puis évaporé sous pression réduite. Une chromatographie sur gel de silice (dichlorométhane/tétrahydrofurane : 97/3) permet d'isoler le produit attendu.

### Stade 5 : 2-[1-(1-Pyrrolidinyl)cyclopropyl]éthanol

Le produit est obtenu selon le procédé de la préparation L stade 4, en utilisant comme substrat le produit obtenu précédemment (liquide).

### PREPARATION S : tert-butyl méthyl[1-(2-oxoéthyl)cyclopropyl]carbamate

Le produit est obtenu selon le procédé de la préparation L stade 1, en utilisant comme substrat le produit de la préparation Q.
**Point de fusion** **: 93°C**

### EXEMPLE 1 : Chlorhydrate de (±) cis-2-(diméthylamino)cyclopropyl acétate

Une solution, sous atmosphère inerte, de 0,28 g du composé de la préparation A dans 4 ml d'acide acétique, additionnée de 0,22 g de chlorure d'acétyle est agitée 16 heures à température ambiante puis concentrée. Le résidu est repris par 10 ml de dioxane et concentré à sec de nouveau. L'opération est répétée jusqu'à cristallisation. Les cristaux obtenus sont dilués dans 10 ml d'éther, filtrés et séchés permettant d'isoler le produit attendu.
**Point de fusion** **: amorphe**

| **Microanalyse élémentaire :** | | | | |
|---|---|---|---|---|
| | **C** | **H** | **N** | **Cl** |
| **% calculé** | 46,11 | 7,90 | 7,68 | 19,44 |
| **% trouvé** | 46,01 | 7,70 | 7,48 | 19,63 |

### EXEMPLE 2 : Chlorhydrate de (±) cis 2-(diméthylamino)cyclopropyl méthylcarbamate

Une solution de 0,4 g du composé de la préparation A dans 30 ml d'acétonitrile et 0,25 g d'isocyanate de méthyle est chauffée 8 heures à 80°C, puis évaporée. Le résidu est repris par un mélange eau/éther, et saturé par du carbonate de potassium. La phase organique est traitée de façon classique et le résidu est chromatographié sur gel de silice (dichlorométhane/méthanol: 97/3). Le composé obtenu est transformé en son chlorhydrate de façon usuelle. Le produit obtenu cristallise dans un mélange de 5 % d'acétonitrile dans l'éther.
**Point de fusion** **: 105-110°C**

| **Microanalyse élémentaire :** | | | | |
|---|---|---|---|---|
| | **C** | **H** | **N** | **Cl** |
| **% calculé** | **41,65** | **7,89** | **13,88** | **17,56** |
| **% trouvé** | **41,68** | **7,88** | **13,93** | **18,25** |

### EXEMPLE 3 : Chlorhydrate de (±) trans 2-(diméthylamino)cyclopropyl acétate

Le produit est obtenu selon le procédé de l'exemple 1 en utilisant comme substrat le composé de la préparation B.
**Point de fusion** **: 148-150°C**

| **Microanalyse élémentaire :** | | | | |
|---|---|---|---|---|
| | **C** | **H** | **N** | **Cl** |
| **% calculé** | **46,80** | **7,85** | **7,80** | **19,83** |
| **% trouvé** | **46,67** | **7,82** | **7,50** | **19,97** |

### EXEMPLE 4 : Chlorhydrate de (±) trans 2-(diméthylamino)cyclopropyl méthylcarbamate

Le produit est obtenu selon le procédé de l'exemple 2 en utilisant comme substrat le composé de la préparation B.
**Point de fusion** **: 158-160°C**

| **Microanalyse élémentaire :** | | | | |
|---|---|---|---|---|
| | **C** | **H** | **N** | **Cl** |
| **% calculé** | **42,60** | **7,81** | **14,19** | **17,96** |
| **% trouvé** | **42,54** | **7,69** | **13,97** | **18,09** |

### EXEMPLE 5 : Chlorhydrate de (±) trans 2-[(benzyloxy)méthyl]-N,N-diméthylcyclopropanamine

Une solution refroidie à 5°C de 7 g du composé de la préparation C dans 140 ml de tétrahydrofurane, additionnée de 60 ml d'une solution de Red-Al® à 65 % dans le toluène, est agitée 2 heures à 5°C, 16 heures à température ambiante, puis refroidie à 0°C et hydrolysée. La solution est alors diluée à l'éther, filtrée et décantée, et la phase organique est concentrée. Le résidu est transformé en son chlorhydrate de façon classique permettant d'obtenir le produit attendu.
**Point de fusion** **: 118-120°C**

| **Microanalyse élémentaire :** | | | | |
|---|---|---|---|---|
| | **C** | **H** | **N** | **Cl** |
| **% calculé** | **64,59** | **8,34** | **5,79** | **14,66** |
| **% trouvé** | **64,14** | **8,46** | **5,78** | **14,54** |

### EXEMPLE 6 : Chlorhydrate de (±) cis 2-[(benzyloxy)méthyl]-N,N-diméthylcyclopropanamine

Le produit est obtenu selon le procédé de l'exemple 5 en utilisant comme substrat le composé de la préparation D.
**Point de fusion** **: 90-92°C**

| **Microanalyse élémentaire :** | | | | |
|---|---|---|---|---|
| | **C** | **H** | **N** | **Cl** |
| **% calculé** | **64,59** | **8,34** | **5,79** | **14,66** |
| **% trouvé** | **64,35** | **8,07** | **5,84** | **14,84** |

### EXEMPLE 7 : Chlorhydrate de (±) trans [2-(diméthylamino)cyclopropyl]méthyl acétate

### Stade 1 : Chlorhydrate de (±) trans [2-(diméthylamino)cyclopropyl]méthanol

A une solution de 4 g du composé de l'exemple 5 dans 200 ml d'ammoniaque liquide condensé est ajouté, par fractions, 1,2 g de sodium. Après 3 heures, 100 ml d'éther puis 5 ml d'éthanol sont ajoutés. L'agitation est maintenue 16 heures puis le milieu est concentré et repris au dichlorométhane. Le phase organique est traitée de façon classique puis évaporée. Une chromatographie sur gel de silice (dichlorométhane/méthanol: 90/10) permet d'isoler le produit attendu qui est transformé en son chlorhydrate.
**Point de fusion** **: 88-90°C**

### Stade 2 : Chlorhydrate de (±) trans [2-(diméthylamino)cyclopropyl]méthyl acétate

Le produit est obtenu selon le procédé de l'exemple 1 en utilisant comme substrat le composé obtenu au stade 1.
**Point de fusion** **: 144-146°C**

| **Microanalyse élémentaire :** | | | | |
|---|---|---|---|---|
| | **C** | **H** | **N** | **Cl** |
| **% calculé** | **49,61** | **8,33** | **7,23** | **18,31** |
| **% trouvé** | **49,58** | **8,34** | **7,11** | **18,09** |

### EXEMPLE 8 : Chlorhydrate de (±) cis [2-(diméthylamino)cyclopropyl]méthyl acétate

### Stade 1 : Chlorhydrate de (±) cis [2-(diméthylamino)cyclopropyl]méthanol

Le produit est obtenu selon le procédé du stade 1 de l'exemple 7 en utilisant comme substrat le composé de l'exemple 6.
**Point de fusion** **: 112-114°C**

### Stade 2 : Chlorhydrate de (±) cis [2-(diméthylamino)cyclopropyl] méthyl acétate

Le produit est obtenu selon le procédé de l'exemple 1 en utilisant comme substrat le composé obtenu dans le stade 1.
**Point de fusion** **: 108-110°C**

| **Microanalyse élémentaire :** | | | | |
|---|---|---|---|---|
| | **C** | **H** | **N** | **Cl** |
| **% calculé** | **49,61** | **8,33** | **7,23** | **18,31** |
| **% trouvé** | **49,37** | **8,34** | **7,16** | **18,24** |

### EXEMPLE 9 : Chlorhydrate de (±) trans 2-[(diméthylamino)méthyl]cyclopropyl acétate

Le produit est obtenu selon le procédé de l'exemple 1 en utilisant comme substrat le composé de la préparation E.
**Point de fusion** **: 100-105°C**

| **Microanalyse élémentaire :** | | | | |
|---|---|---|---|---|
| | **C** | **H** | **N** | **Cl** |
| **% calculé** | **49,61** | **8,33** | **7,23** | **18,31** |
| **% trouvé** | **49,32** | **8,37** | **7,11** | **18,26** |

### EXEMPLE 10 : Chlorhydrate de [1-(diméthylamino)cyclopropyl]méthyl acétate

Le produit est obtenu selon le procédé de l'exemple 1 en utilisant comme substrat le composé de la préparation F.
**Point de fusion** **: 100-102°C**

| **Microanalyse élémentaire :** | | | | |
|---|---|---|---|---|
| | **C** | **H** | **N** | **Cl** |
| **% calculé** | **49,61** | **8,33** | **7,23** | **18,31** |
| **% trouvé** | **49,27** | **8,37** | **7,16** | **18,28** |

### EXEMPLE 11 : Chlorhydrate de [1-(diméthylamino)cyclopropyl]méthyl diméthylcarbamate

A une solution, sous atmosphère inerte, de 2,1 g du composé de la préparation F dans 20 ml de pyridine sont ajoutés 1,95 g de chlorure de diméthylcarbamoyle, puis après 48 heures à température ambiante, la même quantité de réactif. La réaction est portée 3 heures à reflux, puis évaporée. Le résidu est repris au dioxane puis concentré de nouveau, repris à l'éther, lavé par une solution de NaHCO₃. La phase organique est extraite avec une solution d'HCl 0,1 N et la phase aqueuse est alcalinisée avec du carbonate de sodium puis extraite à l'éther. Les phases organiques sont traitées de façon classique et concentrées. Une chromatographie sur gel de silice du résidu (dichlorométhane/tétrahydrofurane : 90/10) permet d'isoler le produit attendu qui est transformé en son chlorhydrate.
**Point de fusion** **: 166-168°C**

| **Microanalyse élémentaire :** | | | | |
|---|---|---|---|---|
| | **C** | **H** | **N** | **Cl** |
| **% calculé** | **48,54** | **8,60** | **12,58** | **15,92** |
| **% trouvé** | **48,55** | **8,53** | **12,22** | **15,61** |

### EXEMPLE 12 : Chlorhydrate de 1-[(diméthylamino)méthyl]cyclopropyl acétate

Le produit est obtenu selon le procédé de l'exemple 1 en utilisant comme substrat le composé de la préparation G.
**Point de fusion** **: 166-170°C**

| **Microanalyse élémentaire :** | | | | |
|---|---|---|---|---|
| | **C** | **H** | **N** | **Cl** |
| **% calculé** | **49,61** | **8,33** | **7,23** | **18,31** |
| **% trouvé** | **49,52** | **8,46** | **7,21** | **18,88** |

### EXEMPLE 13 : Chlorhydrate de 2-[1-(diméthylamino)cyclopropyl]éthyl acétate

Le produit est obtenu selon le procédé de l'exemple 1 en utilisant comme substrat le composé de la préparation H.
**Point de fusion** **: 79-81°C**

| **Microanalyse élémentaire :** | | | | |
|---|---|---|---|---|
| | **C** | **H** | **N** | **Cl** |
| **% calculé** | **51,16** | **8,78** | **6,63** | **16,78** |
| **% trouvé** | **51,05** | **8,60** | **6,90** | **16,94** |

### EXEMPLE 14 : Fumarate de 2-[1-(diméthylamino)cyclopropyl]éthyl diméthylcarbamate

Une solution à 5°C de 1,3 g du composé de la préparation H dans 25 ml de tétrahydrofurane, additionnée de 0,4 g d'hydrure de sodium est agitée 10 minutes à 5°C, 1 heure à température ambiante puis 2 heures à 40°C et enfin ramenée à 5°C. 1,2 g de chlorure de diméthylcarbamoyle sont alors additionnés lentement et le milieu réactionnel est agité 1 heure à 5°C, 1 heure à température ambiante puis 7 heures à 40°C et enfin concentré sous pression réduite. Le résidu est repris au dichlorométhane, lavé par une solution saturée de chlorure de sodium, séché et concentré. Une chromatographie sur gel de silice (acétate d'éthyle) permet d'isoler le produit attendu qui est transformé en son fumarate de façon classique.
**Point de fusion** **: 148-149°C**

| **Microanalyse élémentaire :** | | | |
|---|---|---|---|
| | **C** | **H** | **N** |
| **% calculé** | **53,15** | **7,65** | **8,86** |
| **% trouvé** | **52,71** | **7,52** | **8,56** |

### EXEMPLE 15 : Fumarate de 2-[1-(diméthylamino)cyclopropyl]éthyl méthylcarbamate

A une solution, refroidie à 5°C, de 1,3 g du composé de la préparation H dans 26 ml d'éther est additionné 0,63 g de méthylisocyanate, puis le milieu est porté au reflux 4 heures. On répète trois fois les ajouts de méthylisocyanate alternativement avec les périodes de reflux de 4 heures. En fin de réaction, le milieu est concentré sous pression réduite. Une chromatographie sur gel de silice du résidu (dichlorométhane/méthanol: 95/5) permet d'isoler le produit attendu qui est transformé en son fumarate selon un procédé classique.
**Point de fusion** **: 118-119°C**

| **Microanalyse élémentaire :** | | | |
|---|---|---|---|
| | **C** | **H** | **N** |
| **% calculé** | **51,65** | **7,33** | **9,27** |
| **% trouvé** | **51,67** | **7,38** | **9,08** |

### EXEMPLE 16 : Chlorhydrate de [1-(diméthylamino)cyclopropyl]méthyl nicotinate

Une solution de 0,9 g de chlorhydrate de chlorure d'acide nicotinique, 0,76 g du composé de la préparation F, 0,06 g de 4-diméthylaminopyridine dans 15 ml de pyridine est chauffée 5 heures à 80°C puis concentrée sous pression réduite. Le résidu est repris par un mélange d'éther et d'une solution saturée en NaHCO₃. La phase organique est traitée de façon classique puis concentrée. Une chromatographie sur gel de silice (dichlorométhane/méthanol: 97/3) permet d'isoler le produit qui est transformé en son chlorhydrate de façon classique.
**Point de fusion** **: > 130°C**

| **Microanalyse élémentaire :** | | | | |
|---|---|---|---|---|
| | **C** | **H** | **N** | **Cl** |
| **% calculé** | **45,01** | **6,61** | **8,75** | **22,14** |
| **% trouvé** | **45,06** | **6,70** | **8,61** | **21,03** |

### EXEMPLE 17 : Chlorhydrate de N,N-diméthyl-1-[(3-pyridinylméthoxy)méthyl] cyclopropanamine

A une solution de 3,4 g du composé de la préparation F dans 55 ml de diméthylformamide sont ajoutés 1,32 g d'hydrure de sodium. La réaction est maintenue 1 h 30 à 45°C, puis ramenée à température ambiante avant d'être refroidie à 0°C, et 0,036 mol de chlorure de 3-picolyle est ajoutée. Le milieu réactionnel est agité 16 heures à température ambiante suivi de 5 heures à 50°C puis concentré sous pression réduite. Le résidu est repris par une solution saturée de carbonate de sodium puis extrait à l'acétate d'éthyle. La phase organique est traitée de façon classique et évaporée. Une chromatographie sur gel de silice du résidu (dichlorométhane/méthanol : 97,5/2,5) permet d'isoler le produit attendu qui est transformé en son chlorhydrate de façon classique.
**Point de fusion** **: 205-210°C**

| **Microanalyse élémentaire :** | | | | |
|---|---|---|---|---|
| | **C** | **H** | **N** | **Cl** |
| **% calculé** | **51,62** | **7,22** | **10,03** | **25,40** |
| **% trouvé** | **51,72** | **7,22** | **9,78** | **25,46** |

### EXEMPLE 18 : Chlorhydrate de N-méthyl-1-[(3-pyridinyloxy)méthyl] cyclopropanamine

### Stade 1 : tert-Butyl-N-méthyl{1-[(3-pyridinyloxy)méthyl]cyclopropyl}carbamate

A une solution, refroidie à 0°C, de 7,9 g de triphénylphosphine dans 80 ml de tétrahydrofurane sont additionnés 4,7 ml de diéthylazodicarboxylate. Après 45 minutes, la réaction est ramenée à température ambiante et 2,9 g de 3-hydroxypyridine et 4 g du composé de la préparation I en suspension dans 40 ml de tétrahydrofurane sont ajoutés. Après 24 heures d'agitation, le milieu est concentré sous pression réduite puis 100 ml d'acide chlorhydrique 1N sont ajoutés. La phase aqueuse est extraite à l'acétate d'éthyle, alcalinisée par addition de carbonate de potassium solide, puis réextraite à l'éther. Les phases organiques réunies sont traitées de façon usuelle puis évaporées. Une chromatographie sur gel de silice (dichlorométhane/tétrahydrofurane : 97/3) permet d'isoler le produit attendu.

### Stade 2 : Chlorhydrate de N-méthyl-1-[(3-pyridinyloxy) méthyl]cyclopropanamine

Une solution contenant 0,32 g du composé obtenu au stade 1 dans 3 ml de dioxane, et 3 ml d'acide chlorhydrique 4N dans le dioxane, est agitée 1 heure, à température ambiante et sous atmosphère inerte, puis diluée à l'éther. La phase liquide est alors décantée et le résidu repris par 25 ml d'éthanol. La solution est concentrée jusqu'à 2 ml, puis diluée avec 20 ml d'éther, agitée, et le précipité formé est filtré, rincé, et séché permettant d'isoler le produit attendu.
**Point de fusion** **: 152-155°C**

| **Microanalyse élémentaire :** | | | | |
|---|---|---|---|---|
| | **C** | **H** | **N** | **Cl** |
| **% calculé** | **46,82** | **6,52** | **10,92** | **27,64** |
| **% trouvé** | **46,83** | **6,48** | **10,68** | **27,68** |

### EXEMPLE 19 : Chlorhydrate de N,N-diméthyl-1-[(3-pyridinyloxy) méthyl]cyclopropanamine

A une solution, refroidie à 0°C, de 1,38 g d'acide formique, 1,12 ml de formaldéhyde à 37 % dans l'eau, et 0,15 ml d'eau distillée sont additionnés 1,07 g du composé de l'exemple 18 dilué dans 1,2 ml d'eau. La réaction est portée 16 heures à reflux puis refroidie à 0°C et 10 ml de soude 4N sont ajoutés. Le milieu est extrait à l'éther. La phase organique est traitée de façon usuelle et évaporée. Le résidu est repris à l'éthanol, et de nouveau concentré, puis dilué dans 20 ml d'éther et 4 ml d'une solution d'acide chlorhydrique 4N dans le dioxane. Le mélange est agité 20 minutes, filtré, rincé à l'éther puis séché permettant d'isoler le produit attendu.
**Point de fusion** **: 215-220°C**

### EXEMPLE 20 : Dichlorhydrate de N,N-diméthyl-1-[2-(3-pyridinyloxy) éthyl]cyclopropanamine

Une solution contenant 0,58 g du sel de sodium de 3-hydroxypyridine dans 5 ml de diméthylsulfoxyde, et 0,46 g du composé de la préparation J est portée 16 heures à reflux, puis ramenée à température ambiante, et reprise par un mélange éther/solution saturée en carbonate de sodium. Après décantation, la phase aqueuse est extraite à l'acétate d'éthyle puis les phases organiques sont lavées par une solution de soude 1N, puis par une solution à 10 % de chlorure de lithium. Après concentration, le résidu est repris par 20 ml d'éther et le dichlorhydrate du produit attendu est obtenu de façon classique.
**Point de fusion** **: 173-175°C**

| **Microanalyse élémentaire :** | | | | |
|---|---|---|---|---|
| | **C** | **H** | **N** | **Cl** |
| **% calculé** | **50,75** | **7,31** | **11,34** | **24,97** |
| **% trouvé** | **50,76** | **7,28** | **9,72** | **24,37** |

### EXEMPLE 21 : Chlorhydrate de 4-({2-[1-(diméthylamino)cyclopropyl]éthyl} sulfanyl)phenol

Une solution contenant 0,4 g de 4-hydroxythiophénol à 90 %, 0,46 g du composé de la préparation J, dans 10 ml de diméthylformamide et 0,7 g de carbonate de potassium est agitée 24 heures à température ambiante, puis diluée à l'éther. Le milieu est acidifié par addition de 15 ml d'acide chlorhydrique 1N, puis décanté. La phase organique est réextraite par 15 ml d'acide chlorhydrique 1N et les phases aqueuses sont alcalinisées et extraites à l'acétate d'éthyle. Après séchage des phases acétylées et concentration de celles-ci, le résidu obtenu correspond au produit attendu qui est transformé en son chlorhydrate de façon classique.
**Point de fusion** **: 193-195°C**

### EXEMPLE 22 : Dichlorhydrate de 1-[(3-pyridinyloxy)méthyl]cyclopropanamine

### Stade 1 : tert-butyl 1-[(3-pyridinyloxy)méthyl]cyclopropylcarbamate

Le produit est obtenu selon le procédé du stade 1 de l'exemple 18 en utilisant comme substrat le composé de la préparation K.
**Point de fusion** **: 112-114°C**

### Stade 2 : Dichlorhydrate de 1-[(3-pyridinyloxy)méthyl]cyclopropanamine

Le produit est obtenu selon le procédé de l'exemple 18 stade 2 en utilisant comme substrat le composé obtenu dans le stade 1.
**Point de fusion** **: 120-122°C**

### EXEMPLE 23 : Chlorhydrate de N-méthyl-1-{[(6-méthyl-3-pyridinyl). oxy]méthyl}cyclopropanamine

### Stade 1 : tert-butyl méthyl(1-{[(6-méthyl-3-pyridinyl)oxy]méthyl} cyclopropyl)carbamate

Le produit est obtenu selon le procédé du stade 1 de l'exemple 18 en utilisant comme substrat le composé de la préparation I et la 6-méthyl-pyridin-3-ol.
**Point de fusion** **: 62-64°C**

### Stade 2 : Chlorhydrate de N-méthyl-1-{[(6-méthyl-3-pyridinyl) oxy]méthyl}cyclopropanamine

Le produit est obtenu selon le procédé de l'exemple 18 stade 2 en utilisant comme substrat le composé obtenu dans le stade 1.
**Point de fusion** **: 172-175°C**

### EXEMPLE 24 : Chlorhydrate de N-méthyl-1-{[(6-chloro-3-pyridinyl) oxy]méthyl}cyclopropanamine

### Stade 1 : tert-butyl méthyl(1-{[(6-chloro-3-pyridinyl)oxy]méthyl} cyclopropyl)carbamate

Le produit est obtenu selon le procédé du stade 1 de l'exemple 18 en utilisant comme substrat le composé de la préparation I et la 2-chloro-5-hydroxypyridine.
**Point de fusion** **: 70-72°C**

### Stade 2 : Chlorhydrate de N-méthyl-1-{[(6-chloro-3-pyridinyl)oxy] méthyl}cyclopropanamine

Le produit est obtenu selon le procédé de l'exemple 18 stade 2 en utilisant comme substrat le composé obtenu dans le stade 1.
**Point de fusion** **: 120-122°C**

### EXEMPLE 25 : Chlorhydrate de N-{1-[(3-lluorophénoxy)méthyl]cyclopropyl}-N-méthylamine

### Stade 1 : tert-butyl méthyl(1-{[(6-méthyl-3-pyridinyl)oxy]méthyl} cyclopropyl)carbamate

Le produit est obtenu selon le procédé du stade 1 de l'exemple 18 en utilisant comme substrat le composé de la préparation I et le 3-fluorophénole.

### Stade 2 : Chlorhydrate de N-{1-[(3-fluorophénoxy)méthyl]cyclopropyl}-N-méthylamine

Le produit est obtenu selon le procédé de l'exemple 18 stade 2 en utilisant comme substrat le composé obtenu dans le stade 1.
**Point de fusion** **: 98-100°C**

### EXEMPLE 26 : Fumarate de 3-[1-(diméthylamino)cyclopropyl]propyl diméthylcarbamate

Le produit est obtenu selon le procédé de l'exemple 11 en utilisant comme substrat le composé obtenu dans la préparation L.
**Point de fusion** **: 110-111°C**

### EXEMPLE 27 : Fumarate de 3-[1-(diméthylamino)cyclopropyl]propyl méthylcarbamate

Le produit est obtenu selon le procédé de l'exemple 15 en utilisant comme substrat le composé obtenu dans la préparation L.
**Point de fusion** **: 150-151°C**

### EXEMPLE 28 : Dichlorhydrate de N-méthyl-1-[(2-pyridinylsulfanyl) méthyl]cyclopropanamine

### Stade 1 : tert-butyl méthyl{1-[(2-pyridinylsulfanyl)méthyl] cyclopropyl}carbamate

1,75 g de carbonate dipotassique sont ajoutés à une solution de 1,33 g de 2-mercaptopyridine et 2,65 g du composé de la préparation M dans 25 ml de diméthylformamide. Après 24 heures d'agitation à 20°C, le milieu réactionnel est repris dans l'éther, filtré et concentré. Une chromatographie sur gel de silice du résidu (dichlorométhane) permet d'isoler le produit attendu
**Point de fusion** **: 62-64°C**

### Stade 2 : Dichlorhydrate de N-méthyl-1-[(2-pyridinylsulfanyl) méthyl]cyclopropanamine

Le produit est obtenu selon le procédé de l'exemple 18 stade 2 en utilisant comme substrat le composé obtenu dans le stade 1.
**Point de fusion** **: 174-178°C**

### EXEMPLE 29 : Dichlorhydrate de N-méthyl-1-[3-(3-pyridinyloxy)propyl] cyclopropanamine

### Stade 1 : tert-butyl méthyl{1-[3-(3-pyridinyloxy)propyl]cyclopropyl}carbamate

Le produit est obtenu selon le procédé du stade 1 de l'exemple 18 en utilisant comme substrat le composé de la préparation L.

### Stade 2 : Dichlorhydrate de N-méthyl-1-[3-(3-pyridinyloxy)propyl] cyclopropanamine

Le produit est obtenu selon le procédé de l'exemple 18 stade 2 en utilisant comme substrat le composé obtenu dans le stade 1.
**Point de fusion** **: 118-120°C**

### EXEMPLE 30 : Dichlorhydrate de N-méthyl-1-[2-(3-pyridinyl)éthyl] cyclopropanamine

### Stade 1 : tert-butyl méthyl{1-[(Z)-2-(3-pyridinyl)ethenyl]cyclopropyl}carbamate

On ajoute 24,7 g du produit obtenu lors de la préparation O à un mélange contenant 6,5 g de terbutylate de potassium dans 100 ml de diméthylsulfoxyde. Après 1 heure d'agitation sont additionnés 5,5 g du produit obtenu au stade 1 de la préparation L dans 20 ml de diméthylsulfoxyde. Après 1 heure 30 de réaction, le milieu est dilué à l'éther et 120 ml d'eau sont ajoutés. Les phases organiques sont extraites à l'acide chlorhydrique 1N puis traitées de façon usuelle. Une chromatographie sur gel de silice du résidu (dichlorométhane/tétrahydrofurane : 97/3) permet d'isoler le produit attendu.

### Stade 2 : tert-butyl méthyl{1-[2-(3-pyridinyl)éthyl]cyclopropyl}carbamate

On hydrogène 5,1 g du produit obtenu au stade 1 en solution dans 120 ml de méthanol en présence de 1 g de palladium à 10 % sur charbon (20°C, 1 bar, 3 heures). On filtre, rince le catalyseur au méthanol, concentre à sec, sèche à 45°C sous 0,5 torr, permettant d'obtenir le produit souhaité.

### Stade 3 : Dichlorhydrate de N-méthyl-1-[2-(3-pyridinyl)éthyl]cyclopropanamine

On ajoute 20 ml d'acide chlorhydrique 4N dans le dioxane à un mélange contenant 4,4 g du produit obtenu au stade 2 dans 20 ml de dioxane. On agite 4 heures à 20°C puis ajoute 20 ml d'éther. Une cristallisation est observée. Les cristaux sont filtrés, lavés à l'éther et séchés à 50°C sous 0,5 torr permettant d'obtenir le produit attendu.
**Point de fusion** **: 202-205°C**

### EXEMPLE 31 : Fumarate de N-méthyl-1-[(Z)-2-(3-pyridinyl)éthényl] cyclopropanamine

On ajoute 12 ml d'acide chlorhydrique 4N dans le dioxane à un mélange contenant 2,3 g du produit obtenu, au stade 1 de l'exemple 30, en solution dans 12 ml de dioxane, agite 4 heures à 20°C, puis ajoute 30 ml d'éther et agite à nouveau 16 heures à 20°C. Un précipité se forme et la phase surnageante est décantée. Le résidu est repris par une solution aqueuse de carbonate de sodium puis extrait plusieurs fois à l'éther. Les phases éthérées jointes sont séchées sur sulfate de sodium et concentrées à sec. Le résidu est dissous dans l'éthanol et 1,2 g d'acide fumarique dissous dans l'éthanol sont additionnés permettant d'isoler, après filtration et rinçage des cristaux formés à l'éther, le produit attendu.
**Point de fusion** **: 116-118°C**

### EXEMPLE 32 : Fumarate de [1-(1-pyrrolidinyl)cyclopropyl]méthyl diméthylcarbamate

A une solution de 1,98 g du produit de la préparation P dans 40 ml de tétrahydrofurane est additionné 0,62 g d'hydrure de sodium. Le milieu est agité 20 minutes à 5°C, 1 heure à 20°C, 2 heures à 40°C, puis refroidi à 5°C et 1,8 g de chlorure de diméthylcarbamoyle dissous dans 10 ml de tétrahydrofurane sont ajoutés. Après 30 minutes, la réaction est ramenée à température ambiante pendant 20 heures puis évaporée. Le résidu est repris dans le dichorométhane et traité de façon usuel. Une chromatographie sur gel de silice (dichlorométhane/méthanol : 97/3) permet d'isoler le produit souhaité qui est transformé en fumarate par addition de 0,95 g d'acide fumarique.
**Point de fusion** **: 123-124°C**

### EXEMPLE 33 : Chlorhydrate de N,N-diméthyl-1-[2-(3-pyridinyl)éthyl] cyclopropanamine

1,5 g du composé de l'exemple 30 sont dissous avec une solution aqueuse à 10 % de carbonate de sodium, puis extraits à l'éther, séchés sur sulfate de sodium et concentrés à sec. 1,17 ml de formol et 1,43 g d'acide formique à 5°C sont alors ajoutés au résidu obtenu, puis 0,15 ml d'eau. Le mélange est porté au reflux pendant 5 heures, refroidi à 5°C, puis alcalinisé par addition de soude 4N. Une extraction à l'éther, suivie d'une évaporation de la phase aqueuse permet d'obtenir un résidu qui est repris par un mélange de 20 ml d'éther et 3 ml de dioxane/acide chlorhydrique 4N. Le mélange obtenu est dilué avec 50 ml d'éther et agité 20 heures, puis filtré et séché permettant d'isoler le produit attendu.
**Point de fusion** **: 200-203°C**

### EXEMPLE 34 : Fumarate de 3-{[1-(1-pyrrolidinyl)cyclopropyl]méthoxy}pyridine

On introduit goutte à goutte à -15°C, 15,2 g de diisopropylazodicarboxylate dans un mélange contenant 19,7 g de triphénylphosphine et 300 ml de tétrahydrofurane, puis refroidit à -20°C et ajoute par petites fractions 4,95 g de 3-hydroxypyridine en solution dans 25 ml de tétrahydrofurane, puis 7,06 g du composé de la préparation P en solution dans 25 ml de tétrahydrofurane. Après 20 heures à -20°C, le milieu est concentré à sec, puis du cyclohexane est ajouté au résidu. Après filtration et concentration du filtrat, le résidu est chromatographié sur gel de silice (toluène/éthanol : 85/15) permettant d'isoler le produit attendu qui est transformé en son fumarate par action d'une solution éthanolique d'acide fumarique.
**Point de fusion** **: 118-120°C**

### EXEMPLE 35 : Fumarate de N-méthyl-1-[2-(3-pyridinyloxy)éthyl] cyclopropanamine

### Stade 1 : tert-butyl méthyl{1-[2-(3-pyridinyloxy)éthyl]cyclopropyl}carbamate

A une solution de 1,7 g de triphénylphosphine et 0,6 g de 3-hydroxypyridine dans 25 ml de tétrahydrofurane sont ajoutés successivement 1,3 g du composé de la préparation Q, et 1,35 g de diisopropylazodicarboxylate. Après 20 heures d'agitation à température ambiante, le milieu est concentré, puis repris par de l'éther, extrait à l'acide chlorhydrique 1 N. Après traitement usuel, les phases organiques sont évaporées sous pression réduite. Une chromatographie sur gel de silice (dichlorométhane/tétrahydrofurane : 97/3) permet d'isoler le produit attendu.

### Stade 2 : Fumarate de N-méthyl-1-[2-(3-pyridinyloxy)éthyl] cyclopropanamine

Une solution de 1,2 g du produit obtenu au stade 1 dissous dans 5 ml de dioxane et 4 ml d'acide chlorhydrique 4 N dans le dioxane est agitée 20 heures à température ambiante, puis 10 ml d'éther sont ajoutés. Le précipité formé est décanté, repris dans 15 ml d'eau, alcalinisé par addition de carbonate de potassium. La phase aqueuse est alors extraite au dichlorométhane. Les phases organiques suivent un traitement usuelle qui après concentration sous pression réduite permettent d'obtenir le produit attendu qui est transformé en fumarate par traitement avec une solution éthanolique d'acide fumarique.
**Point de fusion** **: 110-112°C**

### EXEMPLE 36 : Chlorhydrate de 2-[1-(méthylamino)cyclopropyl]éthyl diméthylcarbamate

### Stade 1 : 2-{1-[(tert-butoxycarbonyl)(méthyl)amino]cyclopropyl}éthyl diméthylcarbamate

Le produit est obtenu selon le procédé de l'exemple 11 en utilisant comme substrat le composé de la préparation Q

### Stade 2 : Chlorhydrate de 2-[1-(méthylamino)cyclopropyl]éthyl diméthylcarbamate

Le produit est obtenu selon le procédé de l'exemple 18 stade 2 en utilisant comme substrat le composé obtenu au stade précédent.
**Point de fusion** **: 104-108°C**

### EXEMPLE 37 : Fumarate de 2-[1-(1-pyrrolidinyl)cyclopropyl]éthyl diméthylcarbamate

Le produit est obtenu selon le procédé de l'exemple 36 en utilisant comme substrat le composé de la préparation R.
**Point de fusion :** **108-110°C**

### ETUDES PHARMACOLOGIQUES DES COMPOSES DE L'INVENTION

### EXEMPLE 38 : Déplacement de la fixation de l'[¹²⁵I]-α-bungarotoxine sur les récepteurs nicotiniques de l'organe électrique de torpille

Cette étude, réalisée selon la méthode décrite dans J. Pharmacol. Exp. Ther., 1994, 271 ; 624-631, a pour objectif d'évaluer l'affinité des composés de la présente invention sur les récepteurs nicotiniques de « type musculaire ».
Des membranes (1-5 µg/ml) d'organe électrique de torpille sont incubées (1h, 22°C) en présence d'une gamme de concentration (0,01-10 µM) de chaque composé de l'invention (dilution à partir d'une solution mère à 10 mM dans du DMSO) en présence d'[¹²⁵I]-α-bungarotoxine (A.S. : 7,4 TBq/mmol : 0,2 nM) dans du tampon Krebs (Tris-HCl 50 mM, KCl 5 mM, MgCl₂ 1 mM, CaCl₂ 2 mM, NaCl 100 mM, pH 7.4) avec 0,01 % de BSA, volume final de 500 µl. La liaison non-spécifique est déterminée par l'incubation de membranes en présence d'α-bungarotoxine (1 µM).
Les résultats indiquent que les composés de la présente invention ne possèdent aucune affinité significative vis-à-vis des récepteurs nicotiniques de « type musculaire », jusqu'à la concentration de 10 µM.

### EXEMPLE 39 : Déplacement de la fixation d'[³H]-épibatidine sur les récepteurs nicotiniques de cellules IMR32

Cette étude, réalisée selon la technique décrite dans Molec. Pharmacol., 1995, 48 ; 280-287, a pour but de déterminer l'affinité des composés de la présente invention sur les récepteurs nicotiniques de « type ganglionnaire » (American Soc. Neuroscience, 2000, 26, 138).
Des membranes (250 µg/ml) de cellules neuroblastome IMR-32 sont incubées (2h, 20°C) en présence d'une gamme de concentration (0,01-10 µM) de chaque composé de l'invention (dilution à partir d'une solution mère à 10 mM dans du DMSO) et d'(±)-[³H]-épibatidine (A.S.: 2464 GBq/mmol: 1,5 nM) dans du tampon phosphate (NaH₂PO₄ 20 mM, pH 7,4), volume final de 250 µl. La liaison non-spécifique est déterminée par l'incubation de membranes en présence de 300 µM de (-)nicotine.
Les résultats montrent que les composés de la présente invention n'ont aucune affinité significative sur les récepteurs nicotiniques de « type ganglionnaire », jusqu'à la concentration de 10 µM.

### EXEMPLE 40 : Déplacement de la fixation d'[³H]-oxotrémorine-M sur les récepteurs muscariniques de cerveau de rat

Cette étude, réalisée selon la méthode décrite dans Naumyn-Schmiederberg's Arch. Pharmacol., 2001, 363, 429-438, a pour objectif de déterminer l'affinité des composés de la présente invention sur les récepteurs muscariniques.
Des membranes (250 µg/ml) de cerveau de rat sont incubées (2h, 20°C) en présence d'une gamme de concentration (0,01-10 µM) de chaque composé de l'invention (dilution à partir d'une solution mère à 10 mM dans du DMSO) et d'[³H]-oxotrémorine-M (A.S. : 3174 GBq/mmol : 2 nM) dans du tampon phosphate (NaH₂PO₄ 20 mM, pH 7,4), volume final de 250 µl. La liaison spécifique est déterminée par l'incubation des membranes en présence d'atropine (1 µM). L'affinité des composés de la présente invention vis-à-vis des récepteurs muscariniques est caractérisée par la détermination du Kᵢ.
Les résultats démontrent que la plupart des composés de la présente invention, jusqu'à la concentration de 10 µM, sont dépourvus d'affinité vis-à-vis des récepteurs muscariniques. Certains composés de l'invention présentent un Kᵢ de l'ordre de 10 µM.

### EXEMPLE 41 : Déplacement de la fixation de l'[¹²⁵I]-α-bungarotoxine sur les récepteurs nicotiniques de « type α7 » de cerveau de rat

Cette étude, réalisée selon la méthode décrite dans Molec. Pharmacol., 1986, 30 ; 427-436, a pour objectif de déterminer l'affinité des composés de la présente invention vis-à-vis des récepteurs centraux nicotiniques de type α7.
Des membranes (1000 µg/ml) de cerveau de rat sont incubées (5h, 37°C) en présence d'une gamme de concentration (0,01-10 µM) de chaque composé de la présente invention (dilution à partir d'une solution mère à 10 mM dans du DMSO) et d'[¹²⁵I]-α-bungarotoxine (A.S. : 7,4 TBq/mmol : 1 nM) dans du tampon Krebs (Tris-HCl 50 mM, KCl 5 mM, MgCl₂ 1 mM, CaCl₂ 2 mM, NaCl 100 mM, pH 7,4) avec 0,05 % de BSA, volume final de 500 µl. La liaison non-spécifique est déterminée par l'incubation de membranes en présence d'α-bungarotoxine (1 µM).
L'affinité des composés de la présente invention vis-à-vis des récepteurs nicotiniques de type α7 est caractérisé par la détermination du Kᵢ.
Les résultats indiquent que la plupart des composés de la présente invention, jusqu'à la concentration de 10 µM, sont dépourvus d'affinité vis-à-vis des récepteurs nicotiniques centraux de type α7. Certains composés de l'invention présentent un Kᵢ de l'ordre de 10 µM.

### EXEMPLE 42 : Déplacement de la fixation de [³H]-cytisine sur les récepteurs nicotiniques de « type α4β2 » de cerveau de rat

Cette étude, réalisée selon la technique décrite dans Molec. Pharmacol., 1990, 39 ; 9-12, a pour objectif de déterminer l'affinité des composés de la présente invention vis-à-vis des récepteurs nicotiniques centraux de type α4β2.
Des membranes (250 µg/ml) de cerveau de rat sont incubées (2h, 20°C) en présence d'une gamme de concentration (0,01-10 µM) de chaque composé de la présente invention (dilution à partir d'une solution mère à 10 mM dans du DMSO) et de [³H]-cytisine (A.S. : 1184 GBq/mmol : 2 nM) dans du tampon phosphate (NaH₂PO₄ 20 mM, pH 7,4), volume final de 250µl. La liaison non-spécifique est déterminée par l'incubation de membranes en présence de 10 µM de (-)nicotine. L'affinité des composés de la présente invention vis-à-vis des récepteurs nicotiniques centraux de type α4β2 est caractérisée par la détermination du Kᵢ.
Les résultats obtenus montrent que les composés de la présente invention présentent une forte affinité vis-à-vis des récepteurs nicotiniques centraux de type α4β2 avec des Kᵢ de l'ordre de 10-100 nM.

Ces résultats, ainsi que ceux obtenus dans les exemples 39 à 42 indiquent que les composés de la présente invention sont de puissants ligands nicotiniques centraux spécifiques des récepteurs de type α4β2.

### EXEMPLE 43 : Mesure in vivo de la libération d'acétylcholine par microdialyse intra-corticale chez le rat Wistar vigile

L'administration systémique de nicotine et d'agonistes nicotiniques induit une augmentation *in vivo* d'acétylcholine dans diverses régions cérébrales (Neurochem. Res., 1996, 21, 1181-1186 ; Eur. J. Pharmacol., 1998, 351, 181-188 ; Br. J. Pharmacol., 1999, 127, 1486-1494). Une sonde de microdialyse est implantée au niveau du cortex préfrontal médian de rats mâles Wistar. Six ou sept jours après l'implantation des sondes, celles-ci sont perfusées par du Ringer (NaCl 147 mM, KCl 2.7 mM, CaCl₂ 1.2 mM, MgCl₂ 1 mM, néostigmine 20 nM) à un débit de 1 µl/min chez l'animal libre de se mouvoir. Après 2 heures de stabulation, le produit étudié est administré par voie intrapéritonéale. Un groupe d'animaux témoins reçoit le solvant du produit. Puis, les dialysats (30 µl) sont collectés toutes les 30 minutes pendant 4h afin de mesurer les concentrations extra-synaptiques corticales d'acétylcholine par HPLC en détection ampérométrique. Les résultats sont exprimés en pg d'acétylcholine/dialysat et les comparaisons inter-groupes sont effectuées par une analyse de variance à 2 facteurs (traitement x temps) avec mesures répétées sur le temps.

Les résultats obtenus montrent que les composés de la présente invention augmentent la libération corticale *in vivo* d'acétylcholine de manière dose-dépendante (+ 74 % à + 138 %) pour des doses actives allant de 0.3 à 3 mg/kg IP et indiquent le caractère agoniste α4β2 des composés de la présente invention.

### EXEMPLE 44 : Torsions abdominales induites à la phényl-p-benzoquinone (PBQ) chez la souris NMRI

L'administration intrapéritonéale d'une solution alcoolique de PBQ provoque des crampes abdominales chez la Souris (Proc. Soc. Exp. Biol., 1957, 95, 729-731). Ces crampes sont caractérisées par des contractions répétées de la musculature abdominale, accompagnées d'une extension des membres postérieurs. La plupart des analgésiques antagonisent ces crampes abdominales (Brit. J. Pharmacol. Chem., 1968, 32, 295-310). A t=0 min., les animaux sont pesés et le produit étudié est administré par voie IP. Un groupe d'animaux témoins reçoit le solvant du produit. A t=30 min., une solution alcoolique de PBQ (0.2 %) est administrée par voie IP sous un volume de 0,25 ml/souris. Immédiatement après l'administration de la PBQ, les animaux sont placés dans des cylindres en plexiglass (L = 19,5 cm ; D.I. = 5 cm). De t=35 min. à t=45 min., la réaction des animaux est observée et l'expérimentateur note le nombre total de crampes abdominales par animal. Les résultats sont exprimés par le pourcentage d'inhibition du nombre de crampes abdominales mesuré chez les animaux témoins à la dose active du composé étudié.

Les résultats obtenus montrent une inhibition allant de 50 à 80 %, pour des doses actives allant de 3 à 20 mg/kg IP. Ceci montre que les composés de l'invention sont pourvus de propriétés antalgiques.

### EXEMPLE 45 : Reconnaissance sociale chez le Rat Wistar

Initialement décrit en 1982 (J. Comp. Physiol., 1982, 96, 1000-1006), le test de la reconnaissance sociale a été ensuite proposé par différents auteurs (Psychopharmacology, 1987, 91, 363-368 ; Psychophamacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester par voie intrapéritonéale et, 2 heures plus tard, est remis en présence (5 minutes) du jeune rat. Le comportement de reconnaissance sociale est alors à nouveau observé et la durée en est mesurée. Le critère de jugement est la différence (T2-T1), exprimée en secondes, des temps de « reconnaissance » des 2 rencontres.

Les résultats obtenus montrent une différence (T2-T1) comprise entre (-31) et (-45) s pour des doses allant de 1 à 3 mg/kg IP. Ceci montre que les composés de l'invention augmentent la mémorisation de façon très importante, et à faible dose.

### EXEMPLE 46 : Reconnaissance d'objet chez le Rat Wistar

Le test de la reconnaissance d'objet chez le rat Wistar (Behav. Brain Res., 1988, 31, 47-59) est basé sur l'activité exploratoire spontanée de l'animal et possède les caractéristiques de la mémoire épisodique chez l'homme. Sensible au vieillissement (Eur. J. Pharmacol., 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques.

La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explore de façon préférentielle l'objet nouveau, ce qui signe le souvenir de l'objet déjà présenté.

Les résultats obtenus montrent une différence Delta de l'ordre de 6 s, pour des doses allant de 0.3 à 1 mg/kg PO, ce qui montre que les composés de l'invention augmentent la mémorisation de façon importante, et à très faible dose.

### EXEMPLE 47 : Compositions pharmaceutiques pour 1000 comprimés dosés à 10 mg

| | |
|---|---|
| Composé de l'exemple 18 | 10 g |
| Hydroxypropylméthylcellulose | 10 g |
| Amidon de blé | 15 g |
| Lactose | 90 g |
| Stéarate de magnésium | 2 g |

## Revendications

1. Composés de formule (I) : 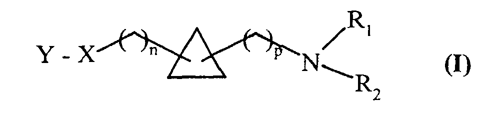 dans laquelle :
p représente un entier compris entre 0 et 6 inclus,
n représente un entier compris entre 0 et 6 inclus,
R₁ et R₂, identiques ou différents, indépendamment l'un de l'autre, représentent un groupement choisi parmi atome d'hydrogène, groupement alkyle (C₁-C₆) linéaire ou ramifié, aryle, et arylalkyle (C₁-C₆) linéaire ou ramifié, ou R₁+R₂ forment ensemble, avec l'atome d'azote qui les porte, un système monocyclique ou bicyclique, saturé, de 3 à 10 chaînons et contenant éventuellement un second hétéroatome choisi parmi oxygène, azote et soufre,
X représente un groupement choisi parmi atome d'oxygène, atome de soufre, groupement -CH=CH-, méthylène, groupement de formule -HC=N-O- et groupement de formule -O-CH₂-CH=CH- dans lesquels l'atome d'oxygène est relié à la partie Y des composés de formule (I),
Y représente un groupement choisi parmi aryle, hétéroaryle, arylalkyle (C₁-C₆) linéaire ou ramifié, hétéroarylalkyle (C₁-C₆) linéaire ou ramifié, -C(O)-A, et -C(S)-A,
A représente un groupement choisi parmi alkyle (C₁-C₆) linéaire ou ramifié, aryle, hétéroaryle, arylalkyle (C₁-C₆) linéaire ou ramifié, hétéroarylalkyle (C₁-C₆) linéaire ou ramifié, et NR₃R₄ dans lequel R₃ et R₄, identiques ou différents, représentent chacun un groupement choisi parmi atome d'hydrogène, groupement alkyle (C₁-C₆) linéaire ou ramifié, aryle, et arylalkyle (C₁-C₆) linéaire ou ramifié, ou R₃+R₄ forment ensemble, avec l'atome d'azote qui les porte, un système monocyclique ou bicyclique en (C₃-C₁₀),
leurs isomères optiques ainsi que leurs sels d'addition à un acide ou à une base pharmaceutiquement acceptable,
étant entendu que :
* *dans le cas de composés deformule (I) 1,1-disubstitué,*
- p est différent de zéro quand X représente un groupement méthylène, n prend la valeur zéro, Y représente un groupement aryle ou hétéroaryle, R₁ et R₂, identiques ou différents, représentent un atome d'hydrogène, un groupement alkyle (C₁-C₄) linéaire ou ramifié, benzyle, phényléthyle, ou forment ensemble avec l'atome d'azote qui les porte un groupement morpholino, thiomorpholino, ou un système cyclique carboné saturé en 5 à 7 chaînons,
- p est différent de zéro quand X représente un groupement méthylène, n prend la valeur zéro, Y représente un groupement acétyle, R₁ et R₂, identiques ou différents, représentent un atome d'hydrogène, un groupement alkyle (C₁-C₄) linéaire ou ramifié, phényle, benzyle, ou forment ensemble avec l'atome d'azote qui les porte un groupement pipéridinyle ou morpholino,
- R₁ et R₂ ne représentent pas simultanément un groupement méthyle :
* soit quand p et n prennent chacun la valeur 1, X représente un atome d'oxygène, et Y représente un groupement choisi parmi p-nitrobenzoyle, p-aminobenzoyle, p-chlorophénylaminocarbonyle, et acétyle,
* soit quand p prend la valeur zéro, n prend la valeur 1, X représente un atome d'oxygène ou de soufre, et Y représente un groupement 2-quinolyle substitué en position trois par un groupement alkyle (C₃-C₄) linéaire ou ramifié, ou phényle,
- Y ne représente pas un groupement 1,2-benzisoxazol-3-yl quand n prend la valeur 1, p prend la valeur zéro et X représente un atome d'oxygène,
* *dans le cas de composés de formule (I) 1*,*2-disubstitué,*
- R₁ et R₂ ne représentent pas simultanément un atome d'hydrogène quand p et n prennent chacun la valeur zéro et X-Y représentent ensemble un groupement phénoxy (éventuellement substitué par un ou deux groupements, identiques ou différents, choisis parmi méthoxy, diméthylamino, atome d'halogène, méthyle, trifluorométhyle, nitro, et amino), phénylsulfanyle, benzyloxy, benzyle, ou 2-phényléthyle,
- R₁ et R₂ ne représentent pas simultanément un groupement méthyle, quand p et n prennent chacun la valeur zéro, et X-Y représentent ensemble un groupement phénoxy (éventuellement substitué par un groupement choisi parmi atome de chlore et trifluorométhyle), un groupement phénylsulfanyle, ou un groupement benzyle,
étant entendu également que les composés de formule (I) sont différents des composés suivants :
- (1-benzylcyclopropyl)méthanamine,
- (1-benzylcyclopropyl)-N,N-diméthylméthanamine,
- 2-(phénoxycyclopropyl)méthanamine,
- 2-(phénoxyméthyl)-cyclopropanamine,
- (N,N-diméthyl)-2-(acétoxyméthyl)-cyclopropaneméthanamine,
- N- {2-[2-(benzyloxy)éthyl]cyclopropyl}-N,N-diméthylamine.
étant entendu aussi que :
■ par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi atomes d'halogène, groupements alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino (éventuellement substitué par un ou deux groupements alkyle (C₁-C₆) linéaire ou ramifié),
■ par groupement hétéroaryle, on comprend un système monocyclique aromatique ou bicyclique de 5 à 12 chaînons, contenant de un à trois hétéroatomes, identiques ou différents, choisis parmi oxygène, azote et soufre, et dont l'un des cycles, dans le cas d'un système bicyclique, possède un caractère aromatique, l'autre cycle pouvant être aromatique ou partiellement hydrogéné, chacun de ces groupements pouvant être éventuellement substitué par un ou plusieurs groupements identiques ou différents, choisis parmi les substituants précédemment définis dans le cas d'un groupement aryle.

2. Composés de formule (I) selon la revendication 1, **caractérisés en ce que** n est un entier compris entre 0 et 2 inclus, leurs isomères optiques ainsi que leurs sels d'addition à un acide ou à une base pharmaceutiquement acceptable.

3. Composés de formule (I) selon la revendication 1 **caractérisés en ce que** R₁ et R₂, identiques ou différents, représentent chacun un atome d'hydrogène ou un groupement alkyle (C₁-C₆) linéaire ou ramifié, leurs isomères optiques ainsi que leurs sels d'addition à un acide ou à une base pharmaceutiquement acceptable.

4. Composés de formule (I) selon la revendication 1 **caractérisés en ce que** X représente un atome d'oxygène, leurs isomères optiques ainsi que leurs sels d'addition à un acide ou à une base pharmaceutiquement acceptable.

5. Composés de formule (I) selon la revendication 1 **caractérisés en ce que** Y représente un groupement choisi parmi -C(O)NR₃R₄ dans lequel R₃ et R₄ sont tels que définis dans la formule (I), acétyle, -C(O)-hétéroaryle, arylalkyle (C₁-C₆) linéaire ou ramifié, et hétéroaryle, leurs isomères optiques ainsi que leurs sels d'addition à un acide ou à une base pharmaceutiquement acceptable.

6. Composés de formule (I) selon la revendication 1 **caractérisés en ce que** Y représente un groupement pyridinyle, leurs isomères optiques ainsi que leurs sels d'addition à un acide ou à une base pharmaceutiquement acceptable.

7. Composés de formule (I) selon la revendication 1 **caractérisés en ce qu'**ils représentent des composés de formule **(IA) :** 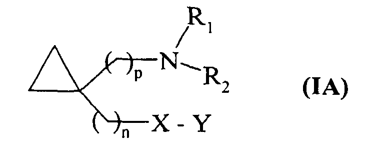 dans laquelle n, p, X, Y, R₁ et R₂ sont tels que définis dans la formule (I), leurs isomères optiques ainsi que leurs sels d'addition à un acide ou à une base pharmaceutiquement acceptable.

8. Composés de formule (I) selon la revendication 1 **caractérisés en ce qu'**ils représentent des composés de formule **(IB) :** 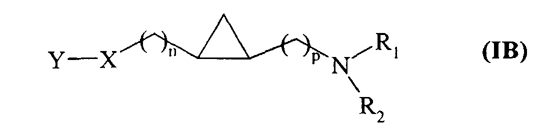 dans laquelle n, p, X, Y, R₁ et R₂ sont tels que définis dans la formule (I), leurs isomères optiques ainsi que leurs sels d'addition à un acide ou à une base pharmaceutiquement acceptable.

9. Composés de formule (I) selon la revendication 1 **caractérisés en ce que** p est un entier prenant la valeur 0 ou 1, leurs isomères optiques ainsi que leurs sels d'addition à un acide ou à une base pharmaceutiquement acceptable.

10. Composés de formule (IB) selon la revendication 8 **caractérisés en ce que** p représente 0 ou 1, n représente 0 ou 1, R₁ et R₂, identiques ou différents, représentent un atome d'hydrogène ou un groupement alkyle (C₁-C₆) linéaire ou ramifié, X représente un atome d'oxygène , et Y représente un groupement choisi parmi phénylalkyl (C₁-C₆) linéaire ou ramifié, pyridinyle, et -C(O)-A dans lequel A représente un groupement alkyle (C₁-C₆) linéaire ou ramifié, monoalkyl(C₁-C₆)amino, ou dialkyl(C₁-C₆)amino, la partie alkyle étant linéaire ou ramifié, leurs isomères optiques ainsi que leurs sels d'addition à un acide ou à une base pharmaceutiquement acceptable.

11. Composés de formule (IA) selon la revendication 7 **caractérisés en ce que** p représente 0 ou 1, n est un entier compris entre 0 et 3 inclus, R₁ et R₂, identiques ou différents, représentent un atome d'hydrogène, un groupement alkyle (C₁-C₆) linéaire ou ramifié, ou forment ensemble avec l'atome d'azote qui les porte un radical pyrrolidinyle, X représente un atome d'oxygène, de soufre ou un groupement -CH=CH-, et Y représente un groupement choisi parmi phényle (éventuellement substitué par un groupement hydroxy, alkyle (C₁-C₆) linéaire ou ramifié, ou un atome d'halogène), pyridinyle, pyridinylalkyle (C₁-C₆) linéaire ou ramifié (le radical pyridinyle dans chacun des groupements étant éventuellement substitué par un groupement choisi parmi atome d'halogène, et groupement alkyle (C₁-C₆) linéaire ou ramifié), et -C(O)-A dans lequel A représente un groupement choisi parmi alkyl (C₁-C₆) linéaire ou ramifié, monoalkyl(C₁-C₆)amino linéaire ou ramifié, dialkyl(C₁-C₆)amino linéaire ou ramifié et pyridinyle, leurs isomères optiques ainsi que leurs sels d'addition à un acide ou à une base pharmaceutiquement acceptable.

12. Composés de formule (I) selon la revendication 1 qui sont le :
■ 2-[1-(diméthylamino)cyclopropyl]éthyl méthylcarbamate,
■ 2-[1-(diméthylamino)cyclopropyl]éthyl dimethylcarbamate,
■ [1-(diméthylamino)cyclopropyl]méthyl dimethylcarbamate,
■ [1-(diméthylamino)cyclopropyl]méthyl acétate,
■ 2-[1-(diméthylamino)cyclopropyl]éthyl acétate,
■ 1-[(diméthylamino)méthyl]cyclopropyl acétate,
■ [1-(diméthylamino)cyclopropyl]méthyl nicotinate,
■ *N,N*-diméthyl-1-[(3-pyridinyloxy)méthyl]cyclopropanamine,
■ *N*-méthyl-1-[(3-pyridinyloxy)méthyl]cyclopropanamine,
■ *N,N*-diméthyl-1-[(3-pyridinylméthoxy)méthyl]cyclopropanamine,
■ *N,N*-diméthyl-1-[2-(3-pyridinyloxy)éthyl]cyclopropanamine,
■ 4-({2-[1-diméthylamino)cyclopropyl]éthyl}sulfanyl)phénol,
■ (±)*cis*-2-(diméthylamino)cyclopropyl méthylcarbamate,
■ (±)*trans*-2-(diméthylamino)cyclopropyl méthylcarbamate,
■ (±)*cis*-2-(diméthylamino)cyclopropyl acétate,
■ (±)*trans*-2-(diméthylamino)cyclopropyl acétate,
■ (±)*cis*-2-(diméthylamino)cyclopropyl]méthyl acétate,
■ (±)*trans*-2-(diméthylamino)cyclopropyl]méthyl acétate,
■ (±)*cis*-2-[(benzyloxy)méthyl]-*N,N*-diméthylcyclopropanamine,
■ (±)*trans*-2-[(benzyloxy)méthyl]-*N,N*-diméthylcyclopropanamine,
■ (±)*trans*-2-[(diméthylamino)méthyl]cyclopropyl acétate,
■ dichlorhydrate de 1-[(3-pyridinyloxy)méthyl]cyclopropanamine,
■ chlorhydrate de *N*-méthyl-1-{[(6-méthyl-3-pyridinyl)oxy]méthyl}cyclopropanamine,
■ chlorhydrate de *N*-méthyl-1-{[(6-chloro-3-pyridinyl)oxy]méthyl}cyclopropanamine,
■ chlorhydrate de *N-*{1-[(3-fluorophénoxy)méthyl]cyclopropyl}-*N*-méthylamine,
■ fumarate de 3-[1-(diméthylamino)cyclopropyl]propyl diméthylcarbamate,
■ fumarate de 3-[1-(diméthylamino)cyclopropyl]propyl méthylcarbamate,
■ dichlorhydrate de *N*-méthyl-1-[(2-pyridinylsulfanyl)méthyl]cyclopropanamine,
■ dichlorhydrate de *N*-méthyl-1-[3-(3-pyridinyloxy)propyl]cyclopropanamine,
■ dichlorhydrate de *N*-méthyl-1-[2-(3-pyridinyl)éthyl]cyclopropanamine,
■ fumarate de *N*-méthyl-1-[(Z)-2-(3-pyridinyl)éthényl]cyclopropanamine,
■ fumarate de [1-(1-pyrrolidinyl)cyclopropyl]méthyl diméthylcarbamate,
■ chlorhydrate de *N,N*-diméthyl-1-[2-(3-pyridinyl)éthyl]cyclopropanamine,
■ fumarate de 3-{[1-(1-pyrrolidinyl)cyclopropyl]méthoxy}pyridine,
■ fumarate de *N*-méthyl-1-[2-(3-pyridinyloxy)éthyl]cyclopropanamine,
■ chlorhydrate de 2-[1-(méthylamino)cyclopropyl]éthyl diméthylcarbamate, et le
■ fumarate de 2-[1-(1-pyrrolidinyl)cyclopropyl]éthyl diméthylcarbamate.
leurs isomères optiques ainsi que leurs sels d'addition à un acide ou à une base pharmaceutiquement acceptable.

13. Procédé de préparation des composés de formule (I), **caractérisé en ce qu'**on utilise comme produit de départ un composé de formule **(II) :** 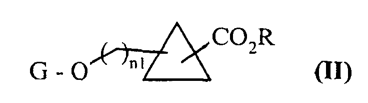 dans lequel G représente un groupement protecteur des fonctions hydroxy classiquement utilisé en synthèse organique, R représente un atome d'hydrogène ou un groupement alkyle (C₁-C₆) linéaire ou ramifié, et n₁ représente 0 ou 1,
composés de formule (II), qui sont :
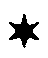 ***soit*** mis à réagir avec de l'ammoniaque liquide en présence d'un cyanure alcalin en solvant alcoolique, pour conduire aux composés de formule **(III) :** 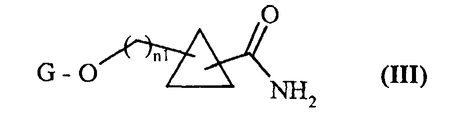 dans laquelle G représente un groupement protecteur des fonctions hydroxy, et n₁ représente 0 ou 1,
composés de formule (III) qui sont traités par un dihalogène en présence d'une base telle que la soude, pour conduire aux composés de formule **(IV) :** 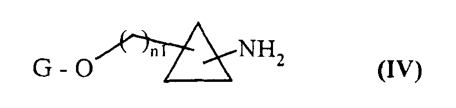 dans laquelle G et n₁ sont tels que définis précédemment,
composés de formule (IV) dont on protège sélectivement la fonction amine primaire par un groupement protecteur G₂ classiquement utilisé en chimie organique tel que le groupement BOC (t-butyloxycarbonyle), pour conduire aux composés de formule **(V) :** 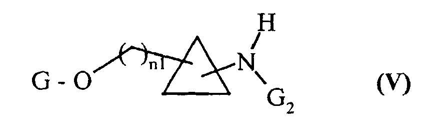 dans lequel n₁, G et G₂ sont tels que définis précédemment,
composés de formule (V) qui sont ensuite successivement :
• traités par un composé de formule **(VIA),** en milieu basique :
R₁ - L₁ **(VIA)**
dans laquelle R₁ est tel que défini dans la formule (I) et L₁ représente un groupe partant usuel de la synthèse organique,
• puis dont la fonction amine est déprotégée puis soit ne subissent pas d'autre traitement et conduisent alors aux composés de formule **(VIIA) :** 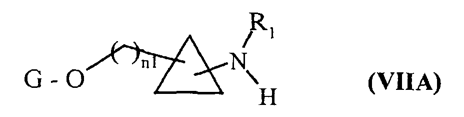 dans laquelle R₁, n₁ et G sont tels que définis précédemment,
soit sont mis à réagir avec un composé de formule **(VIB)** :
R₂ₐ - L₁ **(VIB)**
dans laquelle R₂ₐ a les mêmes définitions que R₂, tel que défini dans la formule (I), à l'exception de la valeur atome d'hydrogène, et L₁ est tel que défini précédemment,
pour conduire aux composés de formule **(VIIB) :** 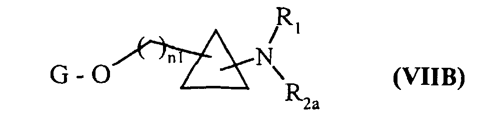 dans laquelle R₁, R₂ₐ, n₁ et G sont tels que définis précédemment,
l'ensemble des composés de formule (VIIA) et (VIIB) formant les composés de formule **(VII) :** 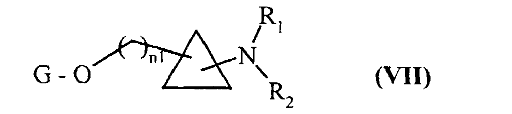 dans laquelle n₁, G, R₁ et R₂ sont tels que définis précédemment,
composés de formule (VII) dont la fonction hydroxy est déprotégée puis qui sont :
◆ soit traités par un composé de formule **(VIII) :**
Y - L₁ **(VIII)**
dans laquelle Y est tel que défini dans la formule (I) et L₁ est tel que défini précédemment,
pour conduire aux composés de formule **(I/a),** cas particulier des composés de formule (I) : 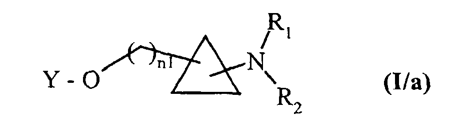 dans laquelle n₁, Y, R₁ et R₂ sont tels que définis dans la formule (I),
◆ **soit** mis à réagir avec du SOCl₂ pour conduire aux composés de formule **(IX)** : 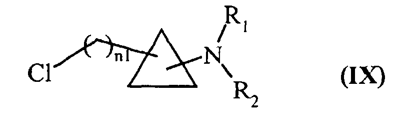 dans laquelle n₁, R₁ et R₂ sont tels que définis précédemment,
composés de formule (IX) qui sont mis à réagir en milieu basique avec un composé de formule **(X)** :
Y₁-SH **(X)**
dans laquelle Y₁ représente un groupement aryle, arylalkyle (C₁-C₆) linéaire ou ramifié, hétéroaryle ou hétéroarylalkyle (C₁-C₆) linéaire ou ramifié,
pour conduire aux composés de formule **(I/b),** cas particulier des composés de formule (I) : 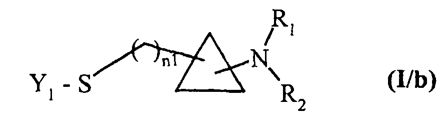 dans laquelle Y₁, n₁, R₁ et R₂ ont les mêmes significations que précédemment,
◆ **soit** soumis, dans le cas où n₁ prend la valeur 1, à l'action d'un agent oxydant classique de la synthèse organique pour conduire aux composés de formule **(XI) :** 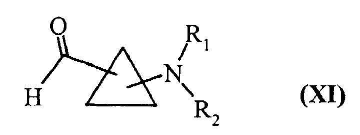 dans laquelle R₁ et R₂ sont tels que définis précédemment,
composés de formule (XI) qui sont :
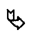 *soit* traités par une hydroxylamine de formule **(XII)** :
H₂N - OY₂ **(XII)**
dans laquelle Y₂ représente un atome d'hydrogène ou un groupement Y₁ tel que défini précédemment,
pour conduire aux composés de formule **(I/c)**, cas particulier des composés de formule (I): 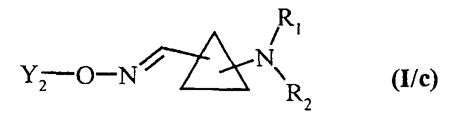 dans laquelle Y₂, R₁ et R₂ sont tels que définis précédemment,
composés de formule (I/c) dans le cas particulier où Y₂ représente un atome d'hydrogène, qui sont soumis à l'action d'un composé de formule **(XIV) :**
Y₃ - L₁ **(XIV)**
dans laquelle L₁ est tel que défini précédemment et Y₃ représente un groupement de formule -C(O)-A ou -C(S)-A tels que définis dans la formule (I), pour conduire aux composés de formule **(I/d),** cas particulier des composés de formule (I): 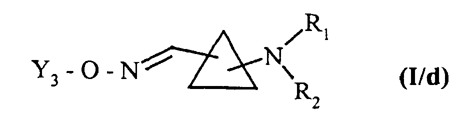 dans laquelle Y₃, R₁ et R₂ sont tels que définis précédemment,
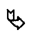 *soit* traités dans des conditions de réaction de Wittig puis soumis à l'action d'un agent réducteur classique de la synthèse organique, pour conduire aux composés de formule **(I/e),** cas particulier des composés de formule (I), 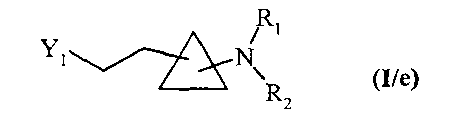 dans laquelle Y₁, R₁ et R₂ sont tels que définis précédemment,
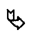 *soit* soumis à l'action d'un composé de formule Ph₃P=CH-CO₂Et puis réduit par action d'un agent réducteur de la synthèse organique pour conduire aux composés de formule **(XV) :** 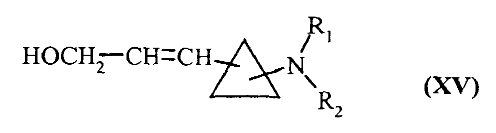 dans laquelle R₁ et R₂ sont tels que définis précédemment,
composés de formule (XV) qui sont traités par un composé de formule (VIII) tel que défini précédemment, pour conduire aux composés de formule (**I/f**), cas particulier des composés de formule (I) : 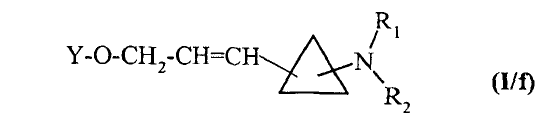 dans laquelle Y, R₁ et R₂ sont tels que définis dans la formule (I),
◆ **soit** transformés, dans le cas où n₁ prend la valeur 1, en leur dérivé halogéné correspondant selon des conditions usuelles de la chimie organique, puis mis à réagir avec un cyanure alcalin en présence de diméthylsulfoxyde, pour conduire aux composés de formule **(XVI) :** 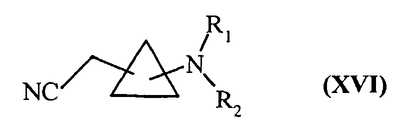 dans laquelle R₁, R₂ sont tels que définis précédemment,
composés de formule (XVI) qui sont transformés en ester selon des conditions classiques puis soumis à un agent réducteur pour conduire aux composés de formule **(XVIIA) :** 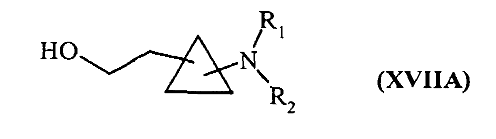 dans laquelle R₁, R₂ sont tels que définis précédemment,
composés de formule (XVIIA) qui peuvent être soumis de nouveau, et de façon répétitive, à la même série de réactions ayant conduit aux composés de formule (XVI) et (XVIIA), pour conduire aux composés de formule **(XVIIB) :** 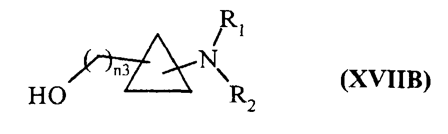 dans laquelle R₁, R₂ sont tels que définis précédemment et n₃ est un entier compris entre 3 et 6 inclus,
l'ensemble des composés de formule (XVIIA) et (XVIIB) forment les composés de formule **(XVIII) :** 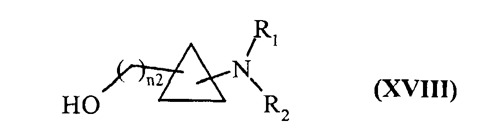 dans laquelle R₁ et R₂ sont tels que définis précédemment et n₂ est un entier compris entre 2 et 6 inclus,
composés de formule (XVIII) qui sont :
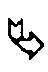 *soit* mis à réagir avec un composé de formule Y-L₁ tel que décrit précédemment, pour conduire aux composés de formule **(I/g),** cas particulier des composés de formule (I) : 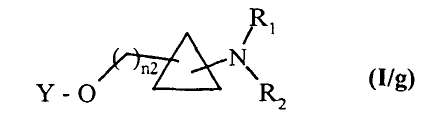 dans laquelle Y, n₂, R₁ et R₂ sont tels que décrits précédemment,
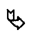 *soit* mis à réagir avec du SOCl₂ puis traités par un composé de formule (X) tel que décrit précédemment pour conduire aux composés de formule **(I/h),** cas particulier des composés de formule (I), 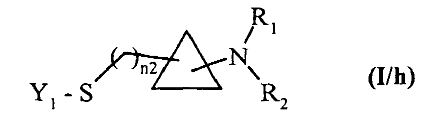 dans laquelle Y₁, n₂, R₁ et R₂ sont tels que décrits précédemment,
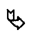 *soit* soumis à l'action d'un agent oxydant, l'aldéhyde intermédiaire obtenu étant alors mis à réagir avec une hydroxylamine de formule (XII) telle que décrite précédemment, et dans laquelle Y₂ représente spécifiquement un atome d'hydrogène puis si on le souhaite les composés obtenus sont soumis à l'action d'un composé de formule (XIV) telle que décrite précédemment, pour conduire aux composés de formule **(I/i),** cas particulier des composés de formule (I): 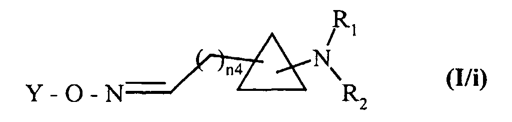 dans laquelle R₁, R₂ et Y sont tels que définis dans la formule (I) et n₄ représente un entier de valeur (n₂-1) avec n₂ tel que défini précédemment,
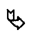 *soit* traités, après action d'un agent oxydant, dans des conditions de réaction de Wittig, puis traités dans des conditions de réduction classique de la synthèse organique, pour conduire aux composés de formule **(I/j),** cas particulier des composés de formule (I): 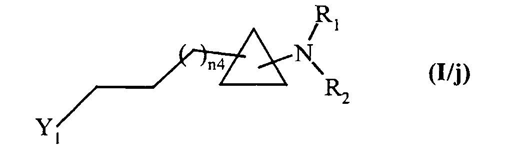 dans laquelle Y₁, n₄, R₁ et R₂ sont tels que décrits précédemment,
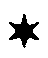 ***soit*** mis à réagir en présence de Me₃Al dans un solvant apolaire, avec un composé de formule **(XIX) :**
HNR₁R₂ **(XIX)**
dans laquelle R₁ et R₂ sont tels que définis dans la formule (I), pour conduire aux composés de formule **(XX) :** 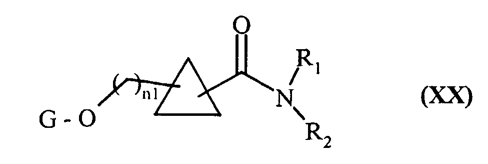 dans laquelle n₁, G, R₁ et R₂ sont tels que définis précédemment,
composés de formule (XX) qui sont soumis à l'action d'un agent réducteur classiquement utilisé en synthèse organique, pour conduire aux composés de formule **(XXI)** : 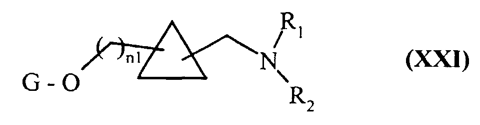 dans laquelle G, n₁, R₁ et R₂ sont tels que définis précédemment,
composés de formule (XXI) qui peuvent être soumis à l'ensemble des réactions auxquelles sont soumis les composés de formule (VII), pour conduire aux composés de formule **(I/k),** cas particulier des composés de formule (I): 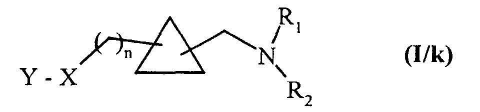 dans laquelle X, Y, n, R₁ et R₂ sont tels que définis dans la formule (I),
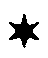 ***soit*** mis à réagir avec du chlorure de thionyle, dans le cas où R représente un atome d'hydrogène, puis mis en présence de diazométhane en milieu aqueux, pour conduire aux composés de formule **(XXII) :** 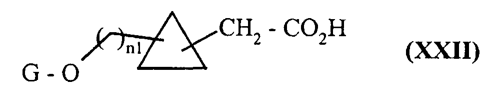 dans laquelle n₁ et G sont tels que définis précédemment,
composés de formule (XXII) qui peuvent à nouveau subir plusieurs fois la même série de réaction, pour conduire aux composés de formule **(XXIII) :** 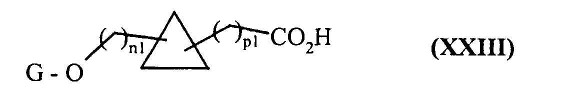 dans laquelle n₁ et G sont tels que définis précédemment, et p₁ représente un entier compris entre 2 et 6 inclus,
composés de formule (XXIII) qui sont mis à réagir avec du diphénylphosphoryle azide, hydrolysés puis traités avec un composé de formule **(VIA)** tel que décrit précédemment, pour conduire aux composés de formule **(XXIV) :** 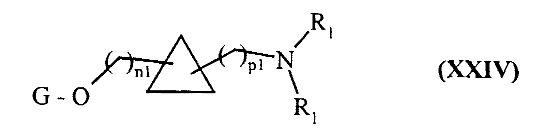 dans laquelle R₁ est tel que défini dans la formule (I) et G, n₁ et p₁ sont tels que définis précédemment,
composés de formule (XXIV) qui peuvent être soumis à l'ensemble des réactions auxquelles sont soumis les composés de formule (VII), pour conduire aux composés de formule **(I/l),** cas particulier des composés de formule (I): 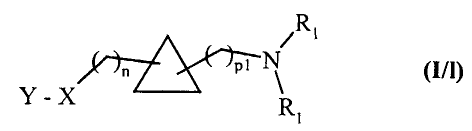 dans laquelle X, Y, R₁ et n sont tels que définis dans la formule (I) et p₁ est tel que défini précédemment,
l'ensemble des composés de formule (I/a) à (I/l) formant l'ensemble des composés de l'invention qui sont purifiés, le cas échéant, selon une technique classique de purification, qui peuvent être séparés en leurs différents isomères, selon une technique classique de séparation et qui sont transformés, le cas échéant, en leurs sels d'addition à un acide ou à une base pharmaceutiquement acceptable.

14. Compositions pharmaceutiques contenant comme principe actif au moins un composé selon l'une quelconque des revendications 1 à 12, seul ou en combinaison avec un ou plusieurs excipients ou véhicules inertes, non toxiques, pharmaceutiquement acceptables.

15. Compositions pharmaceutiques selon la revendication 14 contenant au moins un principe actif selon l'une quelconque des revendications 1 à 12 utiles comme ligand nicotinique spécifique des récepteurs α4β2.

16. Compositions pharmaceutiques selon la revendication 14, contenant au moins un principe actif selon l'une quelconque des revendications 1 à 12 utiles dans le traitement des déficits de mémoire associés au vieillissement cérébral et aux maladies neurodégénératives, ainsi que pour le traitement des troubles de l'humeur, du syndrome de Tourette, du sytndrome d'hyperactivité avec déficits attentionnels, du sevrage tabagique et de la douleur.

17. Compositions pharmaceutiques selon la revendication 14 contenant au moins un principe actif selon l'une quelconque des revendications 1 à 12, utiles dans le traitement des déficits de mémoire associés à la maladie d'Alzheimer, la maladie de Parkinson, la maladie de Pick, la maladie de Korsakoff ou les démences frontales et sous-corticales.

## Patentansprüche

1. Verbindungen der Formel (I): 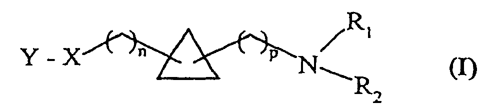 in der:
p eine ganze Zahl mit einem Wert zwischen 0 und 6 einschließlich bedeutet,
n eine ganze Zahl mit einem Wert zwischen 0 und 6 einschließlich bedeutet,
R₁ und R₂, die gleichartig oder verschieden sind, unabhängig voneinander eine Gruppe bedeuten ausgewählt aus dem Wassersoffatom, geradkettigen oder verzweigten (C₁-C₆)-Alkylgruppen, Arylgruppen und geradkettigen oder verzweiten Aryl-(C₁-C₆)-alkylgruppen, oder R₁ + R₂ gemeinsam mit dem sie tragenden Stickstoffatom ein monocyclisches oder bicyclisches, gesättigtes System mit 3 bis 10 Kettengliedern, welches gegebenenfalls ein zweites Heteroatom ausgwählt aus Sauerstoff. Stickstoff und Schwefel enthält, bilden,
X eine Gruppe bedeutet ausgewählt aus dem Sauerstoffatom, dem Schwefelatom, einer Gruppe -CH=CH-, der Methylengruppe, der Gruppe der Formel -HC=N-O- und der Gruppe der Formel -O-CH₂-CH=CH-, worin das Sauerstoffatom an die Gruppe Y der Verbindungen der Formel (I) gebunden ist,
Y eine Gruppe bedeutet ausgewählt aus Aryl, Heteroaryl, geradkettigem oder verzweigtem Aryl-(C₁-C₆)-alkyl, geradkettigem oder verzweigtem Heteroaryl-(C₁-C₆)-alkyl, -C(O)-A und -C(S)-A,
A eine Gruppe bedeutet ausgewählt aus geradkettigem oder verzweigtem(C₁-C₆)-Alkyl, Aryl, Heteroaryl, geradkettigem oder verzweigtem Aryl-(C₁-C₆)-alkyl, geradkettigem oder verzweigtem Heteroaryl-(C₁-C₆)-alkyl und NR₃R₄ worin R₃ und R₄, die gleichartig oder verschieden sind, jeweils eine Gruppe bedeuten ausgewählt aus dem Wasserstoffatom, geradkettigen oder verzweigten (C₁-C₆)-Alkylgruppen. Arylgruppen und geradkettigen oder verzweigten Aryl-(C₁-C₆)-alkylgruppen, oder R₃ + R₄ gemeinsam mit dem sie tragenden Stickstoffatom ein monocyclisches oder bicyclisches (C₃-C₁₀)-System bilden,
deren optische Isomere sowie deren Additionssalze mit einer pharmazeutisch annehmbaren Säure oder Base,
mit der Maßgabe, daß:
◆ *im Fall der 1,1-disubstituierten Verbindungen der Formel (I),*
- p von Null verschieden ist, wenn X eine Methylengruppe bedeutet, n den Wert Null aufweist, Y eine Aryl- oder Heteroarylgruppe bedeutet und R₁ und R₂, die gleichartig oder verschieden sind, ein Wasserstoffatom, eine geradkettige oder verzweigte (C₁-C₄)-Alkylgruppe, Benzylgruppe, Phenylethylgruppe bedeuten oder gemeinsam mit dem sie tragenden Stickstoffatom eine Morpholinogruppe, Thiomorpholinogruppe oder ein gesättigtes cyclisches Kohlenstoffsystem mit 5 bis 7 Kettengliedern bilden,
- p von Null verschieden ist, wenn X eine Methylengruppe bedeutet, n den Wert Null besitzt, Y eine Acetylgruppe bedeutet und R₁ und R₂, die gleichartig oder verschieden sind, ein Wasserstoffatom, eine geradkettige oder verzweigte (C₁-C₄)-Alkylgruppe, Phenylgruppe oder Benzylgruppe bedeuten oder gemeinsam mit dem sie tragenden Stickstoffatom eine Piperidinyl- oder Morpholinogruppe bilden,
- R₁ und R₂ nicht gleichzeitig eine Methylgruppe bedeuten:
* entweder, wenn p und n jeweils den Wert 1 besitzen, X ein Sauerstoffatom darstellt und Y eine Gruppe bedeutet ausgewählt aus p-Nitrobenzoyl, p-Aminobenzoyl, p-Chlorphenylaminocarbonyl und Acetyl,
* oder, wenn p den Wert Null besitzt, n den Wert 1 besitzt, X ein Sauerstoffatom oder ein Schwefelatom darstellt und Y eine 2-Chinolyl-gruppe bedeutet, die in der 3-Stellung durch eine geradkettige oder verzweigte (C₃-C₄)-Alkylgruppe substituiert ist, oder eine Phenylgruppe bedeutet,
- Y keine 1,2-Benzisoxazol-3-yl-gruppe bedeutet, wenn n den Wert 1 besitzt, p den Wert Null besitzt und X ein Sauerstoffatom darstellt,
◆ *im Fall der 1,2-disubstituierten Verbindungen der Formel (I),*
- R₁ und R₂ nicht gleichzeitig ein Wasserstoffatom bedeuten, wenn p und n jeweils den Wert Null besitzen und X-Y gemeinsam eine Phenoxygruppe (gegebenenfalls substituiert durch eine oder zwei gleichartige oder verschiedenartige Gruppen ausgewählt aus Methoxy, Dimethylamino, Halogenatomen, Methyl, Trifluormethyl, Nitro und Amino), Phenylsulfanylgruppe, Benzyloxygruppe, Benzylgruppe oder 2-Phenylethylgruppe bedeuten,
- R₁ und R₂ nicht gleichzeitig eine Methylgruppe bedeuten, wenn p und n jeweils den Wert Null besitzen und X-Y gemeinsam eine Phenoxygruppe (gegebenenfalls substituiert durch eine Gruppe ausgewählt aus dem Chloratom und der Trifluormethylgruppe), eine Phenylsulfanylgruppe oder eine Benzylgruppe bedeuten,
mit der weiteren Maßgabe, daß die Verbindungen der Formel (I) von den folgenden Verbindungen verschieden sind:
- (1-Benzylcyclopropyl)-methanamin,
- (1-Benzylcyclopropyl)-N,N-dimethylmethanamin,
- 2-(Phenoxycyclopropyl)-methanamin,
- 2-(Phenoxymethyl)-cyclopropanamin,
- (N,N-Dimethyl)-2-(acetoxymethyl)-cyclopropanmethanamin,
- N-{2-[2-(Benzyloxy)-ethyl]-cyclopropyl}-N,N-dimethylamin,
wobei es sich versteht, daß man:
■ unter einer Arylgruppe eine Phenyl-, Biphenyl-, Naphthyl-, Dihydronaphthyl-, Tetrahydronaphthyl-, Indanyl- und Indenyl-gruppe versteht, wobei jede dieser Gruppen gegebenenfalls durch eine oder mehrere, gleichartige oder verschiedenartige Gruppen substituiert ist, ausgewählt aus Halogenatomen, geradkettigen oder verzweigten (C₁-C₆)-Alkylgruppen, Hydroxygruppen, Cyanogruppen, Nitrogruppen, geradkettigen oder verzweigten (C₁-C₆)-Alkoxygruppen, geradkettigen oder verzweigten (C₂-C₇)-Acylgruppen, geradkettigen oder verzweigten (C₁-C₆)-Alkoxycarbonylgruppen, geradkettigen oder verzweigten (C₁-C₆)-Trihalogenalkylgruppen, geradkettigen oder verzweigten (C₁-C₆)-Trihalogenalkoxygruppen und Aminogruppen (gegebenenfalls substituiert durch eine oder zwei geradkettige oder verzweigte (C₁-C₆)-Alkylgruppen),
■ unter einer Heteroarylgruppe ein monocyclisches aromatisches oder bicyclisches System mit 5 bis 12 Kettengliedern versteht, welches ein bis drei gleichartige oder verschiedenartige Heteroatome ausgewählt aus Sauerstoff, Stickstoff und Schwefel enthält und bei dem einer der Ringe im Fall eines bicyclischen Systems einen aromatischen Charakter aufweist, während der andere Cyclus aromatisch oder teilweise hydriert sein kann, wobei jede dieser Gruppen gegebenenfalls durch eine oder mehrere, gleichartige oder verschiedenartige Gruppen ausgewählt aus den oben definierten Substituenten für die Arylgruppe substituiert sein kann.

2. Verbindungen der Formel (I) nach Anspruch 1, **dadurch gekennzeichnet, daß** n eine ganze Zahl mit einem Wert zwischen 0 und 2 einschließlich bedeutet, deren optische Isomere sowie deren Additionssalze mit einer pharmazeutisch annehmbaren Säure oder Base.

3. Verbindungen der Formel (I) nach Anspruch 1, **dadurch gekennzeichnet, daß** R₁ und R₂, die gleichartig oder verschieden sind, jeweils ein Wasserstoffatom oder eine geradkettige oder verzweigte (C₁-C₆)-Alkylgruppe bedeuten, deren optische Isomere sowie deren Additionssalze mit einer pharmazeutisch annehmbaren Säure oder Base.

4. Verbindungen der Formel (I) nach Anspruch 1, **dadurch gekennzeichnet, daß** X ein Sauerstoffatom bedeutet, deren optische Isomere sowie deren Additionssalze mit einer pharmazeutisch annehmbaren Säure oder Base.

5. Verbindungen der Formel (I) nach Anspruch 1, **dadurch gekennzeichnet, daß** Y eine Gruppe bedeutet ausgewählt aus -C(O)NR₃R₄, worin R₃ und R₄, die bezüglich der Formel (I) angegebenen Bedeutungen besitzen, Acetyl, -C(O)-Heteroaryl, geradkettiges oder verzweigtes Aryl-(C₁-C₆)-alkyl und Heteroaryl, deren optische Isomere sowie deren Additionssalze mit einer pharmazeutisch annehmbaren Säure oder Base.

6. Verbindungen der Formel (I) nach Anspruch 1, **dadurch gekennzeichnet, daß** Y eine Pyridinylgruppe bedeutet, deren optische Isomere sowie deren Additionssalze mit einer pharmazeutisch annehmbaren Säure oder Base.

7. Verbindungen der Formel (I) nach Anspruch 1, **dadurch gekennzeichnet, daß** sie die Verbindungen der Formel **(IA)** sind: 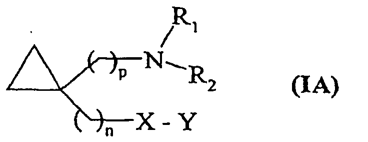 in der n, p, X, Y, R₁ und R₂ die bezüglich der Formel (I) angegebenen Bedeutungen besitzen, deren optische Isomere sowie deren Additionssalze mit einer pharmazeutisch annehmbaren Säure oder Base.

8. Verbindungen der Formel (I) nach Anspruch 1, **dadurch gekennzeichnet, daß** sie die Verbindungen der Formel **(IB)** sind: 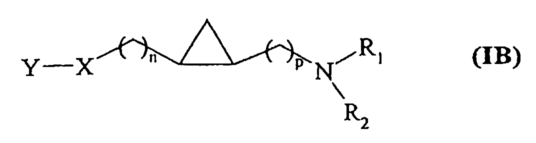 in der n, p, X, Y, R₁ und R₂ die bezüglich der Formel (I) angegebenen Bedeutungen besitzen, deren optische Isomere sowie deren Additionssalze mit einer pharmazeutisch annehmbaren Säure oder Base.

9. Verbindungen der Formel (I) nach Anspruch 1, **dadurch gekennzeichnet, daß** p eine ganze Zahl mit einem Wert von 0 oder 1 bedeutet, deren optische Isomere sowie deren Additionssalze mit einer pharmazeutisch annehmbaren Säure oder Base.

10. Verbindungen der Formel (IB) nach Anspruch 8, **dadurch gekennzeichnet, daß** p 0 oder 1 bedeutet, n 0 oder 1 bedeutet, R₁ und R₂, die gleichartig oder verschieden sind, ein Wasserstoffatom oder eine geradkettige oder verzweigte (C₁-C₆)-Alkylgruppe bedeuten, X ein Sauerstoffatom bedeutet und Y eine Gruppe bedeutet ausgewählt aus geradkettigem oder verzweigtem Phenyl-(C₁-C₆)-alkyl, Pyridinyl und -C(O)-A, worin A eine geradkettige oder verzweigte (C₁-C₆)-Alkylgruppe, (C₁-C₆)-Monoalkylaminogruppe oder (C₁-C₆)-Dialkylaminogruppe, worin der Alkylrest geradkettig oder verzweigt ist, bedeutet, deren optische Isomere sowie deren Additionssalze mit einer pharmazeutisch annehmbaren Säure oder Base.

11. Verbindungen der Formel (IA) nach Anspruch 7, **dadurch gekennzeichnet, daß** p 0 oder 1 bedeutet, n eine ganze Zahl mit einem Wert zwischen 0 und 3 einschließlich bedeutet, R₁ und R₂, die gleich oder verschieden sind, ein Wasserstoffatom, eine geradkettige oder verzweigte (C₁-C₆)-Alkylgruppe bedeuten oder gemeinsam mit dem sie tragenden Stickstoffatom einen Pyrrolidinylrest bilden, X ein Sauerstoffatom, ein Schwefelatom oder eine Gruppe -CH=CH- bedeutet und Y eine Gruppe bedeutet ausgewählt aus Phenyl (gegebenenfalls substituiert durch eine Hydroxygruppe, geradkettige oder verzweigte (C₁-C₆)-Alkylgruppe oder ein Halogenatom), Pyridinyl, geradkettiges oder verzweigtes Pyridinyl-(C₁-C₆)-alkyl (wobei der Pyridinylrest in jeder dieser Gruppen gegebenenfalls durch eine Gruppe ausgewählt aus Halogenatomen und geradkettigen oder verzweigten (C₁-C₆)-Alkylgruppen substituiert ist) und -C(O)-A, worin A eine Gruppe bedeutet ausgewählt aus geradkettigem oder verzweigtem (C₁-C₆)-Alkyl, geradkettigem oder verzweigtem (C₁-C₆)-Monoalkylamino, geradkettigem oder verzweigtem (C₁-C₆)-Dialkylamino und Pyridinyl bedeutet, deren optische Isomere sowie deren Additionssalze mit einer pharmazeutisch annehmbaren Säure oder Base.

12. Verbindungen der Formel (I) nach Anspruch 1, nämlich:
■ 2-[1-(Dimethylamino)-cyclopropyl]-ethyl-methylcarbamat,
■ 2-[1-(Dimethylamino)-cyclopropyl]-ethyl-dimethylcarbamat,
■ [1-(Dimethylamino)-cyclopropyl]-methyl-dimethylcarbamat,
■ [1-(Dimethylamino)-cyclopropyl]-methylacetat,
■ 2-[1-(Dimethylamino)-cyclopropyl]-ethylacetat,
■ 1-[(Dimethylamino)-methyl]-cyclopropylacetat,
■ [1-(Dimethylamino)-cyclopropyl]-methyl-nicotinat,
■ *N,N*-Dimethyl-1-[(3-pyridinyloxy)-methyl]-cyclopropanamin,
■ *N*-Methyl-1-[(3-pyridinyloxy)-methyl]-cyclopropanamin,
■ *N,N*-Dimethyl-1-[(3-pyridinylmethoxy)-methyl]-cyclopropanamin,
■ *N,N*-Dimethyl-1-[2-(3-pyridinyloxy)-ethyl]-cyclopropanamin,
■ 4-({2-[1-Dimethylamino)-cyclopropyl]-ethyl}-sulfanyl)-phenol,
■ (±)*cis*-2-(Dimethylamino)-cyclopropyl-methylcarbamat,
■ (±) *trans*-2-(Dimethylamino)-cyclopropyl-methylcarbamat,
■ (±)*cis*-2-(Dimethylamino)-cyclopropyl-acetat,
■ (±)*trans*-2-(Dimethylamino)-cyclopropyl-acetat,
■ (±)*cis*-2-(Dimethylamino)-cyclopropyl]-methylacetat,
■ (±)*trans*-2-(Dimethylamino)-cyclopropyl]-methylacetat,
■ (±)cis-2-[(Benzyloxy)-methyl]-*N,N*-dimethylcyclopropanamin,
■ (±)trans-2-[(Benzyloxy)-methyl]-*N,N*-dimethylcyclopropanamin,
■ (±)*trans*-2-[(Dimethylamino)-methyl]-cyclopropyl-acetat,
■ 1-[(3-Pyridinyloxy)-methyl]-cyclopropanamin, Dihydrochlorid,
■ *N*-Methyl-1-{[(6-methyl-3-pyridinyl)-oxy]-methyl}-cyclopropanamin, Hydrochlorid,
■ *N*-Methyl-1-{[(6-chlor-3-pyridinyl)-oxy]-methyl}-cyclopropanamin, Hydrochlorid,
■ *N*-{1-[(3-Fluorphenoxy)-methyl]-cyclopropyl}-*N*-methylamin, Hydrochlorid,
■ 3-[1-(Dimethylamino)-cyclopropyl]-propyl-dimethylcarbamat, Fumarat,
■ 3-[1-(Dimethylamino)-cyclopropyl]-propyl-methylcarbamat, Fumarat,
■ *N*-Methyl-1-[(2-pyridinylsulfanyl)-methyl]-cyclopropanamin, Dihydrochlorid,
■ *N*-Methyl-1-[3-(3-pyridinyloxy)-propyl]-cyclopropanamin, Dihydrochlorid,
■ *N*-Methyl-1-[2-(3-pyridinyl)-ethyl]-cyclopropanamin, Dihydrochlorid,
■ *N*-Methyl-1-[(Z)-2-(3-pyridinyl)-ethenyl]-cyclopropanamin, Fumarat,
■ [1-(1-Pyrrolidinyl)-cyclopropyl]-methyl-dimethylcarbamat, Fumarat,
■ *N,N*-Dimethyl-1-[2-(3-pyridinyl)-ethyl]-cyclopropanamin, Hydrochlorid,
■ 3-{[1-(1-Pyrrolidinyl)-cyclopropyl]-methoxy}-pyridin, Fumarat,
■ *N*-Methyl-1-[2-(3-pyridinyloxy)-ethyl]-cyclopropanamin, Fumarat,
■ 2-[1-(Methylamino)-cyclopropyl]-ethyl-dimethylcarbamat, Hydrochlorid, und
■ 2-[1-(1-Pyrrolidinyl)-cyclopropyl]-ethyl-dimethylcarbamat, Fumarat.
deren optische Isomere sowie deren Additionssalze mit einer pharmazeutisch annehmbaren Säure oder Base.

13. Verfahren zur Herstellung der Verbindungen der Formel (I), **dadurch gekennzeichnet, daß** man als Ausgangsprodukt eine Verbindung der Formel (II) verwendet: 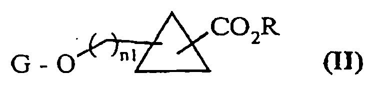 in der G eine klassischerweise in der organischen Synthese verwendete Schutzgruppe für die Hydroxyfunktionen darstellt, R ein Wasserstoffatom oder eine geradkettige oder verzweigte (C₁-C₆)-Alkylgruppe bedeutet und n₁ 0 oder 1 bedeutet, welche Verbindungen der Formel (II) man:
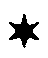 ***entweder*** mit flüssigem Ammoniak in Gegenwart eines Alkalicyanids in alkoholischem Lösungsmittel umsetzt zur Bildung der Verbindungen der Formel **(III)**: 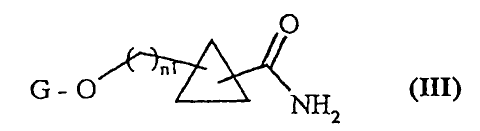 in der G eine Schutzgruppe für die Hydroxyfunktionen darstellt und n₁ 0 oder 1 bedeutet,
welche Verbindungen der Formel (III) in Gegenwart einer Base, wie Natriumhydroxid, mit einem Dihalogen umgesetzt werden zur Bildung der Verbindungen der Formel **(IV)**: 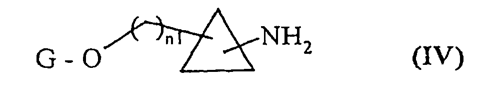 in der G und n₁ die oben angegebenen Bedeutungen besitzen,
welche Verbindungen der Formel (IV) man selektiv an der primären Aminfunktion mit einer klassischerweise in der organischen Chemie verwendeten Schutzgruppe G₂, wie die BOC-Gruppe (t-Butyloxycarbonylgruppe) schützt, zur Bildung der Verbindungen der Formel **(V)**: 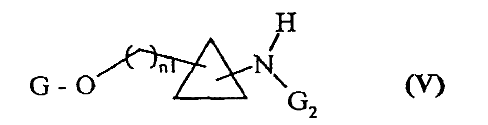 in der n₁, G und G₂ die oben angegebenen Bedeutungen besitzen,
welche Verbindungen der Formel (V) anschließend:
• in basischem Medium mit einer Verbindung der Formel **(VIA)** behandelt werden:
R₁ - L₁ **(VIA)**
in der R₁ die bezüglich der Formel (I) angegebenen Bedeutungen besitzt und L₁ eine in der organischen Synthese übliche austretende Gruppe darstellt,
• deren Schutzgruppe der Aminfunktion abgespalten wird und die dann keiner weiteren Behandlung unterzogen werden und zu den Verbindungen der Formel **(VIIA)** führen: 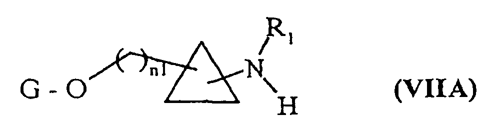 in der R₁, n₁ und G die oben angegebenen Bedeutungen besitzen,
oder mit einer Verbindung der Formel (**VIB**) umgesetzt werden:
R₂ₐ - L₁ **(VIB)**
in der R₂ₐ die gleichen Bedeutungen besitzt, wie sie für R₂ bezüglich der Formel (I) angegeben sind, mit Ausnahme der Bedeutung des Wasserstoffatoms, und L₁ die oben angegebenen Bedeutungen besitzt,
zur Bildung der Verbindungen der Formel **(VIIB)**: 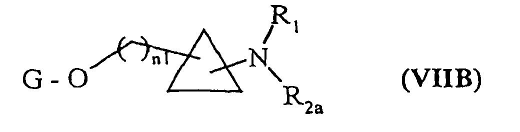 in der R₁, R₂ₐ, n₁ und G die oben angegebenen Bedeutungen besitzen,
wobei die Gesamtheit der Verbindungen der Formeln (VIIA) und (VIIB) die Verbindungen der Formel **(VII)** bilden: 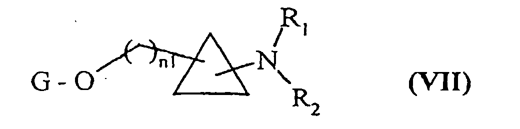 in der n₁, G, R₁ und R₂ die oben angegebenen Bedeutungen besitzen,
von welchen Verbindungen der Formel (VII) die Schutzgruppe der Hydroxyfunktion abgespalten wird und die dann:
◆ **entweder** mit einer Verbindung der Formel **(VIII)** behandelt werden:
Y - L₁ **(VIII)**
in der Y die bezüglich der Formel (I) angegebenen Bedeutungen besitzt und L₁ die oben angegebenen Bedeutungen aufweist,
zur Bildung der Verbindungen der Formel **(I/a),** einem Sonderfall der Verbindungen der Formel (I): 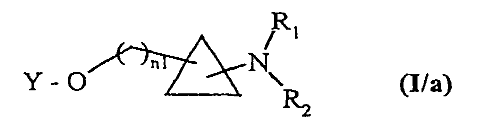 in der n₁, Y, R₁ und R₂ die bezüglich der Formel (I) angegebenen Bedeutungen besitzen,
◆ **oder** mit SOCl₂ umgesetzt werden zur Bildung der Verbindungen der Formel **(IX):** 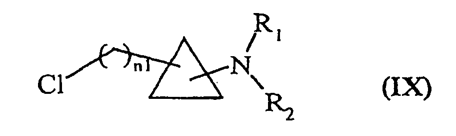 in der n₁, R₁ und R₂ die oben angegebenen Bedeutungen besitzen,
welche Verbindungen der Formel (IX) in basischem Medium mit einer Verbindung der Formel **(X)** umgesetzt werden:
Y₁-SH **(X)**
in der Y₁ eine Arylgruppe, geradkettige oder verzweigte Aryl-(C₁-C₆)-alkylgruppe, Heteroarylgruppe oder geradkettige oder verzweigte Heteroaryl-(C₁-C₆)-alkylgruppe bedeutet,
zur Bildung der Verbindungen der Formel **(I/b)**, einem Sonderfall der Verbindungen der Formel (I): 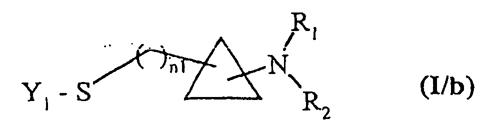 in der Y₁, n₁, R₁ und R₂ die oben angegebenen Bedeutungen besitzen.
◆ **oder** dann, wenn n₁ den Wert 1 besitzt, der Einwirkung eines klassischen Oxidationsmittels der organischen Synthese unterworfen werden zur Bildung der Verbindungen der Formel (**XI**): 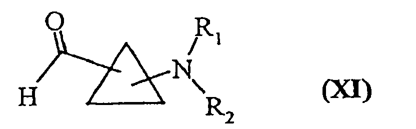 in der R₁ und R₂ die oben angegebenen Bedeutungen besitzen, welche Verbindungen der Formel (XI):
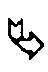 *entwede**r* mit einem Hyrdoxylamin der Formel **(XII)** behandelt werden:
H₂N - OY₂ **(XII)**
in der Y₂ ein Wasserstoffatom oder eine Gruppe Y₁, wie sie oben definiert ist, bedeutet,
zur Bildung der Verbindungen der Formel (**I/c**), einem Sonderfall der Verbindungen der Formel (I): 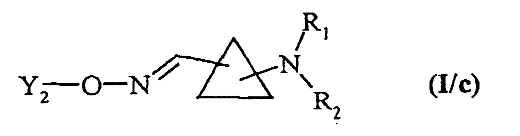 in der Y₂, R₁ und R₂ die oben angegebenen Bedeutungen besitzen,
welche Verbindungen der Formel (I/c) dann, wenn Y₂ ein Wasserstoffatom bedeutet, der Einwirkung einer Verbindung der Formel (**XIV**) unterworfen werden:
Y₃ - L₁ (**XIV**)
in der L₁ die oben angegebenen Bedeutungen besitzt und Y₃ eine Gruppe der Formel -C(O)-A oder -C(S)-A, wie sie bezüglich der Formel (I) definiert worden ist, bedeutet, zur Bildung der Verbindungen der Formel (**I/d**), einem Sonderfall der Verbindungen der Formel (I): 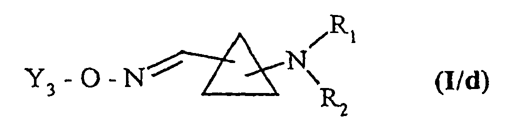 in der Y₃, R₁ und R₂ die oben angegebenen Bedeutungen besitzen,
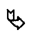 *oder* unter den Bedingungen der Wittig-Reaktion behandelt und dann der Einwirkung eines klassischen Reduktionsmittels der organischen Synthese unterworfen werden zur Bildung der Verbindungen der Formel **(I/e)**, einem Sonderfall der Verbindungen der Formel (I): 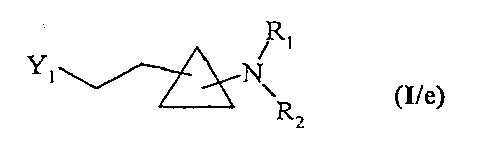 in der Y₁, R₁ und R₂ die oben angegebenen Bedeutungen besitzen,
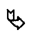 *oder* der Einwirkung einer Verbindung der Formel Ph₃P=CH-CO₂Et unterworfen und dann mit einem Reduktionsmittel der organischen Synthese reduziert werden zur Bildung der Verbindungen der Formel **(XV)**: 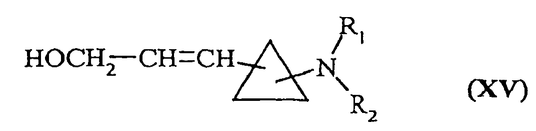 in der R₁ und R₂ die oben angegebenen Bedeutungen besitzen,
welche Verbindungen der Formel (XV) mit einer Verbindung der Formel (VIII), wie sie oben definiert worden ist, behandelt werden zur Bildung der Verbindungen der Formel **(I/f)**, einem Sonderfall der Verbindungen der Formel (I): 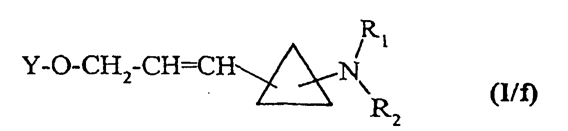 in der Y, R₁ und R₂ die bezüglich der Formel (I) angegebenen Bedeutungen besitzen.
◆ **oder** dann, wenn n₁ den Wert 1 besitzt, unter Anwendung üblicher Bedingungen der organischen Synthese in ihr entsprechendes Halogenderivat umgewandelt und dann mit einem Alkalicyanid in Gegenwart von Dimethylsulfoxid umgesetzt werden zur Bildung der Verbindungen der Formel **(XVI):** 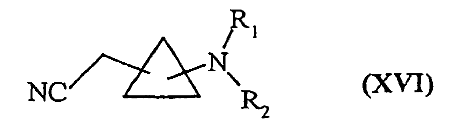 in der R₁ und R₂ die oben angegebenen Bedeutungen besitzen,
welche Verbindungen der Formel (XVI) unter Anwendung klassischer Bedingungen in den Ester überführt und dann mit einem Reduktionsmittel behandelt werden zur Bildung der Verbindungen der Formel (**XVIIA**): 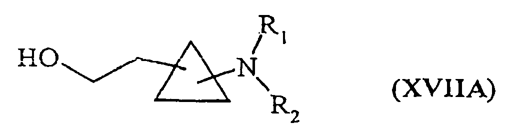 in der R₁ und R₂ die oben angegebenen Bedeutungen besitzen,
welche Verbindungen der Formel (XVIIA) erneut und wiederholt der gleichen Folge von Reaktionen unterworfen werden können, die zu den Verbindungen der Formeln (XVI) und (XVIIA) führen,
zur Bildung der Verbindungen der Formel (**XVIIB**): 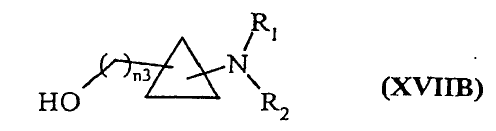 in der R₁ und R₂ die oben angegebenen Bedeutungen besitzen und n₃ eine ganze Zahl mit einem Wert zwischen 3 und 6 einschließlich bedeutet,
wobei die Gesamtheit der Verbindungen der Formeln (XVIIA) und (XVIIB) die Verbindungen der Formel **(XVIII)** bildet: 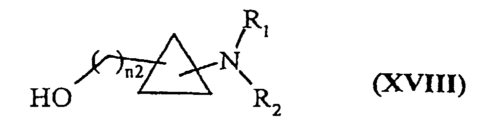 in der R₁ und R₂ die oben angegebenen Bedeutungen besitzen und n₂ eine ganze Zahl mit einem Wert zwischen 2 und 6 einschließlich bedeutet,
welche Verbindungen der Formel (XVIII):
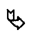 *entweder* mit einer Verbindung der Formel Y-L₁, wie sie oben beschrieben worden ist, umgesetzt werden zur Bildung der Verbindungen der Formel (**I/g**), einem Sonderfall der Verbindungen der Formel (I): 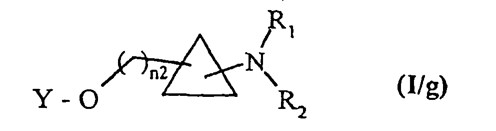 in der Y, n₂, R₁ und R₂ die oben angegebenen Bedeutungen besitzen,
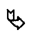 *oder* mit SOCl₂ umsetzt und dann mit einer Verbindung der Formel (X), wie sie oben beschrieben worden ist, behandelt werden zur Bildung der Verbindungen der Formel (**I/h**), einem Sonderfall der Verbindungen der Formel (I): 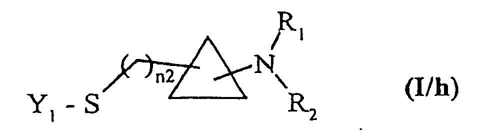 in der Y₁, n₂, R₁ und R₂ die oben angegebenen Bedeutungen besitzen,
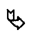 oder der Einwirkung eines Oxidationsmittels unterworfen werden, wonach der als Zwischenprodukt erhaltene Aldehyd mit einem Hydroxylamin der Formel (XII), wie sie oben definiert worden ist und in der Y₂ spezifisch ein Wasserstoffatom bedeutet, umgesetzt werden, wonach man gewünschtenfalls die erhaltenen Verbindungen der Einwirkung einer Verbindung der Formel (XIV), wie sie oben definiert worden ist, unterworfen werden zur Bildung der Verbindungen der Formel (**I/i**), einem Sonderfall der Verbindungen der Formel (I): 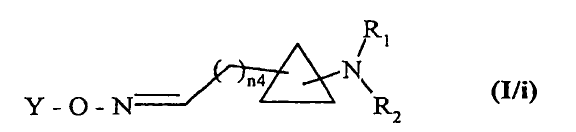 in der R₁, R₂ und Y die bezüglich der Formel (I) angegebenen Bedeutungen besitzen und n₄ eine ganze Zahl mit einem Wert von (n₂-1) darstellt, worin n₂ die oben angegebenen Bedeutungen besitzt,
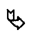 *oder* nach der Einwirkung eines Oxidationsmittels den Bedingungen der Wittig-Reaktion unterworfen werden und dann bei den Bedingungen der klassischen Reduktion der organischen Synthese behandelt werden zur Bildung der Verbindungen der Formel (**I/j**), einem Sonderfall der Verbindungen der Formel (I): 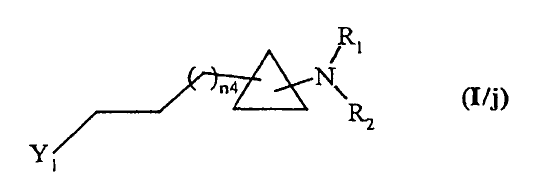 in der Y₁, n₄, R₁ und R₂ die oben angegebenen Bedeutungen besitzen,
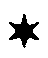 ***oder*** in einem apolaren Lösungsmittel in Gegenwart von Me₃Al mit einer Verbindung der Formel (**XIX**) umgesetzt werden:
HNR₁R₂ (**XIX**)
in der R₁ und R₂ die bezüglich der Formel (I) angegebenen Bedeutungen besitzen, zur Bildung der Verbindungen der Formel (**XX**): 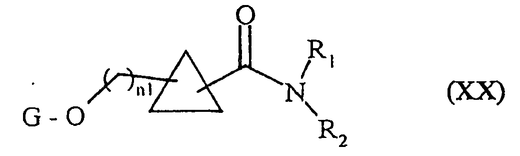 in der n₁, G, R₁ und R₂ die oben angegebenen Bedeutungen besitzen,
welche Verbindungen der Formel (XX) der Einwirkung eines in klassischer Weise in der organischen Synthese verwendeten Reduktionsmittels unterworfen werden zur Bildung der Verbindungen der Formel (**XXI**): 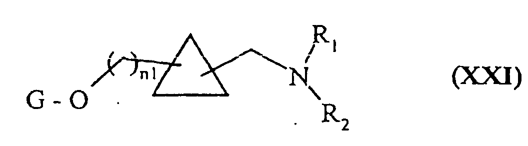 in der G, n₁, R₁ und R₂ die oben angegebenen Bedeutungen besitzen,
welche Verbindungen der Formel (XXI) der Gesamtheit der Reaktionen unterworfen werden können, welche die Verbindungen der Formel (VII) unterworfen worden sind, zur Bildung der Verbindungen der Formel **(I/k)**, einem Sonderfall der Verbindungen der Formel (I): 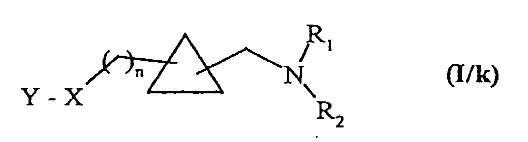 in der X, Y, n, R₁ und R₂ die bezüglich der Formel (I) angegebenen Bedeutungen besitzen,
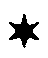 ***oder*** in dem Fall, da R ein Wasserstoffatom bedeutet, mit Thionylchlorid umgesetzt werden und dann mit Diazomethan umgesetzt werden zur Bildung der Verbindungen der Formel (**XXII**): 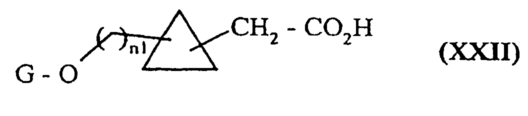 in der n₁ und G die oben angegebenen Bedeutungen besitzen,
wobei die Verbindungen der Formel (XXII) erneut mehrfach der gleichen Folge von Reaktionen unterworfen werden können, zur Bildung der Verbindungen der Formel **(XXIII):** 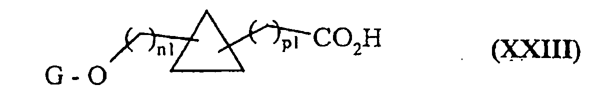 in der n₁ und G die oben angegebenen Bedeutungen besitzen und p₁ eine ganze Zahl mit einem Wert zwischen 2 und 6 einschließlich bedeutet,
welche Verbindungen der Formel (XXIII) mit Diphenylphosphorylazid umgesetzt, dann hydrolysiert und schließlich mit einer Verbindung der Formel (VIA), wie sie oben beschrieben worden ist, behandelt werden zur Bildung der Verbindungen der Formel **(XXIV):** 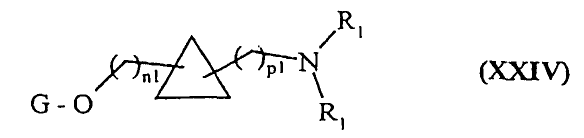 in der R₁ die bezüglich der Formel (I) angegebenen Bedeutungen besitzt und G, n₁ und p₁ die oben angegebenen Bedeutungen aufweisen,
welche Verbindungen der Formel (XXIV) der Gesamtheit der Reaktionen unterworfen werden können, welchen die Verbindungen der Formel (VII) unterworfen worden sind, zur Bildung der Verbindungen der Formel **(I/l),** einem Sonderfall der Verbindungen der Formel (I): 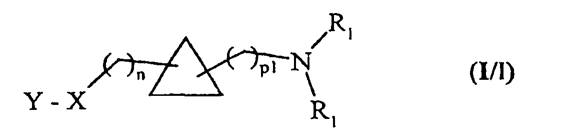 in der X, Y, R₁ und n die bezüglich der Formel (I) angegebenen Bedeutungen besitzen und p₁ die oben angegebenen Bedeutungen aufweist,
wobei die Gesamtheit der Verbindungen der Formeln (I/a) bis (I/1), welche die Gesamtheit der erfindungsgemäßen Verbindungen bildet, gegebenenfalls mit Hilfe einer klassischen Reinigungsmethode gereinigt werden, mit Hilfe einer klassischen Trennmethode in ihre verschiedenen Isomeren aufgetrennt werden können und welche gegebenenfalls in ihre Additionssalze mit einer pharmazeutisch annehmbaren Säure oder Base überführt werden können.

14. Pharmazeutische Zubereitungen, enthaltend als Wirkstoff mindestens eine Verbindung nach einem der Ansprüche 1 bis 12 allein oder in Kombination mit einem oder mehreren inerten, nichttoxischen, pharmazeutisch annehmbaren Trägermaterialien oder Hilfsstoffen.

15. Pharmazeutische Zubereitungen nach Anspruch 14, enthaltend mindestens einen Wirkstoff nach einem der Ansprüche 1 bis 12, nützlich als Nicotinligand, der spezifisch ist für die Rezeptoren α4β2.

16. Pharmazeutische Zubereitungen nach Anspruch 14, enthaltend mindestens einen Wirkstoff nach einem der Ansprüche 1 bis 12, nützlich bei der Behandlung von Gedächtnismängeln, die mit dem Altern des Gehirns und neurodegenerativen Erkrankungen verknüpft sind, sowie zur Behandlung von Gemütsstörungen, des Tourette-Syndroms, des Hyperaktivitätssyndroms mit Aufmerksamkeitsmängeln, des Tabakentzugs und von Schmerzen.

17. Pharmazeutische Zubereitungen nach Anspruch 14, enthaltend mindestens einen Wirkstoff nach einem der Ansprüche 1 bis 12, nützlich für die Behandlung von Gedächtnismängeln, die mit der Alzheimerschen Krankheit, der Parkinsonschen Krankheit, der Pickschen Krankheit, der Korsakoff-Krankheit oder mit frontalen und subkortikalen Demenzien verknüpft sind.

## Claims

1. Compounds of formula (I) : 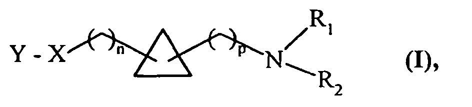 wherein :
p represents an integer of from 0 to 6 inclusive,
n represents an integer of from 0 to 6 inclusive,
R₁ and R₂, which may be identical or different, each independently of the other represent a group selected from a hydrogen atom, a linear or branched (C₁-C₆)alkyl group, an aryl group and an aryl-(C₁-C₆)alkyl group in which the alkyl moiety is linear or branched, or R₁+R₂ form together with the nitrogen atom carrying them a saturated, 3- to 10-membered, monocyclic or bicyclic system optionally containing a second hetero atom selected from oxygen, nitrogen and sulphur,
X represents a group selected from an oxygen atom, a sulphur atom, a group -CH=CH-, methylene, a group of formula -HC=N-O- and a group of formula -O-CH₂-CH=CH-, in which groups the oxygen atom is linked to the Y moiety of the compounds of formula (I),
Y represents a group selected from aryl, heteroaryl, aryl-(C₁-C₆)alkyl in which the alkyl moiety is linear or branched, heteroaryl-(C₁-C₆)alkyl in which the alkyl moiety is linear or branched, -C(O)-A and -C(S)-A,
A represents a group selected from linear or branched (C₁-C₆)alkyl, aryl, heteroaryl, aryl-(C₁-C₆)alkyl in which the alkyl moiety is linear or branched, heteroaryl-(C₁-C₆)alkyl in which the alkyl moiety is linear or branched, and NR₃R₄ wherein R₃ and R₄, which may be identical or different, each represent a group selected from a hydrogen atom, a linear or branched (C₁-C₆)alkyl group, an aryl group and an aryl-(C₁-C₆)alkyl group in which the alkyl moiety is linear or branched, or R₃+R₄ form together with the nitrogen atom carrying them a monocyclic or bicyclic (C₃-C₁₀) system,
their optical isomers and addition salts thereof with a pharmaceutically acceptable acid or base,
with the proviso that:
* *in the case of 1*,*1-disubstituted compounds of formula (I),*
- p is other than zero when X represents a methylene group, n has the value zero, Y represents an aryl or heteroaryl group, and R₁ and R₂, which may be identical or different, represent a hydrogen atom, a linear or branched (C₁-C₄)alkyl group, a benzyl group, a phenylethyl group, or form together with the nitrogen atom carrying them a morpholino group, a thiomorpholino group or a 5- to 7-membered saturated carbocyclic system,
- p is other than zero when X represents a methylene group, n has the value zero, Y represents an acetyl group, and R₁ and R₂, which may be identical or different, represent a hydrogen atom, a linear or branched (C₁-C₄)alkyl group, a phenyl group, a benzyl group, or form together with the nitrogen atom carrying them a piperidyl or morpholino group,
- R₁ and R₂ do not simultaneously represent a methyl group :
* either when p and n each have the value 1, X represents an oxygen atom and Y represents a group selected from p-nitrobenzoyl, p-aminobenzoyl, p-chlorophenylaminocarbonyl and acetyl,
* or when p has the value zero, n has the value 1, X represents an oxygen atom or a sulphur atom and Y represents a 2-quinolyl group substituted in the 3-position by a linear or branched (C₃-C₄)alkyl group, or a phenyl group,
- Y does not represent a 1,2-benzisoxazol-3-yl group when n has the value 1, p has the value zero and X represents an oxygen atom,
* *in the case of 1,2-disubstituted compounds of formula (I),*
- R₁ and R₂ do not simultaneously represent a hydrogen atom when p and n each have the value zero and X-Y together represent a phenoxy group (optionally substituted by one or two identical or different groups selected from methoxy, dimethylamino, halogen, methyl, trifluoromethyl, nitro and amino), a phenylsulphanyl group, a benzyloxy group, a benzyl group or a 2-phenylethyl group,
- R₁ and R₂ do not simultaneously represent a methyl group when p and n each have the value zero and X-Y together represent a phenoxy group (optionally substituted by a group selected from a chlorine atom or trifluoromethyl), a phenylsulphanyl group or a benzyl group,
and also with the proviso that the compounds of formula (I) are other than the following compounds :
- (1-benzylcyclopropyl)methanamine,
- (1-benzylcyclopropyl)-N,N-dimethylmethanamine,
- 2-(phenoxycyclopropyl)methanamine,
- 2-(phenoxymethyl)-cyclopropanamine,
- (N,N-dimethyl)-2-(acetoxymethyl)-cyclopropanemethanamine,
- N-{2-[2-(benzyloxy)ethyl]cyclopropyl}-N,N-dimethylamine,
it also being understood that :
■ an aryl group denotes a phenyl, biphenyl, naphthyl, dihydronaphthyl, tetrahydronaphthyl, indanyl or indenyl group, each of those groups being optionally substituted by one or more identical or different groups selected from halogen atoms, linear or branched (C₁-C₆)alkyl, hydroxy, cyano, nitro, linear or branched (C₁-C₆)alkoxy, linear or branched (C₂-C₇)acyl, linear or branched (C₁-C₆)alkoxycarbonyl, linear or branched (C₁-C₆)trihaloalkyl and linear or branched (C₁-C₆)trihaloalkoxy groups, and amino groups (optionally substituted by one or two linear or branched (C₁-C₆)alkyl groups),
■ a heteroaryl group denotes a 5- to 12-membered, monocyclic aromatic or bicyclic system containing from one to three identical or different hetero atoms selected from oxygen, nitrogen and sulphur, one of the rings of which, in the case of a bicyclic system, is aromatic in character, and the other ring of which may be aromatic or partially hydrogenated, each of those groups being optionally substituted by one or more identical or different groups selected from the substituents defined hereinbefore for an aryl group.

2. Compounds of formula (I) according to claim 1, **characterised in that** n is an integer of from 0 to 2 inclusive, their optical isomers and addition salts thereof with a pharmaceutically acceptable acid or base.

3. Compounds of formula (I) according to claim 1, **characterised in that** R₁ and R₂, which may be identical or different, each represent a hydrogen atom or a linear or branched (C₁-C₆)alkyl group, their optical isomers and addition salts thereof with a pharmaceutically acceptable acid or base.

4. Compounds of formula (I) according to claim 1, **characterised in that** X represents an oxygen atom, their optical isomers and addition salts thereof with a pharmaceutically acceptable acid or base.

5. Compounds of formula (I) according to claim 1, **characterised in that** Y represents a group selected from -C(O)NR₃R₄ wherein R₃ and R₄ are as defined for formula (I), acetyl, -C(O)-heteroaryl, aryl-(C₁-C₆)alkyl in which the alkyl moiety is linear or branched, and heteroaryl, their optical isomers and addition salts thereof with a pharmaceutically acceptable acid or base.

6. Compounds of formula (I) according to claim 1, **characterised in that** Y represents a pyridyl group, their optical isomers and addition salts thereof with a pharmaceutically acceptable acid or base.

7. Compounds of formula (I) according to claim 1, **characterised in that** they represent compounds of formula **(IA)** : 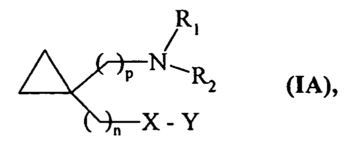 wherein n, p, X, Y, R₁ and R₂ are as defined for formula (I), their optical isomers and addition salts thereof with a pharmaceutically acceptable acid or base.

8. Compounds of formula (I) according to claim 1, **characterised in that** they represent compounds of formula **(IB) :** 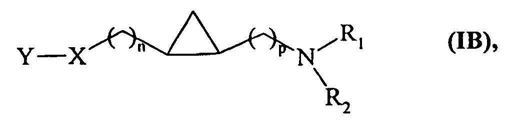 wherein n, p, X, Y, R₁ and R₂ are as defined for formula (I), their optical isomers and addition salts thereof with a pharmaceutically acceptable acid or base.

9. Compounds of formula (I) according to claim 1, **characterised in that** p is an integer having the value 0 or 1, their optical isomers and addition salts thereof with a pharmaceutically acceptable acid or base.

10. Compounds of formula (IB) according to claim 8, **characterised in that** p represents 0 or 1, n represents 0 or 1, R₁ and R₂, which may be identical or different, represent a hydrogen atom or a linear or branched (C₁-C₆)alkyl group, X represents an oxygen atom and Y represents a group selected from phenyl-(C₁-C₆)alkyl in which the alkyl moiety is linear or branched, pyridyl, and -C(O)-A wherein A represents a linear or branched (C₁-C₆)alkyl group, mono(C₁-C₆)alkylamino or di(C₁-C₆)alkylamino, an alkyl moiety being linear or branched, their optical isomers and addition salts thereof with a pharmaceutically acceptable acid or base.

11. Compounds of formula (IA) according to claim 7, **characterised in that** p represents 0 or 1, n is an integer of from 0 to 3 inclusive, R₁ and R₂, which may be identical or different, represent a hydrogen atom, a linear or branched (C₁-C₆)alkyl group or form together with the nitrogen atom carrying them a pyrrolidinyl radical, X represents an oxygen atom, a sulphur atom or a group -CH=CH-, and Y represents a group selected from phenyl (optionally substituted by a hydroxy group, a linear or branched (C₁-C₆)alkyl group or a halogen atom), pyridyl, pyridyl-(C₁-C₆)alkyl in which the alkyl moiety is linear or branched (the pyridyl radical in each of those groups being optionally substituted by a group selected from a halogen atom and a linear or branched (C₁-C₆)alkyl group), and -C(O)-A wherein A represents a group selected from linear or branched (C₁-C₆)alkyl, linear or branched mono(C₁-C₆)alkylamino, linear or branched di(C₁-C₆)alkylamino, and pyridyl, their optical isomers and addition salts thereof with a pharmaceutically acceptable acid or base.

12. Compounds of formula (I) according to claim 1, which are :
■ 2-[1-(dimethylamino)cyclopropyl]ethyl methylcarbamate,
■ 2-[1-(dimethylamino)cyclopropyl]ethyl dimethylcarbamate,
■ [1-(dimethylamino)cyclopropyl]methyl dimethylcarbamate,
■ [1-(dimethylamino)cyclopropyl]methyl acetate,
■ 2-[1-(dimethylamino)cyclopropyl]ethyl acetate,
■ 1-[(dimethylamino)methyl]cyclopropyl acetate,
■ [1-(dimethylamino)cyclopropyl]methyl nicotinate,
■ *N,N* dimethyl-1-[(3-pyridyloxy)methyl]cyclopropanamine,
■ *N*-methyl-1-[(3-pyridyloxy)methyl]cyclopropanamine,
■ *N,N*-dimethyl-1-[(3-pyridylmethoxy)methyl]cyclopropanamine,
■ *N,N*-dimethyl-1-[2-(3-pyridyloxy)ethyl]cyclopropanamine,
■ 4-({2-[1-dimethylamino)cyclopropyl]ethyl}sulphanyl)phenol,
■ (±)-*cis*-2-(dimethylamino)cyclopropyl methylcarbamate,
■ (±)-*trans-*2-(dimethylamino)cyclopropyl methylcarbamate,
■ (±)-*cis*-2-(dimethylamino)cyclopropyl acetate,
■ (±)-*trans-*2-(dimethylamino)cyclopropyl acetate,
■ (±)-*cis-*2-(dimethylamino)cyclopropyl]methyl acetate,
■ (±)-*trans-*2-(dimethylamino)cyclopropyl]methyl acetate,
■ (±)-*cis*-2-[(benzyloxy)methyl]-*N,N*-dimethyleyclopropanamine,
■ (±)-*trans-*2-[(benzyloxy)methyl]-*N,N*-dimethylcydopropanamine,
■ (±)-*trans*-2-[(dimethylamino)methyl]cyclopropyl acetate,
■ 1-[(3-pyridyloxy)methyl]cyclopropanamine dihydrochloride,
■ *N*-methyl-1-{[(6-methyl-3-pyridyl)oxy]methyl}cyclopropanamine hydrochloride,
■ *N*-methyl-1-([(6-chloro-3-pyridyl)oxy]methyl}cydopropanamine hydrochloride,
■ *N*-{1-[(3-fluorophenoxy)methyl]cyclopropyl}-*N*-methylamine hydrochloride,
■ 3-[1-(dimethylamino)cyclopropyl]propyl dimethylcarbamate fumarate,
■ 3-[1-(dimethylamino)cyclopropyl]propyl methylcarbamate fumarate,
■ *N*-methyl-1-[(2-pyridylsulphanyl)methyl]cyclopropanamine dihydrochloride,
■ *N*-methyl-1-[3-(3-pyridyloxy)propyl]cyclopropanamine dihydrochloride,
■ *N*-methyl-1-[2-(3-pyridyl)ethyl]cyclopropanamine dihydrochloride,
■ *N*-methyl-1-[(Z)-2-(3-pyridyl)ethenyl]cyclopropanamine fumarate,
■ [1-(1-pyrrolidinyl)cyclopropyl]methyl dimethylcarbamate fumarate,
■ *N,N-*dimethyl-1-[2-(3-pyridyl)ethyl]cyclopropanamine hydrochloride,
■ 3-{[1-(1-pyrrolidinyl)cyclopropyl]methoxy}pyridine fumarate,
■ *N*-methyl-1-[2-(3-pyridyloxy)ethyl]cyclopropanamine fumarate,
■ 2-[1-(methylamino)cyclopropyl]ethyl dimethylcarbamate hydrochloride, and
■ 2-[1-(1-pyrrolidinyl)cyclopropyl]ethyl dimethylcarbamate fumarate,
their optical isomers and addition salts thereof with a pharmaceutically acceptable acid or base.

13. Process for the preparation of compounds of formula (I), **characterised in that** there is used as starting material a compound of formula **(II)** : 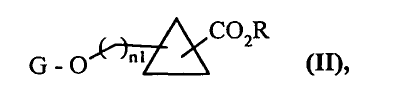 wherein G represents a protecting group for hydroxy functions that is conventionally used in organic synthesis, R represents a hydrogen atom or a linear or branched (C₁-C₆)alkyl group, and n₁ represents 0 or 1,
which compounds of formula (II) are :
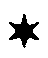 ***either*** reacted with liquid ammonia in the presence of an alkali metal cyanide in an alcoholic solvent to yield the compounds of formula **(III)** : 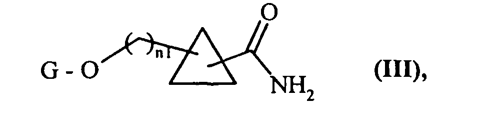 wherein G represents a protecting group for hydroxy functions and n₁ represents 0 or 1,
which compounds of formula (III) are treated with a dihalogen in the presence of a base such as sodium hydroxide to yield the compounds of formula **(IV)** : 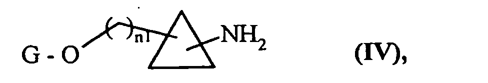 wherein G and n₁ are as defined hereinbefore,
the primary amine function of which compounds of formula (IV) is selectively protected by a protecting group G₂ customarily used in organic chemistry such as the group BOC (t-butoxycarbonyl) to yield the compounds of formula **(V)** : 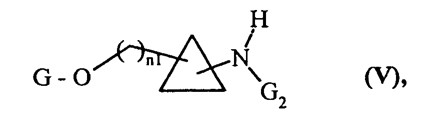 wherein n₁, G and G₂ are as defined hereinbefore,
which compounds of formula (V) are then successively :
• treated, in a basic medium, with a compound of formula **(VIA)** :
R₁ - L₁ **(VIA)**,
wherein R₁ is as defined for formula (I) and L₁ represents a customary leaving group of organic synthesis,
• the amine function of which is then deprotected, which compounds either undergo no further treatment and consequently yield compounds of formula **(VIIA)** : 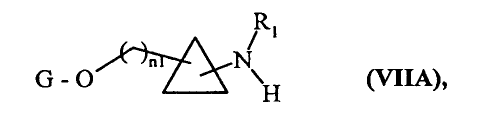 wherein R₁, n₁ and G are as defined hereinbefore,
or are reacted with a compound of formula **(VIB) :**
R₂ₐ - L₁ **(VIB),**
wherein R₂ₐ has the same meanings as R₂ as defined for formula (I) except for the meaning of a hydrogen atom and L₁ is as defined hereinbefore,
to yield compounds of formula **(VIIB) :** 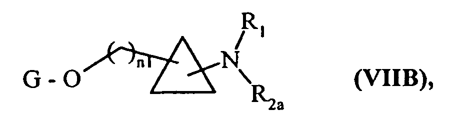 wherein R₁, R₂ₐ, n₁ and G are as defined hereinbefore,
the totality of the compounds of formulae (VIIA) and (VIIB) forming the compounds of formula **(VII)** : 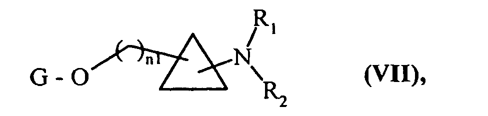 wherein n₁, G, R₁ and R₂ are as defined hereinbefore,
the hydroxy function of which compounds of formula (VII) is deprotected, which compounds are then :
◆ **either** treated with a compound of formula **(VIII)**:
Y - L₁ **(VIII),**
wherein Y is as defined for formula (I) and L₁ is as defined hereinbefore,
to yield compounds of formula **(I/a)**, a particular case of the compounds of formula (I) : 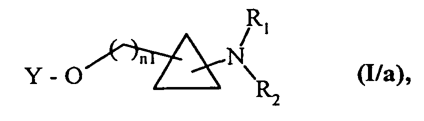 wherein n₁, Y, R₁ and R₂ are as defined for formula (I),
◆ or reacted with SOCl₂ to yield compounds of formula **(IX)** : 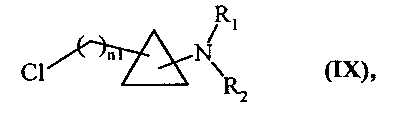 wherein n₁, R₁ and R₂ are as defined hereinbefore,
which compounds of formula (IX) are reacted, in a basic medium, with a compound of formula **(X)** :
Y₁ - SH **(X)**,
wherein Y₁ represents an aryl group, an aryl-(C₁-C₆)alkyl group in which the alkyl moiety is linear or branched, a heteroaryl group or a heteroaryl-(C₁-C₆)alkyl group in which the alkyl moiety is linear or branched,
to yield compounds of formula **(I/b)**, a particular case of the compounds of formula (I) : 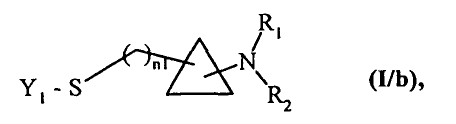 wherein Y₁, n₁, R₁ and R₂ are as defined hereinbefore,
◆ **or**, when n₁ has the value 1, subjected to the action of a conventional oxidising agent of organic synthesis to yield compounds of formula **(XI) :** 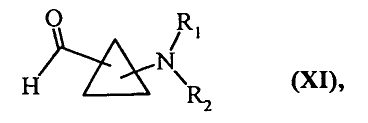 wherein R₁ and R₂ are as defined hereinbefore,
which compounds of formula (XI) are :
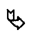 *either* treated with a hydroxylamine of formula (**XII**) :
H₂N - OY₂ **(XII)**,
wherein Y₂ represents a hydrogen atom or a group Y₁ as defined hereinbefore,
to yield compounds of formula **(I/c)**, a particular case of the compounds of formula (I) : 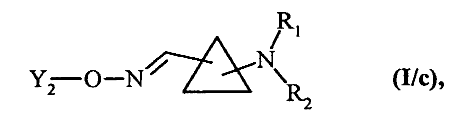 wherein Y₂, R₁ and R₂ are as defined hereinbefore,
which compounds of formula (I/c), in the particular case where Y₂ represents a hydrogen atom, are subjected to the action of a compound of formula **(XIV) :**
Y₃ - L₁ **(XIV),**
wherein L₁ is as defined hereinbefore and Y₃ represents a group of formula -C(O)-A or -C(S)-A as defined for formula (I), to yield compounds of formula **(I/d),** a particular case of the compounds of formula (1) : 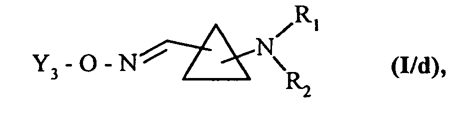 wherein Y₃, R₁ and R₂ are as defined hereinbefore,
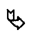 *or* treated under Wittig reaction conditions and then subjected to the action of a customary reducing agent of organic synthesis to yield compounds of formula **(I/e),** a particular case of the compounds of formula (I), 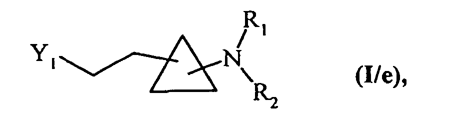 wherein Y₁, R₁ and R₂ are as defined hereinbefore,
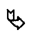 *or* subjected to the action of a compound of formula Ph₃P=CH-CO₂Et and then reduced by the action of a reducing agent of organic synthesis to yield compounds of formula (XV) : 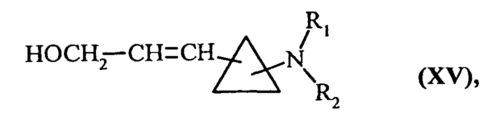 wherein R₁ and R₂ are as defined hereinbefore,
which compounds of formula (XV) are treated with a compound of formula (VIII) as defined hereinbefore to yield compounds of formula **(I/f),** a particular case of the compounds of formula (I) : 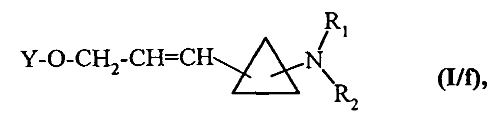 wherein Y, R₁ and R₂ are as defined for formula (I),
◆ **or**, when n₁ has the value 1, converted into their corresponding halogenated derivative under customary conditions of organic chemistry and then reacted with an alkali metal cyanide in the presence of dimethyl sulphoxide to yield compounds of formula **(XVI)** : 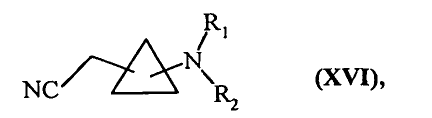 wherein R₁, R₂ are as defined hereinbefore,
which compounds of formula (XVI) are converted into an ester under conventional conditions and then subjected to a reducing agent to yield compounds of formula **(XVIIA)** : 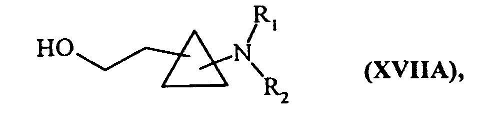 wherein R₁ and R₂ are as defined hereinbefore,
which compounds of formula (XVIIA) may be subjected again, in repetitive manner, to the same series of reactions that yielded the compounds of formulae (XVI) and (XVIIA) to yield compounds of formula **(XVIIB) :** 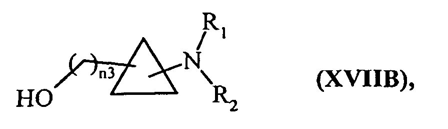 wherein R₁ and R₂ are as defined hereinbefore and n₃ is an integer of from 3 to 6 inclusive, the totality of the compounds of formulae (XVIIA) and (XVIIB) forming the compounds of formula **(XVIII)** : 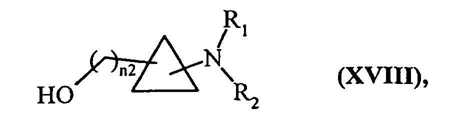 wherein R₁ and R₂ are as defined hereinbefore and n₂ is an integer of from 2 to 6 inclusive, which compounds of formula (XVIII) are :
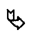 *either* reacted with a compound of formula Y-L₁ as described hereinbefore to yield compounds of formula **(I/g),** a particular case of the compounds of formula (I) : 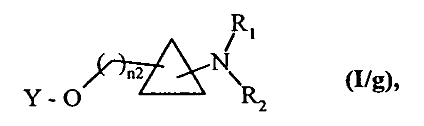 wherein Y, n₂, R₁ and R₂ are as defined hereinbefore,
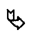 or reacted with SOCl₂ and then treated with a compound of formula (X) as described hereinbefore to yield compounds of formula (**I/h**), a particular case of the compounds of formula (I), 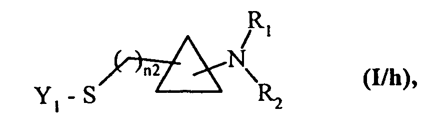 wherein Y₁, n₂, R₁ and R₂ are as defined hereinbefore,
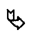 *or* subjected to the action of an oxidising agent, the aldehyde intermediate obtained then being reacted with a hydroxylamine of formula (XII) as described hereinbefore and wherein Y₂ specifically represents a hydrogen atom and then, if desired, the compounds obtained are subjected to the action of a compound of formula (XIV) as described hereinbefore to yield compounds of formula **(I/i),** a particular case of the compounds of formula **(I) :** 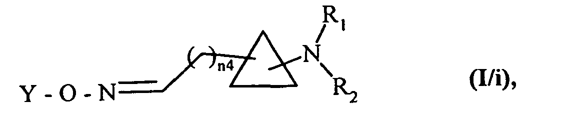 wherein R₁, R₂ and Y are as defined for formula (I) and n₄ represents an integer having a value of (n₂-1) wherein n₂ is as defined hereinbefore,
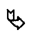 *or* treated, after the action of an oxidising agent, under Wittig reaction conditions and then treated under conventional reduction conditions of organic synthesis to yield compounds of formula **(I/j),** a particular case of the compounds of formula (I) : 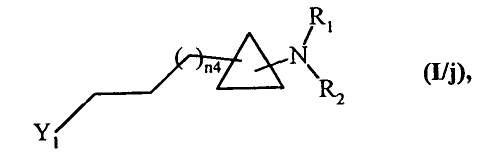 wherein Y₁, n₄, R₁ and R₂ are as described hereinbefore,
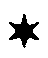 ***or*** reacted in the presence of Me₃Al in a non-polar solvent with a compound of formula **(XIX)** :
HNR₁R₂ **(XIX)**,
wherein R₁ and R₂ are as defined for formula (I), to yield compounds of formula **(XX)** : 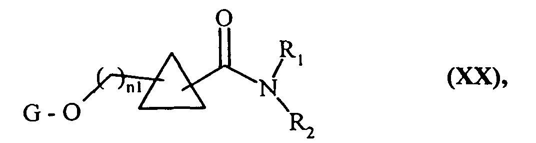 wherein n₁, G, R₁ and R₂ are as defined hereinbefore,
which compounds of formula (XX) are subjected to the action of a reducing agent conventionally used in organic synthesis, to yield the compounds of formula (**XXI**) : 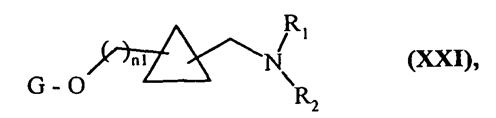 wherein G, n₁, R₁ and R₂ are as defined hereinbefore,
which compounds of formula (XXI) may be subjected to the totality of reactions to which the compounds of formula (VII) are subjected, to yield compounds of formula **(I/k)**, a particular case of the compounds of formula (I) : 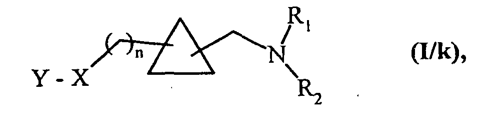 wherein X, Y, n, R₁ and R₂ are as defined for formula (I),
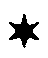 ***or*** reacted with thionyl chloride, when R represents a hydrogen atom, and then placed in the presence of diazomethane in an aqueous medium to yield compounds of formula **(XXII)** : 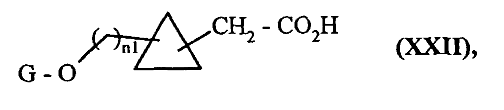 wherein n₁ and G are as defined hereinbefore,
which compounds of formula (XXII) may again be subjected several times to the same reaction series to yield compounds of formula **(XXIII)** : 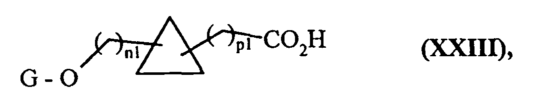 wherein n₁ and G are as defined hereinbefore and p₁ represents an integer of from 2 to 6 inclusive,
which compounds of formula (XXIII) are reacted with diphenylphosphoryl azide, hydrolysed and then treated with a compound of formula (VIA) as described hereinbefore to yield compounds of formula **(XXIV)** : 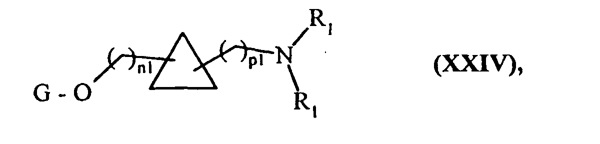 wherein R₁ is as defined for formula (I) and G, n₁ and p₁ are as defined hereinbefore,
which compounds of formula (XXIV) may be subjected to the totality of reactions to which the compounds of formula (VII) are subjected, to yield compounds of formula **(I/l),**
a particular case of the compounds of formula (I) : 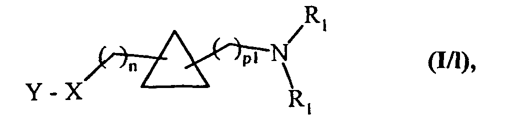 wherein X, Y, R₁ and n are as defined for formula (I) and p₁ is as defined hereinbefore,
the totality of compounds of formulae (I/a) to (I/l) constituting the totality of the compounds of the invention, which are purified, where appropriate, according to a conventional purification technique, which may be separated into their different isomers according to a conventional separation technique, and which are converted, where appropriate, into addition salts thereof with a pharmaceutically acceptable acid or base.

14. Pharmaceutical compositions comprising as active ingredient at least one compound according to any one of claims 1 to 12, alone or in combination with one or more inert, non-toxic, pharmaceutically acceptable excipients or carriers.

15. Pharmaceutical compositions according to claim 14 comprising at least one active ingredient according to any one of claims 1 to 12 for use as a specific nicotinic ligand of α4β2 receptors.

16. Pharmaceutical compositions according to claim 14 comprising at least one active ingredient according to any one of claims 1 to 12 for use in the treatment of deficiencies of memory associated with cerebral ageing and with neurodegenerative diseases, and also for the treatment of mood disorders, Tourette's syndrome, attention-deficit hyperactivity syndrome, tobacco withdrawal and pain.

17. Pharmaceutical compositions according to claim 14 comprising at least one active ingredient according to any one of claims 1 to 12 for use in the treatment of deficiencies of memory associated with Alzheimer's disease, Parkinson's disease, Pick's disease, Korsakoff's disease or frontal lobe and subcortical dementias.
